(19) Europäisches Patentamt
European Patent Office
Office européen des brevets

(11) EP 3 838 900 B1

(12) **EUROPEAN PATENT SPECIFICATION**

(45) Date of publication and mention of the grant of the patent:
**12.02.2025 Bulletin 2025/07**

(21) Application number: **19849974.1**

(22) Date of filing: **16.08.2019**

(51) International Patent Classification (IPC):
***A61K 31/381*** *(2006.01)* ***A61K 31/5377*** *(2006.01)*
***A61K 31/496*** *(2006.01)* ***A61K 31/4015*** *(2006.01)*
***A61K 31/4525*** *(2006.01)* ***A61K 31/4725*** *(2006.01)*
***C07D 277/28*** *(2006.01)* ***C07D 307/52*** *(2006.01)*
***A61P 25/00*** *(2006.01)* ***C07D 307/79*** *(2006.01)*
***C07D 307/86*** *(2006.01)* ***A61P 29/00*** *(2006.01)*
***C07D 407/12*** *(2006.01)* ***A61P 1/00*** *(2006.01)*
***C07D 409/12*** *(2006.01)* ***A61P 11/00*** *(2006.01)*
***A61P 13/02*** *(2006.01)* ***A61P 17/04*** *(2006.01)*
***C07D 409/14*** *(2006.01)*

(52) Cooperative Patent Classification (CPC):
**C07D 333/20; A61K 31/381; A61K 31/4015; A61K 31/4525; A61K 31/4725; A61K 31/496; A61K 31/5377; A61P 1/00; A61P 1/04; A61P 11/00; A61P 13/02; A61P 17/04; A61P 25/00; A61P 25/04; A61P 29/00;** (Cont.)

(86) International application number:
**PCT/CN2019/101197**

(87) International publication number:
**WO 2020/035070 (20.02.2020 Gazette 2020/08)**

(54) **3-ARYLOXY-3-ARYL-PROPYLAMINE COMPOUND AND USES THEREOF**

3-ARYLOXY-3-ARYL-PROPYLAMIN-VERBINDUNG UND VERWENDUNGEN DAVON

COMPOSÉS DE 3-ARYLOXY-3-ARYL-PROPYLAMINE ET UTILISATIONS ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.08.2018 CN 201810942562**

(43) Date of publication of application:
**23.06.2021 Bulletin 2021/25**

(73) Proprietors:
- **Zhangzhou Pien Tze Huang Pharmaceutical Co., Ltd.**
  **Fujian Province, 363000 (CN)**
- **Shanghai Leado Pharmatech Co. Ltd.**
  **Shanghai 201114 (CN)**

(72) Inventors:
- **WANG, Youxin**
  **Shanghai 201114 (CN)**
- **LIN, Jinxia**
  **Zhangzhou, Fujian 363000 (CN)**
- **CHEN, Zhiliang**
  **Zhangzhou, Fujian 363000 (CN)**
- **LAN, Wenliang**
  **Zhangzhou, Fujian 363000 (CN)**
- **ZHANG, Lingling**
  **Shanghai 201114 (CN)**

(74) Representative: **Mewburn Ellis LLP**
**Aurora Building**
**Counterslip**
**Bristol BS1 6BX (GB)**

(56) References cited:
**WO-A1-02/094262**
**WO-A1-2018/115064**
**CN-A- 105 497 020**
**CN-A- 107 625 762**
**CN-A- 107 840 845**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **BOOT, J.R.: "Benzothienyloxy phenylpropanamines, novel dual inhibitors of serotonin and norepinephrine reuptake", BIOORGANIC & MEDICINAL CHEMISTRY LETTERS, vol. 14, no. 21, 9 September 2004 (2004-09-09), pages 5395 - 5399, XP004580536, DOI: 10.1016/j.bmcl.2004.08.005**
- **CASHMAN JOHN R.: "Inhibition of serotonin and norepinephrine reuptake and inhibition of phosphodiesterase by multi-target inhibitors as potential agents for depression", BIOORGANIC & MEDICINAL CHEMISTRY, vol. 17, no. 19, 20 August 2009 (2009-08-20), pages 6890 - 6897, XP026601536**


Remarks:

The file contains technical information submitted after the application was filed and not included in this specification

(52) Cooperative Patent Classification (CPC): (Cont.)
**C07D 277/28; C07D 307/52; C07D 307/79; C07D 307/86; C07D 333/54; C07D 407/12; C07D 409/12; C07D 409/14; C07D 413/12; C07D 417/12; C07D 471/04**

## Description

### FIELD OF THE INVENTION

The present invention relates to the field of medicinal chemistry and pharmacotherapy. Specifically, the present invention relates to a 3-aryloxy-3-aryl-propylamine compound and uses thereof.

### BACKGROUND OF THE INVENTION

Pain is an unpleasant feeling and emotional experience caused by tissue damage or potential tissue damage. It is a warning signal sent by the body. However, severe or long-term pain causes an unbearable torture on the body and seriously affects the patient's life. Therefore, the International Pain Society has designated October 11 of each year as "Global Pain Day" since 2004.

Pain can be divided into acute pain and chronic pain based on duration time. Acute pain is nociceptive pain usually caused by tissue trauma, while chronic pain is a disease mainly dominated by neuropathic pain. Traditional analgesic drugs mainly comprise opioids and non-steroidal anti-inflammatory drugs. Opioids have strong analgesic effect, but long-term use of opioids is easy to cause tolerance, dependence and addiction, and opioids have adverse effects such as respiratory depression and central sedation. Non-steroidal anti-inflammatory drugs only have moderate analgesic effect, while having adverse effects such as gastrointestinal bleeding and cardiotoxicity, etc.

TRPA1, also known as ANKTM1, is a member of TRP ion channel superfamily. TRPA1 is mainly distributed on the primary sensory neurons of dorsal root nerve (DRG), trigeminal nerve (TG) and vagus nerve (VG), and is expressed in peptide energy neurons having rich neuropeptides CGRP, SP and neurotrophic factor receptor TrkA, and in non-peptidergic neurons co-expressing purinergic receptors P2X3, neurturin, artemin, G protein-coupled receptors in Mrg family, and GFR$\alpha_1$, GFR$\alpha_2$ in GDNF receptor family). From the distribution of human system, TRPA1 is highly expressed in the peripheral nervous system, respiratory system, gastrointestinal system and urinary system. When these organs and tissues have abnormal functions, the expression and function of TRPA1 channels are usually abnormal simultaneously. TRPA1 can convert cold stimulation, chemical stimulation and mechanical stimulation into inward currents, trigger a series of physiological functions, and participate in the formation of various pain sensations.

Inflammation is the defensive response of living tissues with vascular system to injury factors. The stimulation of inflammatory mediators such as prostaglandin, serotonin, bradykinin, etc. is the main cause of local pain caused by inflammation. Inflammatory pain is common problem of certain chronic diseases, and there is still no effective treatment method in the clinic. Animal experiments have shown that TRPA1 is involved in inflammatory response and plays an important role in inflammatory pain. The use of TRA1 specific blockers can significantly reduce the inflammatory pain response in rats. The pathogenesis of asthma and cough has become more and more clear as the research continues. From the current research, TRPA1 plays an important role in the occurrence of asthma and cough. Compounds that induce asthma and cough, either cellular endogenous factors or exogenous factors, can activate TRPA1. TRPA1 antagonists can reduce asthma symptoms and block airway hyper-responsiveness.

Visceral pain, as a main visceral sensation, is often caused by viscera stimulation such as mechanical traction, spasm, ischemia, or inflammation, etc. It is confirmed that TRPA1 is involved in the regulation of visceral hypersensitivity through different visceral hypersensitivity animal models such as colitis, rectal dilatation or stress. Neuropathic pain is a pain syndrome caused by central or peripheral nervous system damage or disease, mainly manifested as allodynia, allodynia, and spontaneous pain. Different from inflammatory pain, neurogenic pain is not related to vascular response of central inflammation, but depends on the damage and dysfunction of nervous system, which is often caused by peripheral nerve damage. In recent years, more and more studies have shown that TRPA1 channel plays an important role in different neurogenic pain, such as diabetic neuropathy and neuropathy caused by chemotherapy drugs. Recent studies have also shown that TRPA1 also has a mediating role in toothache, migraine and other pains. The administration of TRPA1 antagonists can significantly alleviate the pain symptoms.

Since TRPA1 is widely distributed and expressed in the human system, the importance of its function is self-evident. In addition to the physiological functions involved by TRPA1, the development of TRPA1 inhibitor indications reported so far also involves inflammatory bowel disease, chronic obstructive pulmonary disease, antitussive, antipruritic, allergic rhinitis, ear disease, diabetic, urinary incontinence, etc. It is proved that TRPA1 is a new target for pain treatment. There is no commercial drug for TRPA1 target. Pain is a refractory disease.

CN107840845A describes a compound:

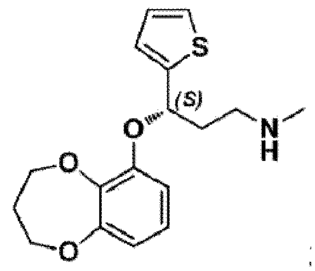

as well as its activity to inhibit RTPA 1 activity and use to treat diseases related to RTP.

WO2018/115064A1 describes compounds of the formulae below showing affinity and activity towards the subunit $\alpha 2\delta$ of voltage-gated calcium channels (VGCC):

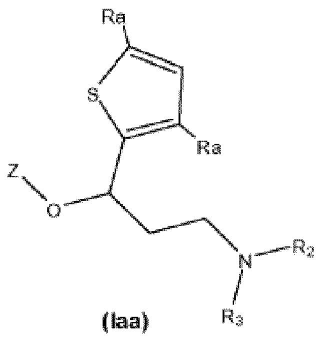

(Iaa)

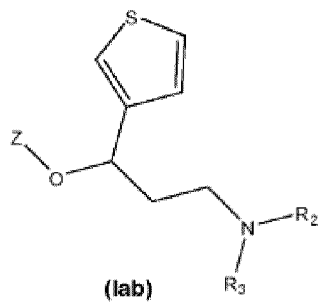

(Iab)

Z is one of the following moieties:

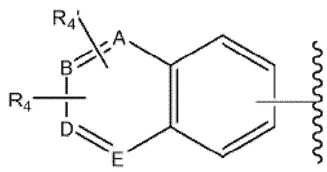

or

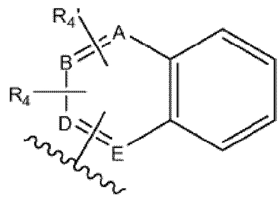

.

BOOT, J.R. et al (Bioorganic & Medicinal Chemistry Letters, vol. 14, no. 21, pages 5395-5399) describes a compound:

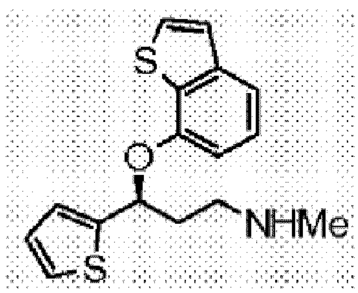

.

WO02/094262A1 describes a compound:

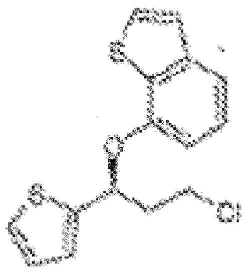

.

CASHMAN JOHN R. et al. (Bioorganic & Medicinal Chemistry, vol. 17, no. 19, pages 6890-6897) describes a compound:

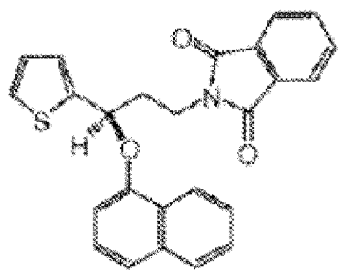

(R)-9 .

CN105497020A describes compounds of the formula below:

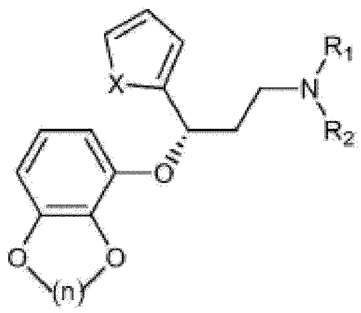

I .

However, there is still an urgent need in the art to develop a medicament targeting TRP, especially TRPA1, to improve therapeutic effect of diseases.

## SUMMARY OF THE INVENTION

It is an object of the present invention to provide novel compounds which target TRP channel, especially TRPA1, and uses thereof.

In the first aspect of the present invention, it provides a compound of formula I, or a pharmaceutically acceptable salt thereof for use in preventing and/or treating a disease related to transient receptor potential channel protein (TRP), wherein the disease is selected from the group consisting of pain, epilepsy, inflammation, respiratory disorder, pruritus, urinary tract disorder and inflammatory bowel disease;

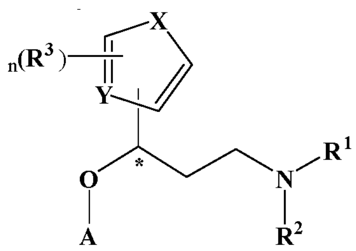

I

wherein,

A is benzofuranyl or indanyl;
$R^1$ and $R^2$ are each independently hydrogen, substituted or unsubstituted $C_1$-$C_6$ alkyl;
X is oxygen atom or sulfur atom;
Y is CH or nitrogen atom;
$R^3$ is hydrogen, halogen, substituted or unsubstituted $C_1$-$C_6$ alkyl;
n is 1;
"*" represents a chiral carbon atom, and the absolute configuration of the chiral carbon atom is S type;
wherein the "substituted" means that 1-4 (preferably 1, 2, or 3) hydrogen atoms on the group are substituted by a substituent selected from the group consisting of $C_1$-$C_6$ alkyl, $C_3$-$C_7$ cycloalkyl, $C_1$-$C_3$ haloalkyl, halogen, nitro, cyano, hydroxyl, $C_1$-$C_4$ carboxyl, $C_2$-$C_4$ ester group, $C_2$-$C_4$ amide group, $C_1$-$C_6$ alkoxyl, $C_1$-$C_6$ haloalkoxy, benzyl, 5- or 6-membered aryl or heteroaryl (preferably $C_6$ aryl or $C_5$ heteroaryl).

In another preferred embodiment, X is S.

In another preferred embodiment, the heteroaryl contains 1-3 heteroatoms selected from the group consisting of N, O and S.

In another preferred embodiment, the transient receptor potential channel protein (TRP) is TRPA1.

In another preferred embodiment, $R^1$ and $R^2$ are each independently hydrogen atom, or $C_1$-$C_3$ alkyl.

In another preferred embodiment, $R^3$ is hydrogen atom, halogen, or substituted or unsubstituted $C_1$-$C_3$ alkyl.

In another preferred embodiment, $R^1$ and $R^2$ are each independently hydrogen, or methyl,.

In another preferred embodiment, $R^3$ is hydrogen atom, chlorine atom or methyl.

In the second aspect of the present invention, it provides a compound selected from the following group, or a pharmaceutically acceptable salt thereof for use in preventing and/or treating a disease related to transient receptor potential channel protein (TRP), wherein the disease is selected from the group consisting of pain, epilepsy, inflammation, respiratory disorder, pruritus, urinary tract disorder and inflammatory bowel disease:

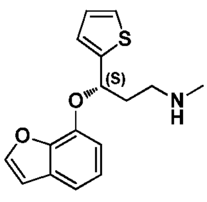

Ic-1

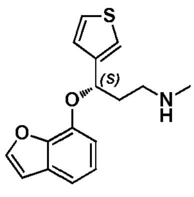

Ic-2

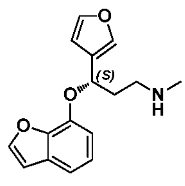

Ic-3

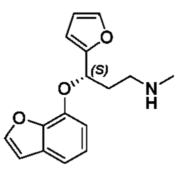

Ic-4

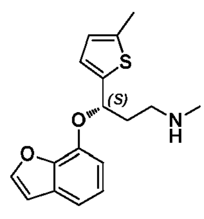

Ic-5

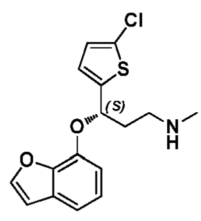

Ic-6

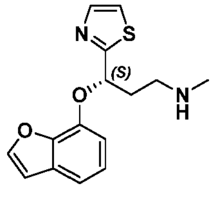

Ic-7

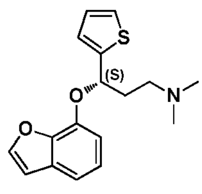

Ic-8

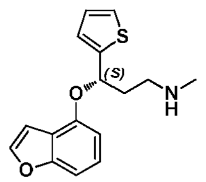

Ic-9

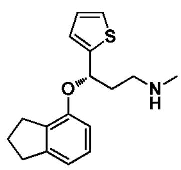

Ic-10

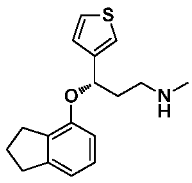

Ic-11

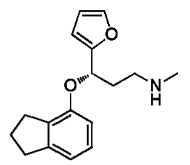

Ic-12

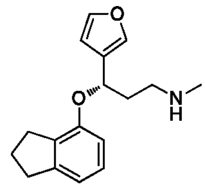

Ic-13

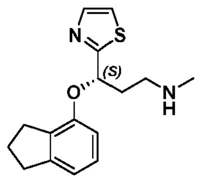

Ic-14

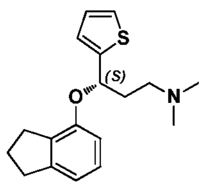

Ic-15

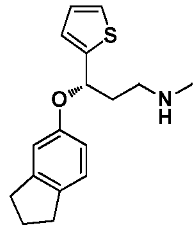

Ic-16

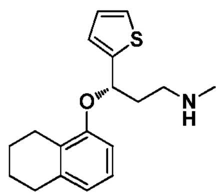

Ic-17

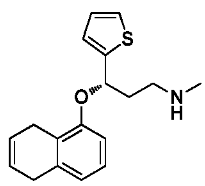

Ic-18

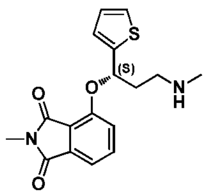

Ic-19

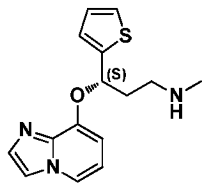

Ic-20

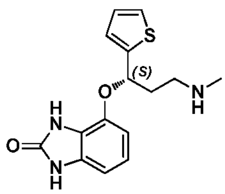

Ic-21

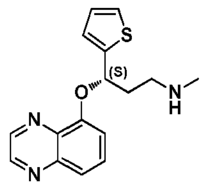

Ic-22

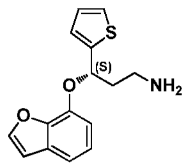

Ic-23

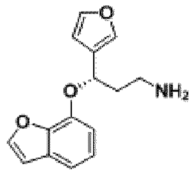

Ic-24

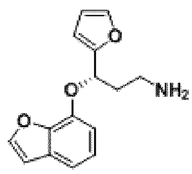

Ic-25

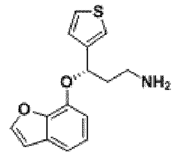

Ic-26

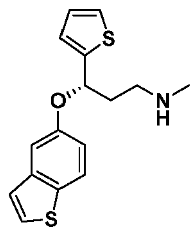

Ic-27

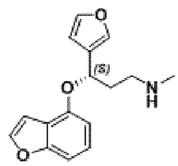

Ic-28

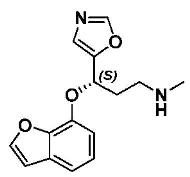

Ic-29

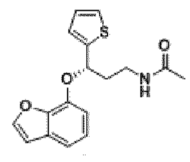

ID

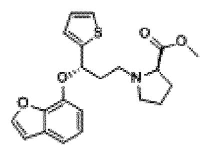

IE

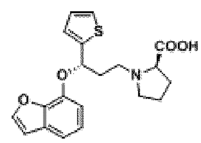

IF

In another preferred embodiment, the transient receptor potential channel protein (TRP) is TRPA1.

In another preferred embodiment, the disease related to transient receptor potential channel protein (TRP) is pain.

In another preferred embodiment, the pain comprises acute inflammatory pain, chronic inflammatory pain, visceral pain, neurogenic pain, fibromyalgia, headache, neuralgia or pain caused by cancer.

In another preferred embodiment, the headache is migraine or muscle tension pain.

In another preferred embodiment, the neuralgia is trigeminal neuralgia, diabetic pain or post-zoster neuralgia.

In another preferred embodiment, the pain is selected from the group consisting of acute pain, fibromyalgia, visceral pain, inflammatory pain, neuralgia, or a combination thereof.

In another preferred embodiment, the other pain is fibromyalgia.

In the third aspect of the present invention, it provides a compound of formula I, or a pharmaceutically acceptable

salt thereof;

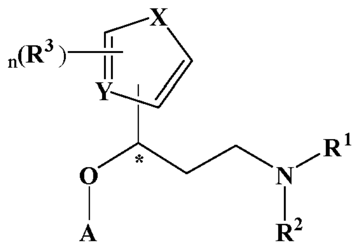

I

wherein,

A is benzofuranyl or indanyl;
$R^1$ and $R^2$ are each independently hydrogen, substituted or unsubstituted $C_1$-$C_6$ alkyl;
X is oxygen atom, or sulfur atom;
Y is CH or nitrogen atom;
at least one of X and Y is heteroatom;
$R^3$ is hydrogen, halogen, substituted or unsubstituted $C_1$-$C_6$ alkyl;
n is 1;
"*" represents a chiral carbon atom, and the absolute configuration of the chiral carbon atom is S type;
wherein, the "substituted" means that 1-4 (preferably 1, 2, or 3) hydrogen atoms on the group are substituted by a substituent selected from the group consisting of $C_1$-$C_6$ alkyl, $C_3$-$C_7$ cycloalkyl, $C_1$-$C_3$ haloalkyl, halogen, nitro, cyano, hydroxyl, $C_1$-$C_4$ carboxyl, $C_2$-$C_4$ ester group, $C_2$-$C_4$ amide group, $C_1$-$C_6$ alkoxyl, $C_1$-$C_6$ haloalkoxy, benzyl, 5- or 6-membered aryl or heteroaryl (preferably $C_6$ aryl or $C_5$ heteroaryl).

In another preferred embodiment, X is S.

In another preferred embodiment, the heteroaryl contains 1-3 heteroatoms selected from the group consisting of N, O and S.

In another preferred embodiment, $R^1$ and $R^2$ are each independently hydrogen atom, or $C_1$-$C_3$ alkyl.

In another preferred embodiment, $R^3$ is hydrogen atom, halogen, or substituted or unsubstituted $C_1$-$C_3$ alkyl.

In another preferred embodiment, $R^1$ and $R^2$ are each independently hydrogen, or methyl.

In another preferred embodiment, $R^3$ is hydrogen atom, chlorine atom or methyl.

In the fourth aspect of the present invention, it provides a compound selected from the following group or a pharmaceutically acceptable salt thereof:

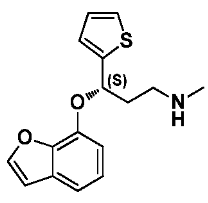

Ic-1

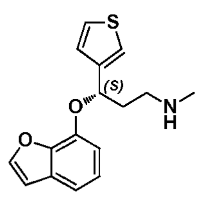

Ic-2

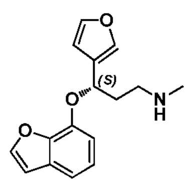

Ic-3

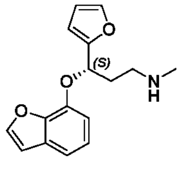

Ic-4

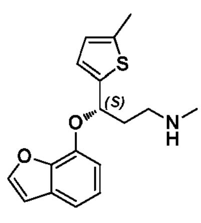

Ic-5

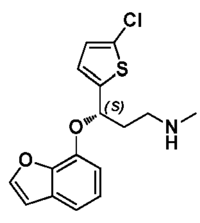

Ic-6

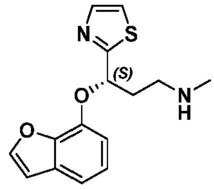

Ic-7

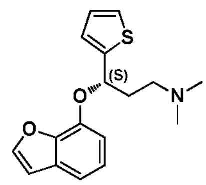

Ic-8

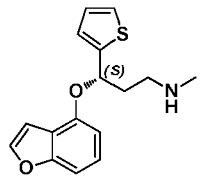
$I_C$-9

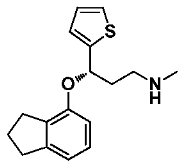
$I_C$-10

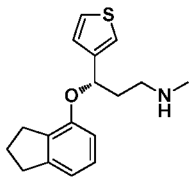
$I_C$-11

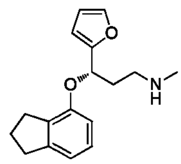
$I_C$-12

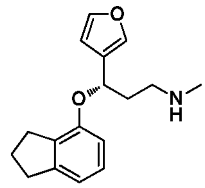
$I_C$-13

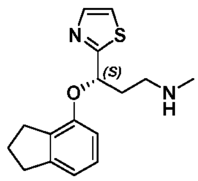
$I_C$-14

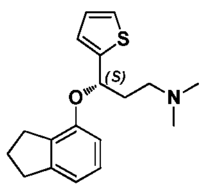
$I_C$-15

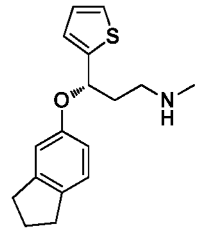
$I_C$-16

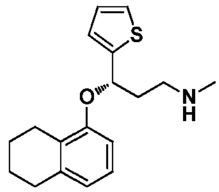
$I_C$-17

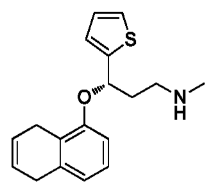
$I_C$-18

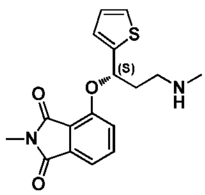
$I_C$-19

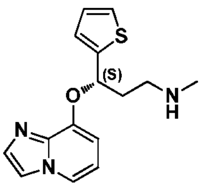
$I_C$-20

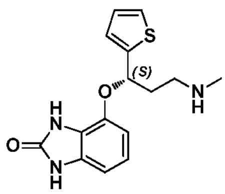
$I_C$-21

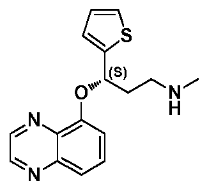
$I_C$-22

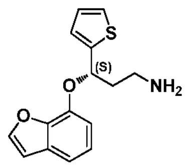
$I_C$-23

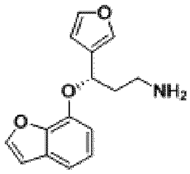
$I_C$-24

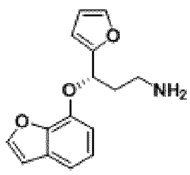
$I_C$-25

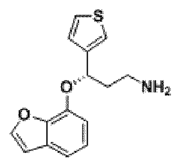
$I_C$-26

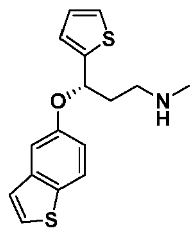
$I_C$-27

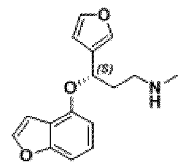
$I_C$-28

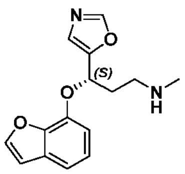
$I_C$-29

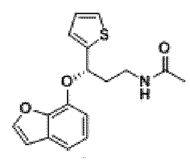
$I_D$

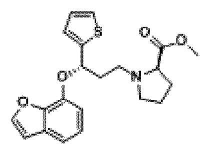
$I_E$

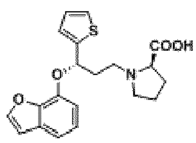
$I_F$

In the fifth aspect of the present invention, it provides a pharmaceutical composition which comprises the compound, or a pharmaceutically acceptable salt thereof according to the third and fourth aspect of the present invention; and a pharmaceutically acceptable carrier.

In the sixth aspect of the present invention, it provides a method for preparing the compound, or a pharmaceutically acceptable salt thereof according to the third and fourth aspect of the present invention, wherein the method comprises: reacting an intermediate of formula II with $R^1$-NH-$R^2$ in an inert solvent, thereby forming the compound,:

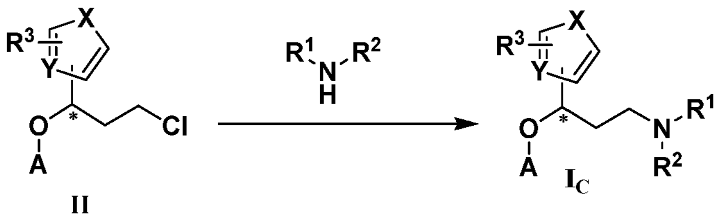

II

$I_C$

wherein X, Y, A, $R^1$, $R^2$, $R^3$ and "*" are as defined in the third aspect of the present invention.

In another preferred embodiment, the method comprises the following step:

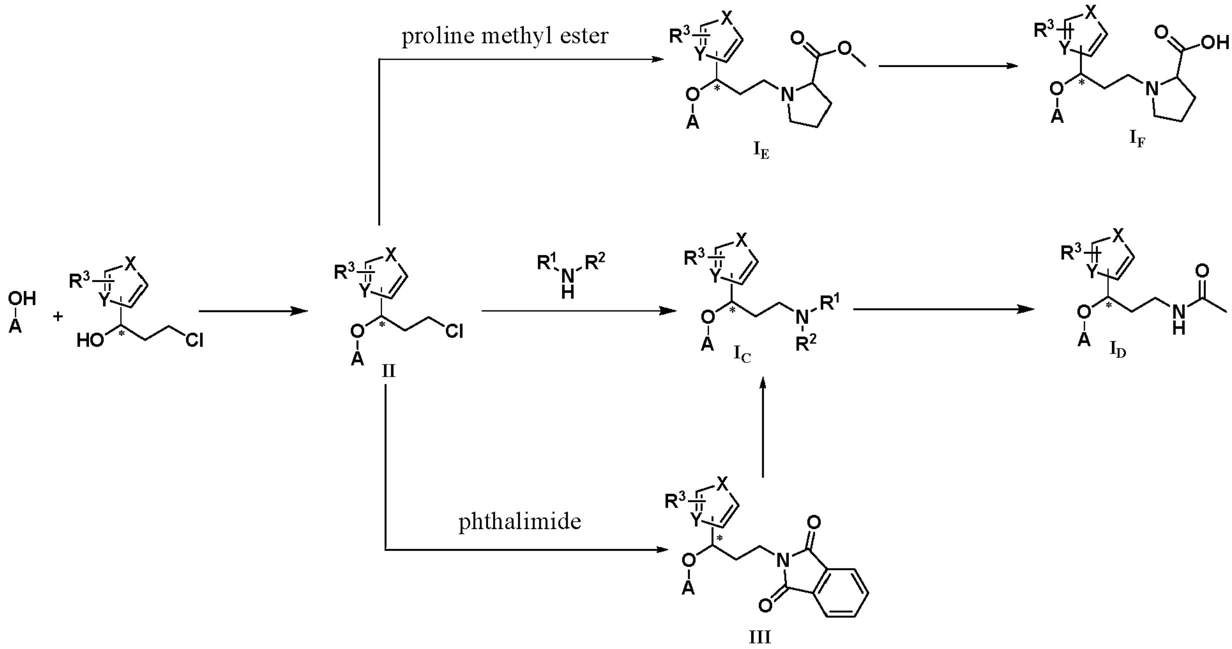


$I_E$ $I_F$ II $I_C$ $I_D$ III

wherein X, Y, A, $R^1$, $R^2$, $R^3$ and "*" are as defined in the third aspect of the present invention;

(a) in an inert solvent and in presence of a condensing agent, reacting a compound A-OH with 1-($R^3$-5-membered heteroaryl)-3-chloro-propanol, thereby forming an intermediate of formula II;
(b) carrying out a reaction selected from the following group to form a compound of formula IC or IE:

(b-1) in an inert solvent, reacting the intermediate of formula II with $R^1$-NH-$R^2$ to form a compound of formula Ic; or
(b-2) in an inert solvent, reacting the intermediate of formula II with phthalimide to form an intermediate of formula III, and subjecting the intermediate of formula III to hydrazinolysis reaction to form the compound of formula Ic; or
(b-3) in an inert solvent, reacting the intermediate of formula II with proline methyl ester to form the compound IE.

In another preferred embodiment, the method further comprises the following step:
(c1) in an inert solvent and in presence of a condensing agent, reacting the compound of formula Ic with acetic acid to form a compound of formula $I_D$;
In another preferred embodiment, the method further comprises the following step:
(c2) in an inert solvent and in presence of a base, subjecting the compound of formula $I_E$ to hydrolysis reaction to form a compound of formula IF.
In another aspect of the present disclosure, it provides an intermediate of formula II or III for preparing the compounds of the present invention:

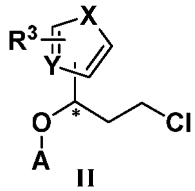

II

or

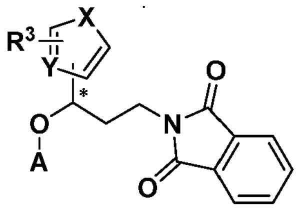

III

wherein, X, Y, A, $R^3$ and "*" are as defined in the third aspect of the present invention.

In the another aspect of the present disclosure, it provides a method for preparing the intermediate for preparing the compounds of the present invention,

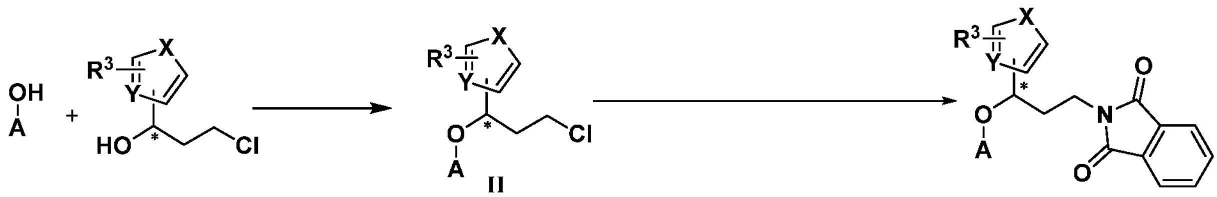

II

III

wherein X, Y, A, $R^1$, $R^2$, $R^3$ and "*" are as defined in the third aspect of the present invention.

(1) the method comprises:

(i) in an inert solvent and in presence of a condensing agent, reacting compound A-OH with 1-($R^3$-5-membered heteroaryl)-3-chloro-propanol, thereby forming an intermediate of formula II;
or (2) the method comprises:

(i) in an inert solvent and in presence of a condensing agent, reacting compound A-OH 1-($R^3$-5- membered heteroaryl)-3-chloro-propanol, thereby forming an intermediate of formula II; and
(ii) in an inert solvent, reacting the intermediate of formula II with phthalimide, thereby forming an intermediate of formula III.

In the seventh aspect of the present invention, it provides a method for non-therapeutically and/or non-diagnostically inhibiting activity of transient receptor potential channel protein *in vitro,* which comprises contacting a transient receptor potential channel protein or a cell expressing the protein with the compound, or a pharmaceutically acceptable salt thereof according to the third and fourth aspect of present invention, thereby inhibiting activity of transient receptor potential channel protein.

It should be understood that, in the present invention, each of the technical features specifically described above and below (such as those in the Examples) can be combined with each other, thereby constituting new or preferred technical solutions which need not be redundantly described one-by-one, as long as they fall within the claims.

## DESCRIPTION OF THE DRAWINGS

Fig. 1A-1E are dose-effect curve of the compounds IC-3, IC-4, IC-8, IC-23 and IC-24 on inhibition of TRPA1 activity.
Fig. 2 shows the results of analgesic activity of compound IC-10 of the present invention in mice formalin pain model.
Fig. 3 shows the results of the analgesic activity of the compound IC-23 of the present invention in mice hot plate pain model.
Fig. 4 shows the results of analgesic activity of compound Ic-1 and duloxetine in mice fibromyalgia model, Mean±SD, n=12, **p<0.01, ***p<0.001 as compared with solvent control group.
Fig. 5 shows the results of analgesic activity of compound IC-1, duloxetine, indomethacin and anisodamine in mice acetic acid writhing pain model.
Fig. 6 shows the results of analgesic activity of compound IC-1 and gabapentin in rat SNL model.
Fig. 7 shows the statistical results of licking claw time of compound Ic-1 and duloxetine in a II phase (10-60 min) at different dosages in the mice formalin model.

## EMBODIMENTS FOR CARRYING OUT THE INVENTION

Based on an extensive and intensive research, the inventors have unexpectedly and firstly developed a compound of formula I, or a pharmaceutically acceptable salt thereof. The experimental results have shown that the compound of present invention have significant inhibitory effect on TRP channels. The compound of present invention can effectively treat a pain and the like related to TRP (especially TRPA1) targets. On this basis, the inventors have completed the present invention.

Terms

As used herein, the terms "comprise", " comprising", and "containing" are used interchangeably, which not only comprise closed definitions, but also semi-closed and open definitions. In other words, the term comprises "consisting of" and "essentially consisting of".

As used herein, "$R^1$", "R1" and "$R_1$" have the same meaning and can be used interchangeably. The other similar definitions have the same meaning.

As used herein, the terms "$C_1$-$C_6$ alkyl" or "$C_1$-$C_3$ alkyl" refer to a linear or branched chain alkyl with 1-6 or 1-3 carbon atoms, such as methyl, ethyl, propyl, isopropyl, butyl, isobutyl, sec-butyl, tert-butyl, or the like.

As used herein, the term "$C_1$-$C_6$ alkoxy" refers to a linear or branched alkoxy having 1 to 6 carbon atoms, such as methoxyl, ethoxyl, propoxyl, isopropoxyl, butoxyl, isobutoxyl, sec-butoxyl, tert-butoxyl, pentoxyl, hexyloxyl, or the like.

As used herein, the term "$C_6$-$C_{12}$ benzoalicyclic group" refers to a group having 6-12 carbon atoms, and comprises indanyl, tetrahydronaphthyl, dihydronaphthyl, or the like.

As used herein, the term "$C_3$-$C_7$ cycloalkyl" refers to a cycloalkyl having 3-7 carbon atoms, and comprises monocyclic, bicyclic or polycyclic ring, such as cyclopropyl, cyclobutyl, methylcyclobutyl, cyclopentyl, cycloheptyl, or the like.

As used herein, the term "$C_2$-$C_6$ ester group" refers to a group having C1-C5 alkyl-COO- structure or a group having -COO-C1-C5 alkyl structure, wherein the alkyl can be linear or branched chain, such as $CH_3COO$-, $C_2H_5COO$-, $C_3H_8COO$-, $(CH_3)_2CHCOO$-, -$COOCH_3$, -$COOC_2H_5$, -$COOC_3H_8$, or the like.

As used herein, the term "$C_2$-$C_4$ amide group" refers to a group having $C_1$-$C_3$ alkyl-CO-NH- structure or a group having -CO-NH-$C_1$-$C_3$ alkyl structure, wherein the alkyl can be linear or branched, such as $CH_3$-CO-NH-, $C_2H_5$-CO-NH-, $C_3H_8$-CO-NH-, -$COOCH_3$, -CO-NH-$C_2H_5$, -CO-NH-$C_3H_8$, or the like.

As used herein, the term "$C_2$-$C_4$ acyl" refers to a group having $C_1$-$C_3$ alkyl-CO-structure, wherein the alkyl can be linear or branched, such as $CH_3$-CO-, $C_2H_5$-CO-, $C_3H_8$-CO-, or the like.

As used herein, the term "$C_3$-$C_7$ heterocycloalkyl" refers to a monocyclic and polycyclic heterocycle (preferably monocyclic heterocycle) having 3-7 ring carbon atoms and 1-3 heteroatoms (preferably containing one nitrogen atom, which is the nitrogen atom commonly adjacent to $R^1$ and $R^2$), such as piperidinyl, tetrahydropyrrolyl, or the like.

As used herein, the term "5-7 membered carbocyclic ring" refers to any stable 5-, 6- or 7-membered monocyclic, bicyclic or polycyclic ring. The carbocyclic ring can be a saturated, partially unsaturated, or unsaturated ring, but cannot be an aromatic ring. Examples of carbocyclic ring comprise, but are not limited to, cyclopropyl, cyclobutyl, cyclobutenyl, cyclopentyl, cyclopentenyl, cyclohexyl, cyclohexenyl, cycloheptyl, cycloheptenyl, adamantyl, cyclooctyl, cyclooctenyl, cyclooctadienyl, bicyclo[3.3.0]octanyl, bicyclo[4.3.0]nonanyl, bicyclo[4.4.0]decanyl, bicyclo[2.2.2] octanyl, fluorenyl, indanyl.

As used herein, the term "heterocyclic ring" refers to any stable monocyclic, bicyclic or polycyclic ring, for example, 5-, 6- or 7 membered ring, wherein the heterocyclic ring contains one or more (such as 1-3) heteroatom selected from N, O and S, the heterocyclic ring can be a saturated, partially unsaturated, or unsaturated ring, but can not be an aromatic ring.

As used herein, the terms "$C_1$-$C_6$ haloalkyl" and "$C_1$-$C_3$ haloalkyl" mean that one or more hydrogen atoms of a linear or branched alkyl having 1-6 and 1-3 carbon atoms are substituted by halogen, such as monochloromethanyl, dichloroethanyl, trichloropropanyl, or the like.

As used herein, the term "$C_1$-$C_4$ carboxyl" refers to $C_1$-$C_3$ alkyl-COOH, wherein the alkyl can be linear or branched, such as $CH_3COOH$, $C_2H_5COOH$, $C_3H_8COOH$, $(CH_3)_2CHCOOH$, or the like.

As used herein, the term "$C_6$-$C_{12}$ aryl" refers to a monocyclic or bicyclic aromatic hydrocarbon group having 6 to 12 carbon atoms in the ring, such as phenyl, naphthyl, biphenyl, or the like.

As used herein, the term "heteroaryl" refers to an optionally substituted aromatic group (for example, 5- to 7-membered monocyclic ring) which contains at least one heteroatom and at least one carbon atom, for example pyrrolyl, thienyl, pyridyl, pyridazinyl, pyrimidinyl, pyrazinyl, furanyl, imidazolyl, thiazolyl, oxazolyl, triazolyl or the like.

As used herein, the term "halogen" refers to F, Cl, Br, and I.

As used herein, the term "substituted" means that a hydrogen atom on the group is replaced by a non-hydrogen atom group, but the valence requirement must be met and the substituted compound is chemically stable. In the

specification, it should be understood that each substituent is unsubstituted, unless it is expressly described as "substituted" herein.

Similarly, it should be understood that substituents can be connected to a parent group or a substrate on any atom in present invention, unless the connection violates the valence requirement; and the hydrogen atoms of parent group or substrate can be on the same atom or different atoms.

As used herein, as for a numerical range from P1 to P2, the range contains not only the endpoints P1 and P2, but also any numerical points between the endpoints P1 and P2. In addition, when both P1 and P2 are positive numbers, for an integer n, its value range contains any integer value point between the endpoints P1 and P2. For example, for an integer, when its value range is 1-10, it contains 1, 2, 3, 4, 5, 6, 7, 8, 9 and 10; the value range 3-7 contains 3, 4, 5, 6, and 7. Typically, as for a group, $C_3$-$C_7$ contains C3, C4, C5, C6 and C7.

**Active ingredient**

As used herein, the terms "compound of present invention" and "3-aryloxy-3-aryl-propylamine compound of present invention" refer to a compound or a pharmaceutically acceptable salt thereof according to the third and fourth second aspect of the present invention,. It should be understood that the term also comprises a mixture of the above components.

The compound of present invention not only has inhibitory effect on TRPA1, but also has certain inhibitory effect on other members of TRP family.

The term "pharmaceutically acceptable salt" refers to a salt formed by a compound of the present invention and an acid or a base suitable for use as a medicine. Pharmaceutically acceptable salts include inorganic salts and organic salts. A preferred type of salt is the salt formed by the compound of the present invention and an acid. Acids suitable for salt formation include (but are not limited to): inorganic acid such as hydrochloric acid, hydrobromic acid, hydrofluoric acid, sulfuric acid, nitric acid, phosphoric acid and the like; organic acid such as formic acid, acetic acid, propionic acid, oxalic acid, malonic acid, succinic acid, fumaric acid, maleic acid, lactic acid, malic acid, tartaric acid, citric acid, picric acid, methane sulfonic acid, toluene sulfonic acid, benzenesulfonic acid and the like; and acidic amino acid such as aspartic acid and glutamic acid. A preferred type of salt is a metal salt formed by the compound of the present invention and a base. Suitable bases for salt formation include (but are not limited to): inorganic base such as sodium hydroxide, potassium hydroxide, sodium carbonate, sodium bicarbonate, sodium phosphate and the like; and organic base such as ammonia, triethylamine, diethylamine and the like.

The preferred compounds of present invention comprise any compound selected from Table 1 (except Compounds $I_A$-1 to $I_A$-4 and $I_B$-1 to $I_B$-3, which are provided as reference examples):

**Table 1**

| No. | Chemical Name | Structure |
|---|---|---|
| Compound $I_A$-1 (reference example) | (S)-N-methyl-3-(quinolin-8-ylo xy)-3-(thiophen-2-yl)propyl-1-a mine | 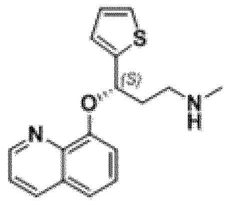 |
| Compound $I_A$-2 (reference example) | (S)-N-methyl-3-(quinolin-6-ylo xy)-3-(thiophen-2-yl)propyl-1-a mine | 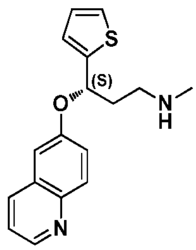 |
| Compound $I_A$-3 (reference example) | (S)-N-methyl-3-(quinolin-4-ylo xy)-3-(thiophen-2-yl)propyl-1-a mine | 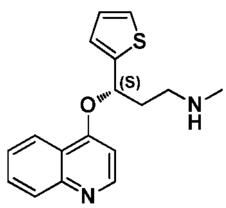 |

(continued)

| No. | Chemical Name | Structure |
|---|---|---|
| Compound IA-4 (reference example) | (S)-N-methyl-3-(quinolin-5-ylo xy)-3-(thiophen-2-yl) propyl-1-amine | 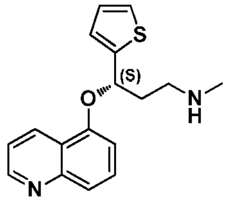 |
| Compound $I_B$-**1** (reference example) | (S)-N-methyl-3 -(isoquinolin-4-yloxy)-3-(thiophen-2-yl)propyl-1-amine | 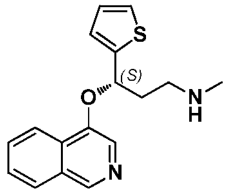 |
| Compound $I_B$-**2** (reference example) | (S)-N-methyl-3-(isoquinolin-8-yloxy)-3-(thiophen-2-yl)propyl-1-amine | 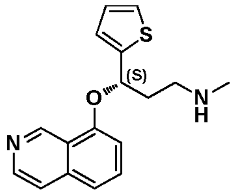 |
| Compound $I_B$-**3** (reference example) | (S)-N-methyl-3-(isoquinolin-5-yloxy)-3-(thiophen-2-yl)propyl-1-amine | 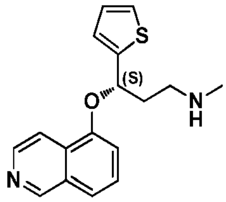 |
| Compound **Ic-1** | (S)-3-(benzofuran-7-yloxy)-N-methyl-3-(thiophen-2-yl)propyl -1-amine | 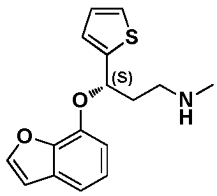 |
| Compound **Ic-2** | (S)-3-(benzofuran-7-yloxy)-N-methyl-3-(thiophen-3-yl)propyl -1-amine | 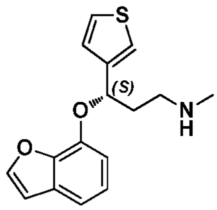 |
| Compound **Ic-3** | (S)-3-(benzofuran-7-yloxy)-N-methyl-3-(furan-3-yl)propyl-1-a mine | 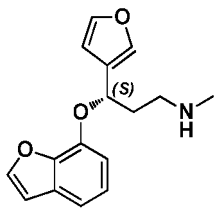 |

(continued)

| No. | Chemical Name | Structure |
|---|---|---|
| Compound **Ic-4** | (S)-3-(benzofuran-7-yloxy)-N-methyl-3 -(furan-2-yl)propyl-1 -a mine | 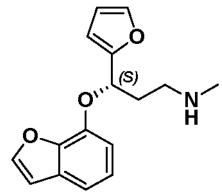 |
| Compound **Ic-5** | (S)-3-(benzofuran-7-yloxy)-N-methyl-3-(5-methylthiophen-2-yl)propyl-1-amine | 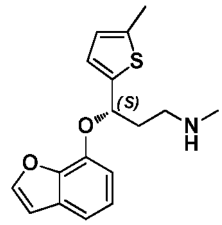 |
| Compound **Ic-6** | (S)-3-(benzofuran-7-yloxy)-N-methyl-3-(5-chlorothiophen-2-y l)propyl-1-amine | 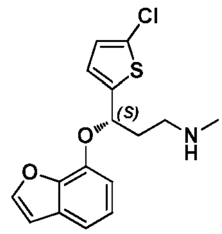 |
| Compound **Ic-7** | (S)-3-(benzofuran-7-yloxy)-N-methyl-3-(thiazol-2-yl)pro-pyl-1 -amine | 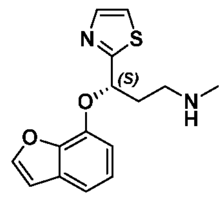 |
| Compound **Ic-8** | (S)-3-(benzofuran-7-yloxy)-N, N-dimethyl-3-(thiophen-2-yl) pr opyl-1-amine | 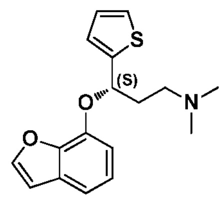 |
| Compound **Ic-9** | (S)-3-(benzofuran-4-yloxy)-N-methyl-3-(thiophen-2-yl)pro-pyl -1-amine | 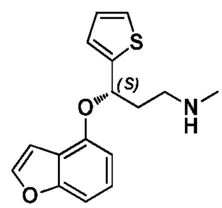 |
| Compound **Ic-10** | (S)-3-(2,3-dihydro-1H-inden-4-yloxy)-N-methyl-3-(2-thienyl) p ropyl-1-amine | 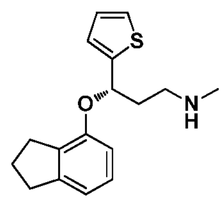 |

(continued)

| No. | Chemical Name | Structure |
|---|---|---|
| Compound **Ic-11** | (S)-3 -(2,3 -dihydro-1H-inden-4-yloxy)-N-methyl-3-(3-thienyl)p ropyl-1-amine | 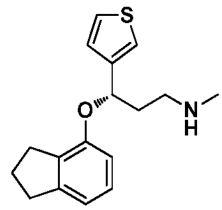 |
| Compound **Ic-12** | (S)-3 -(2,3 -dihydro-1H-inden-4-yloxy)-N-methyl-3-(2-furyl) pro pyl-1-amine | 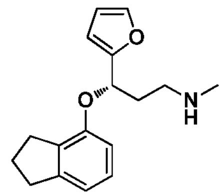 |
| Compound **Ic-13** | (S)-3 -(2,3 -dihydro-1H-inden-4-yloxy)-N-methyl-3-(3-furyl) pro pyl-1-amine | 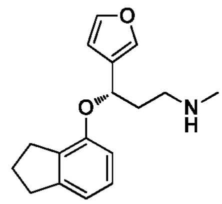 |
| Compound **Ic-14** | (S)-3 -(2,3 -dihydro-1H-inden-4-yloxy)-N-methyl-3-(2-thia-zolyl) propyl-1-amine | 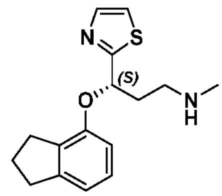 |
| Compound **Ic-15** | (S)-3 -(2,3 -dihydro-1H-inden-4-yloxy)-N,N-dimethyl-3-(2-thien yl)propyl-1-amine | 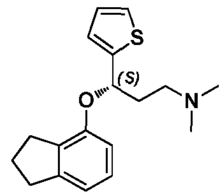 |
| Compound **Ic-16** | (S)-3-(2,3-dihydro-1H-inden-5-yloxy)-N-methyl-3-(2-thienyl) p ropyl-1-amine | 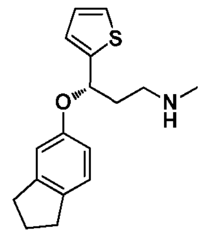 |
| Compound **Ic-17** | (S)-(3-(tetrahydronaphthalen-1-yl)oxy)-N-methyl-3 -(2-thie-nyl) propyl-1-amine | 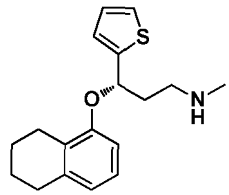 |

(continued)

| No. | Chemical Name | Structure |
|---|---|---|
| Compound **Ic-18** | (S)-(3-(5,8-dihydronaphthalen-l-yl)oxy)-N-methyl-3-(2-thieny l)propyl-1-amine | 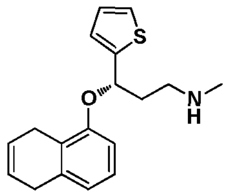 |
| Compound **Ic-19** | (S)-2-methyl-4-(3-(methylamin o)-1 -(thiophen-2-yl)propoxyl)is oindoline-1,3-dione | 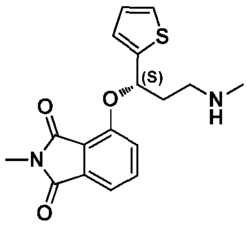 |
| Compound **Ic-20** | (S)-3-(imidazo[1,2-*a*]pyridine-8-oxy)-N-methyl-3-( 2-thienyl) propyl-1-amine | 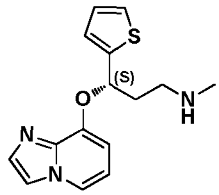 |
| Compound **Ic-21** | (S)-4-(3-(methylamino)-1-(thio phen-2-yl)propoxy)-1,3-dihydro -2H benzo[d]imidazol-2-one | 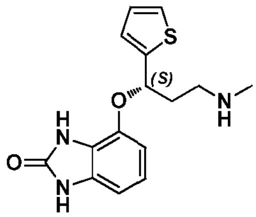 |
| Compound **Ic-22** | (S)-N-methyl-3-(quinoxalin-5-y loxy)-3-(thiophen-2-yl)propyl-1 -amine | 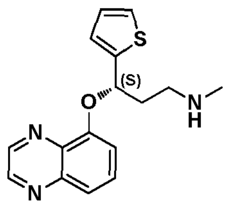 |
| Compound **Ic-23** | (S)-3-(benzofuran-7-yloxy)-3-(t hiophen-2-yl)propyl-1-amine | 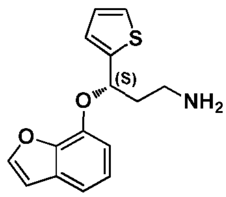 |
| Compound **Ic-24** | (S)-3-(benzofuran-7-yloxy)-3-(f uran-3 -yl)propyl-1 -amine | 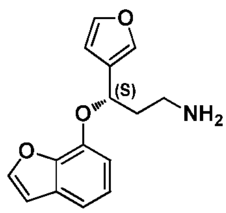 |

(continued)

| No. | Chemical Name | Structure |
|---|---|---|
| Compound **Ic-25** | (S)-3-(benzofuran-7-yloxy)-3-(f uran-2-yl)propyl-1-amine | 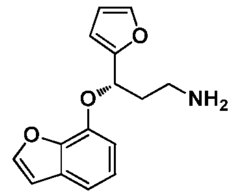 |
| Compound **Ic-26** | (S)-3(benzofuran-7-yloxy)-3-(th iophen-3 -yl)propyl-1 -amine | 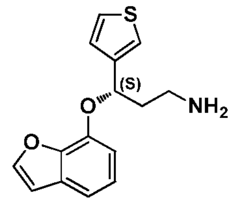 |
| Compound **Ic-27** | (S)-3(benzo[b]thiophen-5-yloxy )-N-methyl-3-(thiophen-2-yl)pr opyl-1-amine | 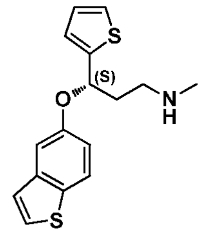 |
| Compound **Ic-28** | (S)-3-(benzofuran-4-yloxy)-N-methyl-3-(furan-3-yl)-propyl-1-amine | 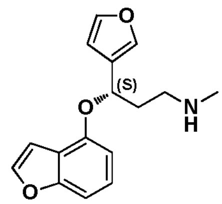 |
| Compound **Ic-29** | (S)-3-(benzofuran-7-yloxy)-N-methyl-3-(oxazol-5-yl)propyl-1 -amine | 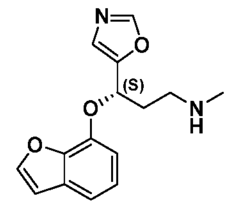 |
| Compound **In** | (S)-N-(3 -(benzofuran-7-yloxy)-3-(thiophen-2-yl)propyl) acetam ide | 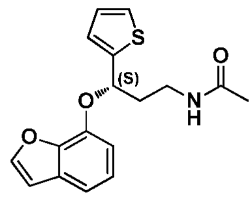 |
| Compound **I$_E$** | ((S)-3-(benzofuran-7-yloxy)-3-( thiophen-2-yl)propyl)-L-proline methyl ester | 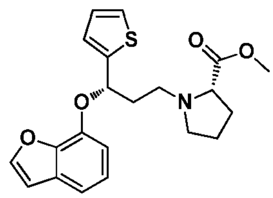 |

(continued)

| No. | Chemical Name | Structure |
|---|---|---|
| Compound $I_F$ | ((S)-3-(benzofuran-7-yloxy)-3-( thiophen-2-yl)propyl)-L-proline | 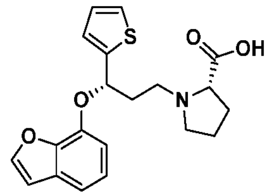 |

**Preparation method**

The present disclosure further provides a method for preparing 3-aryloxy-3-aryl-propylamine compounds of formula $I_A$ to $I_F$.

The present invention further provides a method for preparing intermediates of formula II-III which are useful for preparing the above-mentioned compounds.

The specific synthesis method are as follows:

Synthesis of compounds of formula $I_A$ and $I_B$ (not part of the present invention:

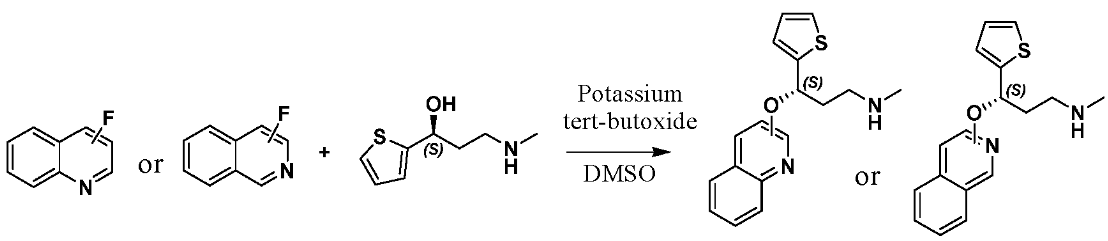


$I_A$ $I_B$

Potassium tert-butoxide is added into dimethyl sulfoxide (DMSO) solution of (S)-3-(methylamino)-1-(thiophen-2-yl)-1-propanol, and the mixture is stirred for 10-20 minutes. - Quinoline or isoquinoline substituted by fluorine at different position is added, and the reaction is carried out at 80-120°C overnight. After the reaction is completed, water is added into the reaction system, then reaction system is extracted three times with ethyl acetate, washed with water and saturated brine, dried over anhydrous sodium sulfate, filtered, and concentrated. The residue is separated by column chromatography to obtain compounds of formula $I_A$ and $I_B$.

Synthesis of compound of formula Ic:

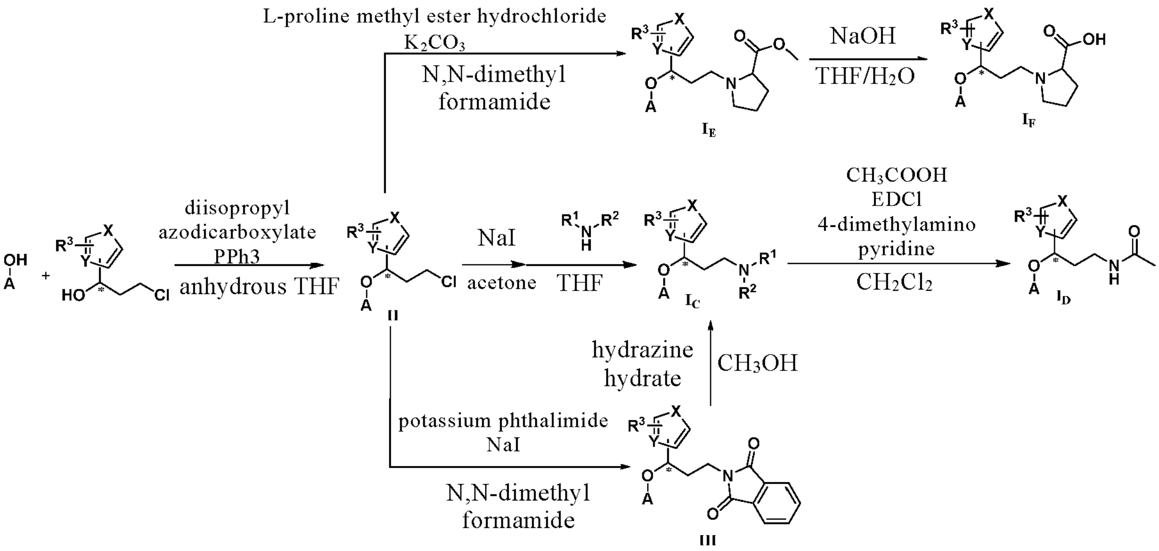


wherein, A, X, Y, $R^1$, $R^2$, $R^3$ and "*" are as defined above.

1) $C_6$-$C_{12}$ bicyclic heteroaryl phenol or phenol containing aliphatic ring, 1-($R^3$-5-membered heteroaryl)-3-chloropropanol and triphenylphosphine ($PPh_3$) are dissolved in anhydrous tetrahydrofuran (THF), and diisopropyl azodicarboxylate is slowly added dropwise into the system in ice bath. After the dropwise addition is completed, the reaction is carried out at 20-25°C overnight. After the reaction is completed, the system is directly spin-dried, and the residue is separated and purified by column chromatography to afford an intermediate of formula II.

2) The intermediate of formula II is dissolved in saturated sodium iodide acetone solution, and the reaction is carried out at 50-70°C overnight. After the reaction is completed, the solvent is spin-dried, and water is added into the system. The mixture is extracted three times with ethyl acetate, washed with saturated brine, dried with anhydrous sodium sulfate, filtered, and concentrated. The residue is dissolved in tetrahydrofuran solution, and 40% methylamine aqueous solution is added, the reaction is carried out at 20-25°C overnight. After the reaction is completed, the solvent was spin-dried, sodium hydroxide aqueous solution is added into the system; and the mixture is extracted three times with ethyl acetate, washed with saturated brine, dried with anhydrous sodium sulfate, filtered, and concentrated. The residue is separated by column chromatography to afford compound I.

3) The intermediate of formula II, potassium phthalimide and sodium iodide are dissolved in N,N-dimethylformamide, and the mixture is reacted at 70-90°C overnight. After the reaction is completed, water is added into the system, and the mixture is extracted three times with ethyl acetate, washed with water and saturated brine, dried with anhydrous sodium sulfate, filtered, and concentrated. The residue is separated by column chromatography to afford an intermediate of formula III.

4) The intermediate of formula III is dissolved in methanol solution, hydrazine hydrate is added, and the mixture is reacted at 20-25 °C overnight. After the reaction is completed, the solvent is spin-dried, and the residue is separated by column chromatography to afford compound I.

5) Acetic acid, 1-(3-dimethylaminopropyl)-3-ethylcarbodiimide hydrochloride, 4-dimethylaminopyridine and triethylamine are dissolved in anhydrous dichloromethane solution, the mixture is stirred at room temperature for one hour, the compound of formula Ic-23 in dichloromethane solution is added into the system, and the mixture is reacted at room temperature overnight. After the reaction is completed, water is added into the system, and the mixture is extracted three times with ethyl acetate, washed with water and saturated brine, dried with anhydrous sodium sulfate, filtered, and concentrated. The residue is separated by column chromatography to afford an intermediate of formula $I_D$.

6) The intermediate of formula II, L-proline methyl ester hydrochloride, potassium carbonate and sodium iodide are dissolved in acetonitrile solution, and the reaction is carried out at 60-80°C overnight under nitrogen protection. After the reaction is completed, water is added into the system, and the mixture is extracted three times with ethyl acetate, washed with water and saturated brine, dried with anhydrous sodium sulfate, filtered, and concentrated. The residue is separated by column chromatography to afford an intermediate of formula $I_E$.

7) Compound $I_E$ and sodium hydroxide are dissolved in a mixed solution of water and tetrahydrofuran, the reaction is carried out at 30-50°C overnight. After the reaction is completed, water is added into the system, the mixture is adjusted to pH=6-8 with diluted hydrochloric acid, extracted three times with ethyl acetate, washed with water and saturated brine, dried over anhydrous sodium sulfate, filtered, and concentrated to afford compound $I_F$.

**Synthesis of compound salt**

The compound of the present invention can be converted into its pharmaceutically acceptable salt by conventional methods. For example, a solution of corresponding acid can be added into the solution of above compounds, and the solvent is removed under reduced pressure after the salt is formed, thereby forming the corresponding salt of the compound of the present invention.

**Transient Receptor Potential Channel Protein (TRP)**

Transient receptor potential channel protein is a protein superfamily composed of important cation channels on the cell membrane. Transient receptor potential channel protein comprises multiple subfamily, such as TRPA, TRPC, TRPM, TRPV, TRPML and TRPP subfamily.

Studies have found that TRPA1 channel protein is related to a disease such as pain, epilepsy, inflammation, respiratory disorder, pruritus, urinary tract disorder, inflammatory bowel disease, etc. TRPA1 is a target useful for treating a disease such as pain, epilepsy, inflammation, respiratory disorder, pruritus, urinary tract disorder and inflammatory bowel diseases, etc.

Uses

The present invention further provides a method for inhibiting transient receptor potential channel protein (TPR), and a method for treating a disease related to transient receptor potential channel protein.

The compound of present invention can inhibit transient receptor potential channel protein, thereby preventing or treating a disease related to transient receptor potential channel protein.

In the present invention, the disease related to transient receptor potential channel protein is selected from: pain, epilepsy, inflammation, respiratory disorder, pruritus, urinary tract disorder, and inflammatory bowel disease. Representatively, the pain comprises (but is not limited to): acute inflammatory pain, chronic inflammatory pain, visceral pain, neurogenic pain, fibromyalgia, headache (such as migraine, muscle tension pain, etc.), neuralgia (such as trigeminal neuralgia, diabetic pain, post-zoster neuralgia, etc.), or pain caused by cancer.

In another aspect, the present invention provides a method for non-therapeutically and non-diagnostically inhibiting the activity of transient receptor potential channel protein *in vitro,* which comprises, e.g., in an *in vitro* culture system, contacting a transient receptor potential channel protein or a cell expressing the protein with the compound or a pharmaceutically acceptable salt thereof of the present invention, thereby inhibiting the activity of transient receptor potential channel protein.

**Pharmaceutical composition and administration method**

The invention provides a composition for inhibiting activity of transient receptor potential channel protein. The composition comprises (but is not limited to): pharmaceutical composition, food composition, dietary supplement, beverage composition, etc.

Typically, the composition is a pharmaceutical composition which comprises the compound , or a pharmaceutically acceptable salt thereof of the present invention; and a pharmaceutically acceptable carrier.

In the present invention, the dosage form of pharmaceutical composition comprises (but is not limited to) oral preparation, injection and external preparation.

Representatively, the dosage form comprises (but is not limited to): tablet, injection, infusion, ointment, gel, solution, microsphere, and film.

The term "pharmaceutically acceptable carrier" refers to one or more compatible solid, semi-solid, liquid or gel fillers, which are suitable for use in humans or animals and must have sufficient purity and sufficient low toxicity. The "compatible" means each ingredient of the pharmaceutical composition and active ingredient of the drug can be blended with each other without significantly reducing the efficacy.

It should be understood that the carrier is not particularly limited. In the present invention, the carrier can be selected from materials commonly used in the art, or can be obtained by a conventional method, or is commercially available. Some examples of pharmaceutically acceptable carriers are cellulose and its derivatives (such as methylcellulose, ethylcellulose, hydroxypropylmethylcellulose, sodium carboxymethylcellulose, etc.), gelatin, talc, solid lubricants (such as stearic acid, magnesium stearate), calcium sulfate, plant oil (such as soybean oil, sesame oil, peanut oil, olive oil, etc.), polyols (such as propylene glycol, glycerin, mannitol, sorbitol, etc.), emulsifier (such as Tween), wetting agent (such as sodium lauryl sulfate), buffer agent, chelating agent, thickener, pH regulator, transdermal enhancer, colorant, flavoring agent, stabilizer, antioxidant, preservative, bacteriostatic agent, pyrogen-free water, etc.

Representatively, in addition to the active pharmaceutical ingredient, the liquid formulations can contain inert

diluents commonly used in the art, such as water or other solvents, solubilizers and emulsifiers, for example, ethanol, isopropanol, ethyl carbonate, ethyl acetate, propylene glycol, 1,3-butanediol, dimethylformamide and oils, especially cottonseed oil, peanut oil, corn germ oil, olive oil, castor oil and sesame oil or a mixture thereof. In addition to these inert diluents, the composition can also contain adjuvants such as wetting agents, emulsifiers and suspensions and the like.

The pharmaceutical preparation should be matched with the mode of administration. The formulation of present invention can also be used together with other synergistic therapeutic agents (including before, simultaneous or after administering). When a pharmaceutical composition or preparation is administered, a safe and effective dose of drug is administered to a subject in need (e.g. human or non-human mammals). The safe and effective dose is usually at least 10 μg/kg body weight, and does not exceed about 8 mg/kg body weight in most cases, and preferably the dose is about 10 μg/kg body weight to about 1 mg/kg body weight. Of course, the route of administration, patient health and other factors, should also be taken into account to determine the specific dose, which are within the ability of the skilled physicians.

**The main advantages of the present invention include:**

(a) The present invention provides novel compounds which have excellent inhibitory activity on TRP channels.
(b) The compounds of the present invention have excellent *in vivo* efficacy as analgesia.
(c) The compounds of the present invention have less toxicity and higher activity, so that the safety window is larger.
(d) The compounds of the present invention have good druggability.
(e) The compounds of the present invention have excellent pharmacokinetic properties.
(f) The compounds of the present invention are suitable for oral administration.

The present invention will be further illustrated below with reference to the specific examples. The experimental methods with no specific conditions described in the following examples are generally performed under the conventional conditions, or according to the manufacturer's instructions. Unless indicated otherwise, parts and percentage are calculated by weight.

**Reference Example 1**

(S)-N-methyl-3-(quinolin-8-yloxy)-3-(thiophen-2-yl)propyl-1-amine (Compound $I_A$-1)

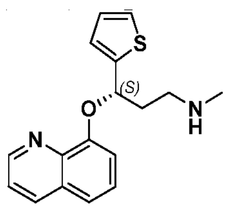

$I_A$-1

257 mg of (S)-3-(methylamino)-1-(thiophen-2-yl)propan-1-ol was dissolved in 10 ml of dimethyl sulfoxide solution, 168 mg of potassium tert-butoxide was added, the mixture was stirred at room temperature for 15 minutes, 883 mg of 8-fluoroquinoline was added, and the reaction was carried out at 90°C overnight. After the reaction was completed, water was added into the reaction system, the mixture was extracted three times with ethyl acetate, washed with water and saturated brine, dried over anhydrous sodium sulfate, filtered, and concentrated. The residue was separated by column chromatography (methanol/dichloromethane=1:15) to afford 217 mg of the title compound as brown oil. Yield: 48.5%.

$^1$H-NMR (400 MHz, $CDCl_3$) $\delta$ 8.97 (dd, *J* = 4.1, 1.3 Hz, 1H), 8.17 - 8.09 (m, 1H), 7.46 - 7.38 (m, 2H), 7.32 (t, *J* = 7.9 Hz, 1H), 7.24 (d, *J* = 4.9 Hz, 1H), 7.05 (d, *J* = 7.6 Hz, 1H), 7.01 (d, *J* = 3.2 Hz, 1H), 6.94 - 6.89 (m, 1H), 5.80 (dd, *J* = 8.6, 4.8 Hz, 1H), 3.01 (dt, *J* = 12.6, 6.9 Hz, 1H), 2.91 (dt, *J* = 12.2, 6.2 Hz, 1H), 2.60 (dq, *J* = 14.4, 6.2 Hz, 1H), 2.52 (s, 3H), 2.37 - 2.26 (m, 1H). MS (ESI, m/z): 298.88 $(M+H)^+$.

**Reference Example 2**

(S)-N-methyl-3-(quinolin-6-yloxy)-3-(thiophen-2-yl)propyl-1-amine (Compound $I_{A-2}$)

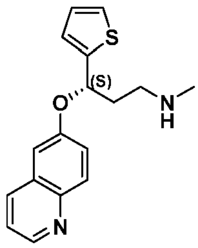

$I_A$-2

Except that 8-fluoroquinoline was replaced with 6-fluoroquinoline, the other required raw materials, reagents and preparation method were the same as Reference Example 1. 161 mg of the title compound as brown oil was obtained. Yield: 36.1%.

$^1$H-NMR (400 MHz, $CDCl_3$) $\delta$ 8.73 (dd, *J* = 4.1, 1.2 Hz, 1H), 7.96 (t, *J* = 7.5 Hz, 2H), 7.41 (dd, *J* = 9.2, 2.6 Hz, 1H), 7.30 (dd, *J* = 8.3, 4.2 Hz, 1H), 7.23 (d, *J* = 5.0 Hz, 1H), 7.14 (d, *J* = 2.6 Hz, 1H), 7.08 (d, *J* = 3.3 Hz, 1H), 6.94 (dd, *J* = 4.8, 3.7 Hz, 1H), 5.73 (dd, *J* = 7.4, 5.7 Hz, 1H), 2.83 - 2.74 (m, 2H), 2.45 (s, 3H), 2.37 (td, *J* = 14.0, 6.9 Hz, 1H), 2.16 (dq, *J* = 13.7, 6.9 Hz, 1H). MS (ESI, m/z): 298.88 $(M+H)^+$.

**Reference Example 3**

(S)-N-methyl-3-(quinolin-4-yloxy)-3-(thiophen-2-yl)propyl-1-amine (Compound $I_{A-3}$)

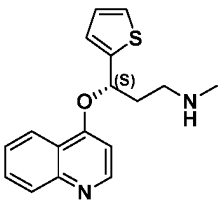

$I_A$-3

Except that 8-fluoroquinoline was replaced with 4-fluoroquinoline, the other required raw materials, reagents and preparation method were the same as Reference Example 1. 157 mg of the title compound as brown oil was obtained. Yield: 30.1%.

$^1$H-NMR (400 MHz, $CDCl_3$) $\delta$ 8.63 (d, *J* = 5.2 Hz, 1H), 8.09 (d, *J*= 8.5 Hz, 1H), 8.03 (dd, *J* = 8.6, 1.3 Hz, 1H), 7.66 (ddd, *J* = 8.4, 6.9, 1.4 Hz, 1H), 7.47 (ddd, *J* = 8.2, 6.8, 1.2 Hz, 1H), 7.25 (dd, *J* = 4.8, 1.7 Hz, 1H), 6.97 - 6.91 (m, 2H), 6.84 (d, *J* = 5.3 Hz, 1H), 5.09 (dd, *J* = 7.8, 5.1 Hz, 1H), 3.50 (hept, *J* = 6.9 Hz, 2H), 3.05 (s, 3H), 2.37 - 2.19 (m, 2H). MS (ESI, m/z): 298.88 $(M+H)^+$.

**Reference Example 4**

(S)-N-methyl-3-(quinolin-5-yloxy)-3-(thiophen-2-yl)propyl-1-amine (Compound $I_{A-4}$)

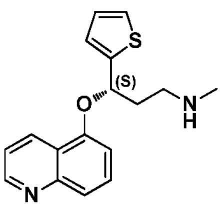

$I_A$-4

Except that 8-fluoroquinoline was replaced with 5-fluoroquinoline, the other required raw materials, reagents and preparation method were the same as Reference Example 1. 161 mg of the title compound as brown oil was obtained. Yield: 35.9%.

$^1$H-NMR (400 MHz, $CDCl_3$) $\delta$ 8.89 (dd, *J* = 4.2, 1.8 Hz, 1H), 8.65 (dt, *J* = 8.5, 1.3 Hz, 1H), 7.65 (d, *J* = 8.5 Hz, 1H), 7.50 (t, *J* = 8.1 Hz, 1H), 7.39 (dd, *J* = 8.5, 4.2 Hz, 1H), 7.22 (dd, *J* = 5.1, 1.2 Hz, 1H), 7.06 (dd, *J* = 3.6, 1.2 Hz, 1H), 6.97 - 6.88 (m, 2H), 5.81 (dd, *J* = 7.6, 5.4 Hz, 1H), 2.83 (t, *J* = 6.4 Hz, 2H), 2.54 - 2.38 (m, 4H), 2.24 (dtd, *J* = 14.0, 7.0, 5.3 Hz, 1H). MS (ESI, m/z): 298.88 (M+H)$^+$

**Reference Example 5**

(S)-N-methyl-3-(isoquinolin-4-yloxy)-3-(thiophen-2-yl)propyl-1-amine (Compound $I_{B-1}$)

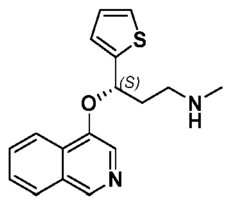

$I_B$-1

Except that 8-fluoroquinoline was replaced with 4-fluoroisoquinoline, the other required raw materials, reagents and preparation method were the same as Reference Example 1. 141 mg of the title compound as brown oil was obtained. Yield: 31.5%.

$^1$H-NMR (400 MHz, $CDCl_3$) $\delta$ 8.84 (s, 1H), 8.27 (d, *J* = 8.3 Hz, 1H), 8.12 (s, 1H), 7.91 (d, *J* = 8.2 Hz, 1H), 7.71 (t, *J* = 7.6 Hz, 1H), 7.61 (t, *J* = 7.5 Hz, 1H), 7.22 (d, *J* = 4.9 Hz, 1H), 7.11 (d, *J* = 3.3 Hz, 1H), 6.95 - 6.88 (m, 1H), 5.94 - 5.86 (m, 1H), 2.96 (q, *J* = 7.3 Hz, 1H), 2.90 (t, *J* = 7.0 Hz, 1H), 2.55 (dd, *J* = 14.0, 7.0 Hz, 1H), 2.50 (s, 3H), 2.32 (dd, *J* = 13.5, 6.2 Hz, 1H). MS (ESI, m/z): 298.88 (M+H)$^+$.

**Reference Example 6**

(S)-N-methyl-3-(isoquinolin-8-yloxy)-3-(thiophen-2-yl)propyl-1-amine (Compound $I_{B-2}$)

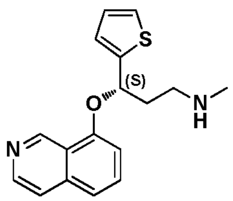

$I_B$-2

Except that 8-fluoroquinoline was replaced with 8-fluoroisoquinoline, the other required raw materials, reagents and preparation method were the same as Reference Example 1. 152 mg of the title compound as brown oil was obtained. Yield: 33.9%.

$^1$H-NMR (400 MHz, $CDCl_3$) $\delta$ 9.72 (s, 1H), 8.53 (d, *J* = 5.7 Hz, 1H), 7.56 (d, *J* = 5.7 Hz, 1H), 7.47 (t, *J* = 8.0 Hz, 1H), 7.33 (d, *J* = 8.2 Hz, 1H), 7.23 (d, *J* = 5.0 Hz, 1H), 7.09 (d, *J* = 3.4 Hz, 1H), 6.99 - 6.93 (m, 2H), 5.85 (dd, *J* = 7.5, 5.5 Hz, 1H), 2.84 (q, *J* = 6.4, 6.0 Hz, 2H), 2.54 - 2.47 (m, 1H), 2.45 (s, 3H), 2.24 (dq, *J* = 13.7, 6.9 Hz, 1H). MS (ESI, m/z): 298.88 (M+H)$^+$.

**Reference Example 7**

(S)-N-methyl-3-(isoquinolin-5-yloxy)-3-(thiophen-2-yl) propyl -1-amine (Compound $I_{B-3}$)

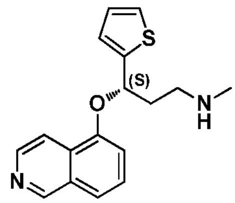

IB-3

Except that 8-fluoroquinoline was replaced with 5-fluoroisoquinoline, the other required raw materials, reagents and preparation method were the same as Reference Example 1, 163 mg of the title compound as brown oil was obtained. Yield: 36.4%.

$^1$H-NMR (400 MHz, $CDCl_3$) $\delta$ 9.17 (s, 1H), 8.53 (d, *J* = 5.9 Hz, 1H), 8.07 (d, *J* = 5.8 Hz, 1H), 7.50 (d, *J* = 8.2 Hz, 1H), 7.39 (t, *J* = 8.0 Hz, 1H), 7.22 (dd, *J* = 5.0, 1.2 Hz, 1H), 7.08 - 7.04 (m, 2H), 6.93 (dd, *J* = 5.1, 3.5 Hz, 1H), 5.83 (dd, *J* = 7.7, 5.4 Hz, 1H), 2.90 - 2.84 (m, 2H), 2.55 - 2.44 (m, 4H), 2.30 - 2.25 (m, 1H). MS (ESI, m/z): 298.88 $(M+H)^+$.

**Reference Example 8**

(S)-7-(3-chloro-1-(thiophen-2-yl)propoxy)benzofuran (Intermediate II-1)

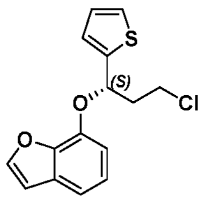

II-1

480 mg of (R)-3-chloro-1-(thiophen-2-yl)propan-1-ol, 364 mg of 7-hydroxybenzofuran and 784 mg of triphenylphosphine were dissolved in 20 ml of anhydrous tetrahydrofuran, and 589 μl of diisopropyl azodicarboxylate was slowly added dropwise into the system under ice bath condition. After the dropwise addition was completed, the system was shifted to room temperature and reacted overnight. After the reaction was completed, the system was directly spin-dried, and the residue was separated and purified by column chromatography to afford 600 mg of the title compound as colorless oil. Yield: 75.43%.

$^1$H NMR (500 MHz, $CDCl_3$) $\delta$ 7.61 (t, *J* = 3.1 Hz, 1H), 7.39 (dd, *J* = 1.7, 0.7 Hz, 1H), 7.21 (dt, *J* = 8.2, 1.9 Hz, 1H), 7.11 - 7.06 (m, 1H), 6.88 (d, *J* = 7.9 Hz, 1H), 6.76 (dd, *J* = 8.1, 2.1 Hz, 1H), 6.33 (d, *J* = 3.2 Hz, 1H), 6.30 (dd, *J* = 3.3, 1.8 Hz, 1H), 5.70 (dd, *J* = 8.4, 5.1 Hz, 1H), 3.89 (ddd, *J* = 11.1, 8.2, 5.4 Hz, 1H), 3.73 - 3.65 (m, 1H), 2.80 - 2.70 (m, 1H), 2.51 - 2.42 (m, 1H). MS (ESI, m/z): 293 $(M+H)^+$.

**Example 9**

(S)-3-(benzofuran-7-yloxy)-N-methyl-3-(thiophen-2-yl)propyl-1-amine (Compound $I_C$-1)

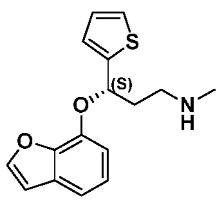

IC-1

600 mg of Intermediate II-1 was dissolved in saturated sodium iodide solution in acetone, and the mixture was refluxed overnight. After the reaction was completed, the solvent was spin-dried, water was added into the system, and the mixture was extracted three times with ethyl acetate, washed with saturated brine, dried with anhydrous sodium sulfate, filtered, and concentrated. The residue was dissolved in 20 ml of tetrahydrofuran solution, 2 ml of 40 % methylamine aqueous solution was added, and the reaction was carried out overnight. After the reaction was completed, the solvent was

spin-dried, sodium hydroxide aqueous solution was added into the system, then the mixture was extracted with ethyl acetate three times, washed with saturated brine, dried over anhydrous sodium sulfate, filtered, and concentrated. The residue was separated by column chromatography (methanol/dichloromethane=1:15) to afford 200 mg of the title compound as colorless oil. Yield: 33.96%.

$^1$H NMR (400 MHz, $CDCl_3$) $\delta$ 7.63 (d, $J$ = 2.0 Hz, 1H), 7.20 (t, $J$ = 6.6 Hz, 2H), 7.08 - 6.99 (m, 2H), 6.88 (dd, $J$ = 4.9, 3.6 Hz, 1H), 6.80 (d, $J$ = 7.9 Hz, 1H), 6.75 (d, $J$ = 2.0 Hz, 1H), 5.93 (dd, $J$ = 8.2, 4.4 Hz, 1H), 3.30 (t, $J$ = 7.0 Hz, 2H), 2.82 - 2.69 (m, 4H), 2.65 - 2.54 (m, 1H). MS (ESI, m/z): 287.87 $(M+H)^+$.

**Example 10**

(S)-3-(benzofuran-7-yloxy)-N-methyl-3-(thiophen-3-yl)propyl-1-amine (Compound Ic-2)

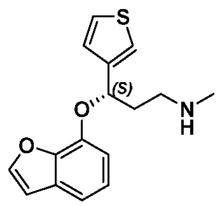

$I_C$-2

Except that (R)-3-chloro-1-(thiophen-2-yl)propyl-1-ol was replaced with (R)-3-chloro-1-(thiophen-3-yl)prop-1-ol, the other required raw materials, reagents and preparation method were the same as Reference Example 8 and Example 9. 200 mg of the title compound as yellow oil was obtained. Yield: 33.9%.

$^1$H NMR (500 MHz, $CDCl_3$) $\delta$ 7.63 (d, $J$ = 2.1 Hz, 1H), 7.27 (dd, $J$ = 5.0, 3.0 Hz, 1H), 7.24 (d, $J$ = 2.1 Hz, 1H), 7.18 - 7.11 (m, 2H), 7.01 (t, $J$ = 7.9 Hz, 1H), 6.75 (d, $J$ = 2.1 Hz, 1H), 6.72 (d, $J$ = 7.8 Hz, 1H), 5.64 (dd, $J$ = 8.0, 5.0 Hz, 1H), 2.94 - 2.81 (m, 2H), 2.48 (s, 3H), 2.46 - 2.32 (m, 1H), 2.24 - 2.13 (m, 1H). MS (ESI, m/z): 287.76 $(M+H)^+$.

**Example 11**

(S)-3-(benzofuran-7-yloxy)-N-methyl-3-(furan-3-yl)propyl-1-amine (Compound Ic-3)

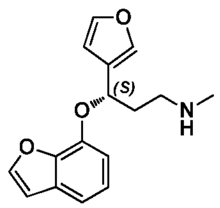

$I_C$-3

Except that (R)-3-chloro-1-(thiophen-2-yl)propyl-1-ol was replaced with (R)-3-chloro-1-(furan-3-yl)prop-1-ol, the other required raw materials, reagents and preparation method were the same as Reference Example 8 and Example 9. 100 mg of the title compound as colorless oil was obtained. Yield: 9.89%.

$^1$H NMR (500 MHz, $CDCl_3$) $\delta$ 7.62 (d, $J$ = 2.1 Hz, 1H), 7.40 (s, 1H), 7.35 (t, $J$ = 1.7 Hz, 1H), 7.17 (dd, $J$ = 7.8, 0.8 Hz, 1H), 7.05 (t, $J$ = 7.9 Hz, 1H), 6.80 (d, $J$ = 7.7 Hz, 1H), 6.75 (d, $J$ = 2.1 Hz, 1H), 6.46 (d, $J$ = 1.1 Hz, 1H), 5.56 (dd, $J$ = 7.7, 5.4 Hz, 1H), 2.90 - 2.78 (m, 2H), 2.46 (s, 3H), 2.35 (td, $J$ = 13.9, 7.4 Hz, 1H), 2.13 (dtd, $J$ = 12.4, 7.0, 5.5 Hz, 1H). MS (ESI, m/z): 271.88 $(M+H)^+$

**Example 12**

(S)-3-(benzofuran-7-yloxy)-N-methyl-3-(furan-2-yl)propyl-1-amine (Compound $I_C$-4

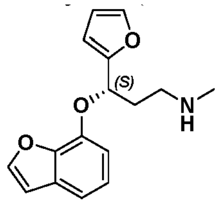

$I_C$-4

Except that (R)-3-chloro-1-(thiophen-2-yl)propyl-1-ol was replaced with (R)-3-chloro-1-(furan-2-yl)prop-1-ol, the other required raw materials, reagents and preparation method were the same as Reference Example 8 and Example 9. 30 mg of the title compound as colorless oil was obtained. Yield: 3.22%.

$^1$H NMR (500 MHz, $CDCl_3$) $\delta$ 7.61 (d, $J$ = 2.1 Hz, 1H), 7.36 (d, $J$ = 1.1 Hz, 1H), 7.20 (d, $J$ = 7.7 Hz, 1H), 7.06 (dd, $J$ = 10.5, 5.3 Hz, 1H), 6.82 (d, $J$ = 7.7 Hz, 1H), 6.74 (dd, $J$ = 7.8, 2.1 Hz, 1H), 6.31 (d, $J$ = 3.2 Hz, 1H), 6.27 (dd, $J$ = 3.2, 1.8 Hz, 1H), 5.60 (dd, $J$ = 7.9, 5.3 Hz, 1H), 3.13 - 2.99 (m, 2H), 2.63 - 2.56 (m, 4H), 2.44 (ddd, $J$ = 14.1, 12.4, 7.0 Hz, 1H). MS (ESI, m/z): 271.88 $(M+H)^+$.

**Example 13**

(S)-3-(benzofuran-7-yloxy)-N-methyl-3-(5-methylthiophen-2-yl)propyl-1-amine (Compound Ic-5)

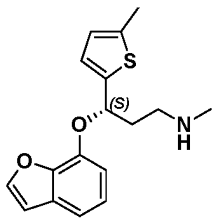

$I_C$-5

Except that (R)-3-chloro-1-(thiophen-2-yl)propyl-1-ol was replaced with (R)-3-chloro-1-(5-methylthiophen-2-yl) propyl -1-ol, the other required raw materials, reagents and preparation method were the same as Reference Example 8 and Example 9. 160 mg of the title compound as colorless oil was obtained. Yield: 14.24%.

$^1$H NMR (500 MHz, $CDCl_3$) $\delta$ 7.62 (d, $J$ = 2.1 Hz, 1H), 7.17 (dd, $J$ = 7.8, 0.8 Hz, 1H), 7.04 (t, $J$ = 7.9 Hz, 1H), 6.82 (d, $J$ = 7.6 Hz, 1H), 6.79 (d, $J$ = 3.4 Hz, 1H), 6.74 (dd, $J$ = 6.6, 2.2 Hz, 1H), 6.58 - 6.51 (m, 1H), 5.71 (dd, $J$ = 7.8, 5.5 Hz, 1H), 2.93 - 2.81 (m, 2H), 2.47 (s, 3H), 2.45 - 2.38 (m, 4H), 2.25 - 2.16 (m, 1H). MS (ESI, m/z): 301.87 $(M+H)^+$.

**Example 14**

(S)-3-(benzofuran-7-yloxy)-N-methyl-3-(5-chlorothiophen-2-yl)propyl-1-amine (Compound Ic-6)

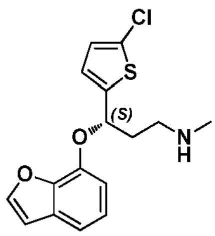

$I_C$-6

Except that (R)-3-chloro-1-(thiophen-2-yl)propyl-1-ol was replaced with (R)-3-chloro-1-(5-chlorothiophene-2-yl) propyl-1-ol, the other required raw materials, reagents and preparation method were the same as Reference Example 8 and Example 9. 300 mg of the title compound as colorless oil was obtained. Yield: 20.84%.

$^1$H NMR (500 MHz, $CDCl_3$) $\delta$ 7.63 (d, $J$ = 2.1 Hz, 1H), 7.20 (d, $J$ = 7.8 Hz, 1H), 7.05 (t, $J$ = 7.9 Hz, 1H), 6.80 (dd, $J$ = 12.0, 5.7 Hz, 2H), 6.75 (t, $J$ = 4.6 Hz, 1H), 6.70 (d, $J$ = 3.8 Hz, 1H), 5.75 (dd, $J$ = 8.1, 5.2 Hz, 1H), 3.04 - 2.89 (m, 2H), 2.57 - 2.43 (m, 4H), 2.27 (ddd, $J$ = 13.7, 12.0, 6.7 Hz, 1H). MS (ESI, m/z): 321.78 $(M+H)^+$.

**Example 15**

(S)-3-(benzofuran-7-yloxy)-N-methyl-3-(thiazol-2-yl)propyl-1-amine (Compound Ic-7)

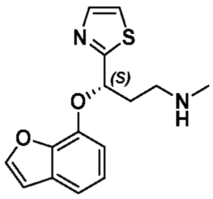

$I_C$-7

Except that (R)-3-chloro-1-(thiophen-2-yl)propyl-1-ol was replaced with 3-chloro-1-(thiazol-2-yl)propyl-1-ol, the other required raw materials, reagents and preparation method were the same as Reference Example 8 and Example 9. After racemate of compound Ic-7 was obtained, 60 mg of the title compound as yellow oil was obtained by chiral resolution. Yield: 6.72%.

$^1$H NMR (500 MHz, $CDCl_3$) $\delta$ 7.76 (d, *J* = 3.2 Hz, 1H), 7.64 (d, *J* = 2.1 Hz, 1H), 7.33 (d, *J* = 3.2 Hz, 1H), 7.25 (d, *J* = 0.9 Hz, 1H), 7.08 (t, *J* = 7.9 Hz, 1H), 6.89 (d, *J* = 7.3 Hz, 1H), 6.78 (d, *J* = 2.2 Hz, 1H), 6.05 (t, *J* = 6.0 Hz, 1H), 3.33 (t, *J* = 6.9 Hz, 2H), 2.81 - 2.67 (m, 5H). MS (ESI, m/z): 288.77 $(M+H)^+$.

**Example 16**

(S)-3-(benzofuran-7-yloxy)-N,N-dimethyl-3-(thiophen-2-yl)propyl-1-amine (compound Ic-8)

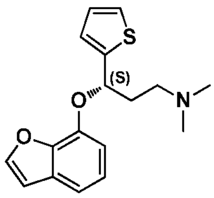

$I_C$-8

Except that methylamine aqueous solution was replaced with dimethylamine, the other required raw materials, reagents and preparation method were the same as Example 9. 130 mg of the title compound as colorless oil was obtained. Yield: 34.67%.

$^1$H NMR (400 MHz, $CDCl_3$) $\delta$ 7.62 (d, *J* = 2.0 Hz, 1H), 7.23 (d, *J* = 5.0 Hz, 1H), 7.17 (d, *J* = 7.8 Hz, 1H), 7.03 (t, *J* = 7.9 Hz, 2H), 6.96 - 6.87 (m, 1H), 6.82 (d, *J* = 7.9 Hz, 1H), 6.74 (d, *J* = 2.1 Hz, 1H), 5.88 - 5.79 (m, 1H), 2.60 (t, *J* = 6.9 Hz, 2H), 2.47 (dt, *J* = 21.7, 7.4 Hz, 1H), 2.32 (s, 6H), 2.22 (dt, *J* = 20.5, 6.8 Hz, 1H). MS (ESI, m/z): 301.88 $(M+H)^+$.

**Example 17**

(S)-3-(benzofuran-4-yloxy)-N-methyl-3-(thiophen-2-yl)propyl-1-amine (compound $I_C$-9)

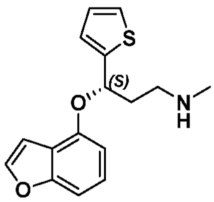

$I_C$-9

Except that 7-hydroxybenzofuran was replaced with 4-hydroxybenzofuran, the other required raw materials, reagents and preparation method were the same as Reference Example 8 and Example 9. 15 mg of the title compound as

yellow oil was obtained. Yield: 1.40%.

$^1$H NMR (400 MHz, $CDCl_3$) $\delta$ 7.53 (d, $J$ = 2.1 Hz, 1H), 7.20 (d, $J$ = 5.0 Hz, 1H), 7.14 - 7.07 (m, 3H), 6.92 - 6.87 (m, 2H), 6.72 (dd, $J$ = 6.6, 2.1 Hz, 1H), 5.87 (dd, $J$ = 7.7, 4.8 Hz, 1H), 3.34 - 3.19 (m, 2H), 2.78 (td, $J$ = 14.6, 7.3 Hz, 1H), 2.66 (dt, $J$ = 15.1, 6.4 Hz, 4H). MS (ESI, m/z): 287.87 $(M+H)^+$.

**Example 18**

(S)-3-(2,3-dihydro-1H-inden-4-yloxy)-N-methyl-3-(2-thienyl)propyl-1-amine (Compound $I_C$-10)

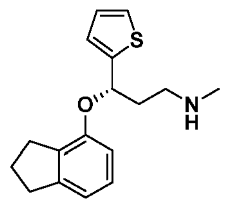

$I_C$-10

Except that 7-hydroxybenzofuran was replaced with 4-indanol, the other required raw materials, reagents and preparation method were the same as Reference Example 8 and Example 9. 16 mg of the title compound as brown oil was obtained. Yield: 9.9%.

$^1$H NMR (400 MHz, $CDCl_3$) $\delta$ 7.21 (dd, $J$ = 5.0, 1.2 Hz, 1H), 7.03 - 6.96 (m, 2H), 6.92 (dd, $J$ = 5.0, 3.5 Hz, 1H), 6.81 (d, $J$ = 7.3 Hz, 1H), 6.65 (d, $J$ = 8.0 Hz, 1H), 5.57 (dd, $J$ = 7.8, 5.0 Hz, 1H), 2.87 (dt, $J$ = 19.9, 7.3 Hz, 6H), 2.47 (s, 3H), 2.41 - 2.30 (m, 1H), 2.25 - 2.14 (m, 1H), 2.06 (p, $J$ = 7.0 Hz, 2H). MS (ESI, m/z): 287.87 $(M+H)^+$.

**Example 19**

(S)-3-(2,3-dihydro-1H-inden-4-yloxy)-N-methyl-3-(3-thienyl)propyl-1-amine (Compound Ic-11)

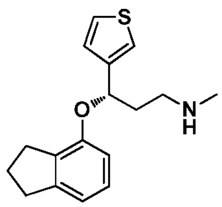

$I_C$-11

Except that (R)-3-chloro-1-(thiophen-2-yl)propyl-1-ol was replaced with (R)-3-chloro-1-(thiophen-3-yl)propyl-1-ol, the other required raw materials, reagents and preparation method were the same as Example 18. 60 mg of the title compound as brown oil was obtained. Yield: 8.6%.

$^1$H NMR (500 MHz, $CDCl_3$) $\delta$ 7.27 (dd, $J$ = 4.0, 2.0 Hz, 1H), 7.20 - 7.15 (m, 1H), 7.06 (dt, $J$ = 3.8, 1.9 Hz, 1H), 6.96 (t, $J$ = 7.9 Hz, 1H), 6.80 (d, $J$ = 7.6 Hz, 1H), 6.54 (d, $J$ = 8.2 Hz, 1H), 5.40 (dd, $J$ = 7.9, 4.7 Hz, 1H), 2.91 (dd, $J$ = 16.1, 8.4 Hz, 4H), 2.82 (ddd, $J$ = 9.8, 7.1, 3.8 Hz, 2H), 2.47 (s, 3H), 2.31 - 2.23 (m, 1H), 2.19 - 2.11 (m, 1H), 2.11 - 2.03 (m, 2H). MS (ESI, m/z): 287.76 $(M+H)^+$.

**Example 20**

(S)-3-(2,3-dihydro-1H-inden-4-yloxy)-N-methyl-3-(2-furyl)propyl-1-amine (Compound Ic-12)

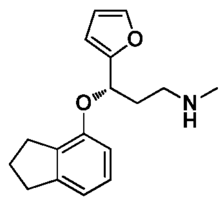

$I_C$-12

Except that (R)-3-chloro-1-(thiophen-2-yl)propyl-1-ol was replaced with (R)-3-chloro-1-(furan-2-yl)propyl-1-ol, the other required raw materials, reagents and preparation method were the same as Example 18. 10 mg of the title compound as brown oil was obtained. Yield: 4.2%.

$^1$H NMR (500 MHz, $CDCl_3$) $\delta$ 7.36 (dd, *J* = 1.8, 0.8 Hz, 1H), 7.03 (dd, *J* = 14.9, 7.1 Hz, 1H), 6.83 (d, *J* = 7.4 Hz, 1H), 6.67 (d, *J* = 8.1 Hz, 1H), 6.30 (dd, *J* = 3.2, 1.8 Hz, 1H), 6.25 (d, *J* = 3.2 Hz, 1H), 5.30 (dd, *J* = 7.6, 5.4 Hz, 1H), 2.92 - 2.79 (m, 6H), 2.48 (s, 3H), 2.35 (td, *J* = 14.0, 7.2 Hz, 1H), 2.23 (ddd, *J* = 14.0, 12.5, 7.1 Hz, 1H), 2.08 - 2.01 (m, 2H). MS (ESI, m/z): 271.88 $(M+H)^+$.

**Example 21**

(S)-3-(2,3-dihydro-1H-inden-4-yloxy)-N-methyl-3-(3-furyl)propyl-1-amine (Compound Ic-13)

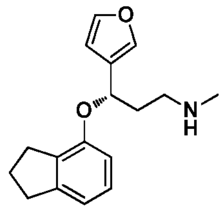

$I_C$-13

Except that (R)-3-chloro-1-(thiophen-2-yl)propyl-1-ol was replaced with (R)-3-chloro-1-(furan-3-yl)propyl-1-ol, the other required raw materials, reagents and preparation method were the same as Example 18. 13 mg of the title compound as brown oil was obtained. Yield: 4.6%.

$^1$H NMR (500 MHz, $CDCl_3$) $\delta$ 7.35 (d, *J*= 1.4 Hz, 2H), 7.01 (t, *J* = 7.8 Hz, 1H), 6.81 (d, *J* = 7.4 Hz, 1H), 6.63 (d, *J* = 8.1 Hz, 1H), 6.38 (t, *J* = 1.3 Hz, 1H), 5.29 (dd, *J* = 7.7, 5.0 Hz, 1H), 2.94 - 2.85 (m, 4H), 2.83 - 2.75 (m, 2H), 2.46 (s, 3H), 2.22 (td, *J* = 13.9, 7.4 Hz, 1H), 2.11 - 2.00 (m, 3H). MS (ESI, m/z): 271.63 $(M+H)^+$.

**Example 22**

(S)-3 -(2,3 -dihydro- 1H-inden-4-yloxy)-N-methyl-3 -(2-thiazolyl)propyl-1 -amine (Compound $I_C$-14

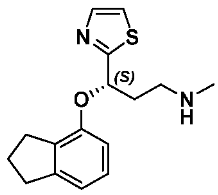

$I_C$-14

Except that (R)-3-chloro-1-(thiophen-2-yl)propyl-1-ol was replaced with (R)-3-chloro-1-(thiazol-3-yl)propyl-1-ol, the other required raw materials, reagents and preparation method were the same as Example 18. After racemate of compound $I_C$-14 was obtained, 35 mg of the title compound as yellow oil was obtained by chiral resolution. Yield: 5.9%.

$^1$H NMR (400 MHz, $CDCl_3$) $\delta$ 7.74 (d, *J* = 3.2 Hz, 1H), 7.28 (d, *J* = 3.2 Hz, 1H), 7.01 (t, *J* = 7.8 Hz, 1H), 6.85 (d, *J* = 7.4 Hz, 1H), 6.65 (d, *J* = 8.1 Hz, 1H), 5.70 (dd, *J* = 7.3, 5.2 Hz, 1H), 3.01 (d, *J* = 7.5 Hz, 1H), 2.98 - 2.82 (m, 5H), 2.48 (s, 3H), 2.44 - 2.24 (m, 2H), 2.14 - 2.02 (m, 2H). MS (ESI, m/z): 288.87 $(M+H)^+$

**Example 23**

(S)-3-(2,3-dihydro-1H-inden-4-yloxy)-N,N-dimethyl-3-(2-thienyl)propyl-1-amine (Compound Ic-15)

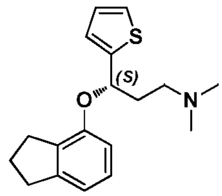

Ic-15

Except that methylamine aqueous solution was replaced with dimethylamine, the other required raw materials, reagents and preparation method were the same as Example 18. 21 mg of the title compound as brown oil was obtained. Yield: 10.1%.

$^1$H NMR (400 MHz, $CDCl_3$) δ 7.22 (dd, *J* = 5.0, 1.2 Hz, 1H), 7.00 (dd, *J* = 10.5, 5.2 Hz, 2H), 6.93 (dd, *J* = 5.0, 3.5 Hz, 1H), 6.82 (d, *J* = 7.5 Hz, 1H), 6.65 (d, *J* = 8.1 Hz, 1H), 5.57 (dd, *J* = 7.6, 5.3 Hz, 1H), 2.90 (dt, *J* = 20.0, 6.5 Hz, 4H), 2.76 (s, 2H), 2.57 - 2.36 (m, 7H), 2.30 (s, 1H), 2.13 - 1.98 (m, 2H). MS (ESI, m/z): 301.77 $(M+H)^+$.

**Example 24**

(S)-3-(2,3-dihydro-1H-inden-5-yloxy)-N-methyl-3-(2-thienyl)propyl-1-amine (Compound Ic-16)

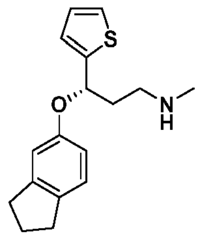

Ic-16

Except that 7-hydroxybenzofuran was replaced with 5-indanol, the other required raw materials, reagents and preparation method were the same as Reference Example 8 and Example 9. 15 mg of the title compound as yellow oil was obtained. Yield: 9.7%.

$^1$H NMR (400 MHz, $CDCl_3$) δ 7.21 (dd, *J* = 5.0, 1.1 Hz, 1H), 7.06 - 6.98 (m, 2H), 6.91 (dd, *J* = 5.0, 3.5 Hz, 1H), 6.78 (s, 1H), 6.68 (d, *J* = 8.6 Hz, 1H), 5.56 (dd, *J* = 7.8, 4.6 Hz, 1H), 3.08 (t, *J* = 7.3 Hz, 2H), 2.79 (dt, *J* = 12.2, 7.4 Hz, 4H), 2.61 (s, 3H), 2.03 (q, *J* = 7.6 Hz, 4H). MS (ESI, m/z): 287.88 $(M+H)^+$.

**Example 25**

(S)-(3 -(tetrahydronaphthalen-1-yl)oxy)-N-methyl-3-(2-thienyl)propyl-1-amine (Compound Ic-17)

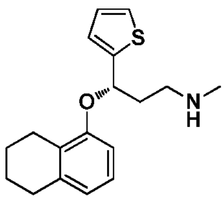

Ic-17

Except that 7-hydroxybenzofuran was replaced with tetrahydronaphthol, the other required raw materials, reagents and preparation method were the same as Reference Example 8 and Example 9. 30 mg of the title compound as

yellow oil was obtained. Yield: 13.6%.

$^1$H NMR (400 MHz, $CDCl_3$) δ 7.19 - 7.14 (m, 1H), 6.98 - 6.93 (m, 2H), 6.67 (d, *J* = 7.9 Hz, 1H), 6.53 (d, *J* = 8.0 Hz, 1H), 4.82 (dd, *J* = 12.5, 3.4 Hz, 1H), 2.83 - 2.64 (m, 5H), 2.56 - 2.31 (m, 5H), 2.12 (m, 1H), 1.84 - 1.66 (m, 4H). MS (ESI, m/z): 301.77 $(M+H)^+$.

**Example 26**

(S)-(3-(5,8-dihydronaphthalen-1-yl)oxy)-N-methyl-3-(2-thienyl)propy1-1-amine (Compound Ic-18)

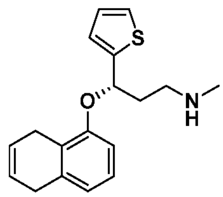

$I_C$-18

Except that 7-hydroxybenzofuran was replaced with 5,8-dihydronaphthol, the other required raw materials, reagents and preparation method were the same as Reference Example 8 and Example 9. 65 mg of the title compound as brown oil was obtained. Yield: 25.3%.

$^1$H NMR (400 MHz, DMSO) δ 9.31 (s, 1H), 7.32 (dd, *J* = 5.1, 1.2 Hz, 1H), 7.02 (d, *J* = 8.4 Hz, 1H), 6.93 (dd, *J* = 5.1, 3.5 Hz, 1H), 6.88 (d, *J* = 3.1 Hz, 1H), 6.70 (d, *J* = 8.3 Hz, 1H), 5.85 (dd, *J* = 22.1, 10.2 Hz, 2H), 4.38 (t, *J* = 7.6 Hz, 1H), 3.42 (s, 1H), 3.15 (d, *J* = 10.1 Hz, 4H), 2.91 - 2.68 (m, 3H), 2.57 - 2.52 (m, 3H), 2.30 - 2.20 (m, 2H). MS (ESI, m/z): 299.76 $(M+H)^+$.

**Example 27**

(S)-2-methyl-4-(3-(methylamino)-1-(thiophen-2-yl)propoxy)isoindoline-1,3-dione (Compound $I_C$-19

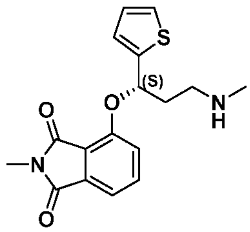

$I_C$-19

Except that 7-hydroxybenzofuran was replaced with 4-hydroxy-2-methylisoindoline-1,3-dione, the other required raw materials, reagents and preparation method were the same as Reference Example 8 and Example 9. 163 mg of the title compound as brown oil was obtained. Yield: 36.4%.

$^1$H NMR (400 MHz, $CDCl_3$) δ 7.52 (t, *J* = 7.8 Hz, 1H), 7.43 (d, *J* = 7.3 Hz, 1H), 7.28 (d, *J* = 4.9 Hz, 1H), 7.04 (dd, *J* = 11.3, 5.6 Hz, 2H), 6.97 (dd, *J* = 4.9, 3.4 Hz, 1H), 6.05 (s, 1H), 3.34 (s, 2H), 3.14 (s, 3H), 3.02 (s, 1H), 2.88 (s, 3H), 2.43 (d, *J* = 15.0 Hz, 1H). MS (ESI, m/z): 330.76 $(M+H)^+$.

**Example 28**

(S)-3-(imidazo[1,2-*a*]pyridine-8-oxy)-N-methyl-3-(2-thienyl)propyl-1-amine (compound Ic-20)

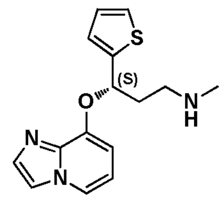

Ic-20

Except that 7-hydroxybenzofuran was replaced with 8-hydroxyimidazo[1,2-a]pyridine, the other required raw materials, reagents and preparation method were the same as Reference Example 8 and Example 9. 43 mg of the title compound as brown oil was obtained. Yield: 11.7%.

$^1$H NMR (400 MHz, $CDCl_3$) $\delta$ 7.95 (d, *J* = 6.4 Hz, 1H), 7.64 (s, 1H), 7.53 (d, *J*= 11.9 Hz, 1H), 7.35 - 7.28 (m, 1H), 6.91 (d, *J* = 3.4 Hz, 2H), 6.69 (t, *J* = 7.1 Hz, 1H), 6.62 (d, *J* = 7.2 Hz, 1H), 5.85 (dd, *J* = 11.5, 2.7 Hz, 1H), 3.55 (t, *J* = 10.1 Hz, 1H), 3.34 - 3.24 (m, 1H), 2.85 (s, 3H), 2.78 (dd, *J* = 11.1, 3.3 Hz, 1H), 2.64 - 2.52 (m, 1H). MS (ESI, m/z): 287.77 $(M+H)^+$.

**Example 29**

(S)-4-(3-(methylamino)-1-(thiophen-2-yl)propoxy)-1,3-dihydro-2H benzo[*d*]imidazol-2-one (compound $I_C$-21)

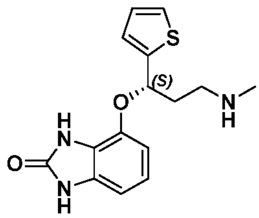

Ic-21

Except that 7-hydroxybenzofuran was replaced with 4-hydroxy-1H-benzo[d]imidazole-2(3H)-one, the other required raw materials, reagents and preparation method were the same as Reference Example 8 and Example 9. 43 mg of the title compound as brown oil was obtained. Yield: 9.4%.

$^1$H NMR (400 MHz, $CD_3OD$) $\delta$ 7.35 (dd, *J* = 5.1, 1.1 Hz, 1H), 7.11 (d, *J* = 2.8 Hz, 1H), 6.95 (dd, *J* = 5.1, 3.5 Hz, 1H), 6.88 - 6.81 (m, 1H), 6.67 (dd, *J* = 8.1, 2.8 Hz, 2H), 5.77 (dd, *J* = 8.1, 4.8 Hz, 1H), 3.11 - 2.98 (m, 2H), 2.60 (s, 3H), 2.50 - 2.39 (m, 1H), 2.32 - 2.23 (m, 1H). MS (ESI, m/z): 303.88 $(M+H)^+$.

**Example 30**

(S)-N-methyl-3-(quinoxalin-5-yloxy)-3-(thiophen-2-yl)- 1-amine (Compound Ic-22)

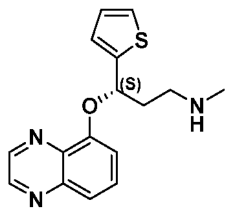

Ic-22

Except that 7-hydroxybenzofuran was replaced with 5-fluoroquinoxaline, the other required raw materials, reagents and preparation method were the same as Reference Example 8 and Example 9. 230 mg of the title compound as brown oil was obtained. Yield: 40.5%.

$^1$H NMR (400 MHz, $CDCl_3$) $\delta$ 8.98 (d, *J* = 11.6 Hz, 2H), 7.83 (d, *J* = 8.5 Hz, 1H), 7.58 (t, *J*= 8.1 Hz, 1H), 7.35 (d, *J* = 4.7 Hz, 1H), 7.05 - 6.95 (m, 3H), 5.67 (d, *J* = 6.1 Hz, 1H), 3.45 (s, 2H), 2.93 (s, 3H), 2.76 (s, 1H), 2.64 (s, 1H). MS (ESI, m/z): 299.88 $(M+H)^+$.

**Reference Example 31**

(S)-2-(3-(benzofuran-7-yloxy)-3-(thiophen-2-yl)propyl)isoindoline-1,3-dione (Intermediate III-1)

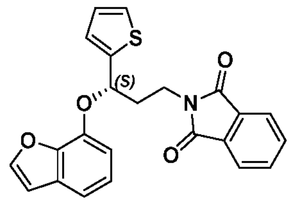

III-1

160 mg of Intermediate II-1, 303 mg of potassium phthalimide and 25 mg of sodium iodide were dissolved in 5 ml of N,N-dimethylformamide, and the reaction was carried out at 90°C under nitrogen protection overnight. After the reaction was completed, water was added into the system, and the mixture was extracted three times with ethyl acetate, washed with water and saturated brine, dried over anhydrous sodium sulfate, filtered, and concentrated. The residue was separated by column chromatography (ethyl acetate/petroleum ether=1: 5) to afford 140 mg of the title compound as yellow solid. Yield: 63.49%.

$^1$H NMR (500 MHz, $CDCl_3$) $\delta$ 7.81-7.78 (m, 2H), 7.68 (dd, *J* = 5.5, 3.0 Hz, 2H), 7.45 (d, *J* = 2.1 Hz, 1H), 7.18 (dd, *J* = 5.0, 1.1 Hz, 1H), 7.14 (dd, *J* = 7.8, 0.8 Hz, 1H), 7.06 (d, *J* = 3.0 Hz, 1H), 7.04 - 6.97 (m, 1H), 6.86 (dt, *J* = 10.3, 5.2 Hz, 1H), 6.78 (d, *J* = 7.4 Hz, 1H), 6.68 (d, *J* = 2.1 Hz, 1H), 5.82 (dd, *J* = 7.8, 5.2 Hz, 1H), 4.07 - 3.89 (m, 2H), 2.66 (td, *J* = 14.4, 7.3 Hz, 1H), 2.47 - 2.36 (m, 1H). MS (ESI, m/z): 404 $(M+H)^+$.

**Example 32**

(S)-3-(benzofuran-7-yloxy)-3-(thiophen-2-yl) propyl -1-amine (compound Ic-23)

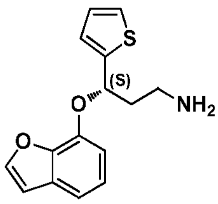

$I_C$-23

140 mg of Intermediate III-1 and 87 mg of hydrazine hydrate were dissolved in 5 ml of methanol solution, and the reaction was carried out at room temperature overnight. After the reaction was completed, the solvent was spin-dried, and the residue was separated by column chromatography (methanol/dichloromethane=1:15) to afford 30 mg of the title compound as colorless oil. Yield: 31.63%.

$^1$H NMR (500 MHz, DMSO) $\delta$ 7.97 (d, *J* = 2.1 Hz, 1H), 7.49 (dd, *J* = 5.0, 1.1 Hz, 1H), 7.24 - 7.17 (m, 2H), 7.11 - 7.03 (m, 1H), 6.99 (dd, *J* = 5.0, 3.5 Hz, 1H), 6.95 (d, *J* = 7.5 Hz, 1H), 6.93 (d, *J* = 2.1 Hz, 1H), 6.04 (dd, *J* = 7.8, 5.5 Hz, 1H), 2.99 - 2.86 (m, 2H), 2.44 - 2.35 (m, 1H), 2.21 (ddt, *J* = 11.5, 9.3, 5.8 Hz, 1H). MS (ESI, m/z): 273.77 $(M+H)^+$.

**Example 33**

(S)-3-(benzofuran-7-yloxy)-3-(furan-3-yl)propyl-1-amine (compound Ic-24)

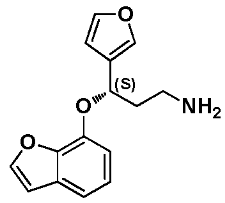

$I_C$-24

Except that (R)-3-chloro-1-(thiophen-2-yl)propyl-1-ol was replaced with (R)-3-chloro-1-(furan-3-yl)propyl-1-ol, the other required raw materials, reagents and preparation method were the same as Reference Examples 8 and 31 and Example 32. 90 mg of the title compound as colorless oil was obtained. Yield: 12.71%.

$^1$H NMR (500 MHz, $CDCl_3$) $\delta$ 7.62 (d, *J* = 2.1 Hz, 1H), 7.42 - 7.37 (m, 1H), 7.35 (t, *J* = 1.7 Hz, 1H), 7.18 (dd, *J* = 7.8, 0.9 Hz, 1H), 7.08 - 7.01 (m, 1H), 6.79 (dd, *J* = 7.9, 0.7 Hz, 1H), 6.75 (d, *J* = 2.2 Hz, 1H), 6.45 (dd, *J* = 1.7, 0.7 Hz, 1H), 5.57 (dd, *J* = 8.1, 5.0 Hz, 1H), 3.03 - 2.91 (m, 2H), 2.31 (ddt, *J* = 14.1, 8.1, 6.4 Hz, 1H), 2.06 (dtd, *J* = 9.4, 7.1, 5.1 Hz, 1H). MS (ESI, m/z): 257.77(M+H)$^+$.

**Example 34**

(S)-3-(benzofuran-7-yloxy)-3-(furan-2-yl) propyl-1-amine (compound $I_C$-25

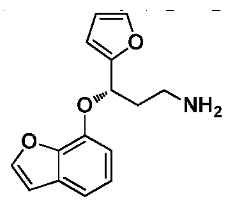

$I_C$-25

Except that (R)-3-chloro-1-(thiophen-2-yl)propyl-1-ol was replaced with (R)-3-chloro-1-(furan-2-yl)propyl-1-ol, the other required raw materials, reagents and preparation method were the same as Reference Examples 8 and 31 and Example 32. 85 mg of the title compound as colorless oil was obtained. Yield: 16.53%.

1H NMR (500 MHz, $CDCl_3$) $\delta$ 7.62 (d, J = 2.1 Hz, 1H), 7.37 (dd, J = 1.6, 1.0 Hz, 1H), 7.20 (dd, J = 7.8, 0.9 Hz, 1H), 7.06 (t, J = 7.9 Hz, 1H), 6.87 - 6.80 (m, 1H), 6.75 (d, J = 2.1 Hz, 1H), 6.33 - 6.24 (m, 2H), 5.59 (dd, J = 8.0, 5.5 Hz, 1H), 3.09 - 2.93 (m, 2H), 2.43 (dq, J = 7.9, 6.4 Hz, 1H), 2.22 (qd, J = 12.5, 6.9 Hz, 1H). MS (ESI, m/z): 257.64(M+H)$^+$.

**Example 35**

(S)-3(benzofuran-7-yloxy)-3-(thiophen-3-yl) propyl -1-amine (compound Ic-26)

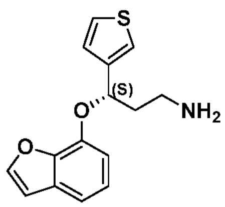

$I_C$-26

Except that (R)-3-chloro-1-(thiophen-2-yl)propyl-1-ol was replaced with (R)-3-chloro-1-(thiophen-3-yl)propyl-1-ol, the other required raw materials, reagents and preparation method were the same as Reference Example 8 and 31 and Example 32. 60 mg of the title compound as colorless oil was obtained. Yield: 14.56%.

1H NMR (500 MHz, $CDCl_3$) $\delta$ 7.62 (d, J = 1.9 Hz, 1H), 7.25 - 7.22 (m, 2H), 7.13 (t, J = 7.4 Hz, 1H), 7.09 (d, J = 4.6 Hz, 1H), 6.97 (t, J = 7.9 Hz, 1H), 6.71 (d, J = 2.0 Hz, 1H), 6.67 (d, J = 8.0 Hz, 1H), 5.65 (dd, J = 7.9, 4.2 Hz, 1H), 3.30 - 3.11 (m, 2H), 2.49 (dd, J = 14.1, 7.4 Hz, 1H), 2.34 (dd, J = 12.9, 5.8 Hz, 1H). MS (ESI, m/z): 273.77(M+H)$^+$.

**Example 36**

(S)-3-(benzo[b]thiophen-5-yloxy)-N-methyl-3-(thiophen-2-yl)propyl-1-amine (compound $I_C$-27

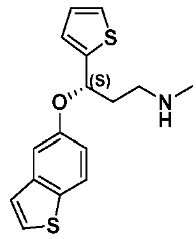

Ic-27

Except that 7-hydroxybenzofuran was replaced with benzo[b]thiophen-5-ol, the other required raw materials, reagents and preparation method were the same as Reference Example 8 and Example 9. 20 mg of the title compound as yellow oil was obtained. Yield: 3.30%.

1H NMR (500 MHz, $CDCl_3$) $\delta$ 7.68 (d, J = 8.8 Hz, 1H), 7.39 (d, J = 5.4 Hz, 1H), 7.31 (d, J = 2.4 Hz, 1H), 7.22 (dd, J = 5.0, 1.1 Hz, 1H), 7.18 (d, J = 5.4 Hz, 1H), 7.04 - 6.99 (m, 2H), 6.92 (dd, J = 5.0, 3.5 Hz, 1H), 5.63 (dd, J = 7.7, 5.2 Hz, 1H), 2.93 - 2.81 (m, 2H), 2.49 (s, 3H), 2.39 (tt, J = 12.5, 6.3 Hz, 1H), 2.22 (ddd, J = 14.0, 12.4, 7.0 Hz, 1H). MS (ESI, m/z): 303.76$(M+H)^+$.

**Example 37**

(S)-3-(benzofuran-4-yloxy)-N-methyl-3-(furan-3-yl)-propyl-1-amine (compound Ic-28)

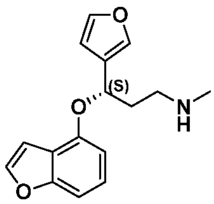

Ic-28

Except that (R)-3-chloro-1-(thiophen-2-yl)propyl-1-ol was replaced with (R)-3-chloro-1-(furan-3-yl)propan-1-ol, the other required raw materials, reagents and preparation method were the same as Example 17. 40 mg of the title compound as colorless oil was obtained. Yield: 5.08%.

$^1H$ NMR (500 MHz, $CDCl_3$) $\delta$ 7.52 (dd, *J* = 10.8, 2.2 Hz, 1H), 7.41 (d, *J* = 0.7 Hz, 1H), 7.35 (t, *J* = 1.7 Hz, 1H), 7.15 - 7.07 (m, 2H), 6.87 (d, *J* = 2.1 Hz, 1H), 6.70 - 6.64 (m, 1H), 6.44 - 6.39 (m, 1H), 5.49 (dd, *J* = 7.6, 5.0 Hz, 1H), 2.95 (t, *J* = 7.2 Hz, 2H), 2.53 (s, 3H), 2.42 (td, *J* = 14.3, 7.5 Hz, 1H), 2.30 - 2.23 (m, 1H). MS (ESI, m/z): 271.75$(M+H)^+$.

**Example 38**

(S)-3-(benzofuran-7-yloxy)-N-methyl-3-(oxazol-5-yl)propyl-1-amine (Compound $I_C$-29)

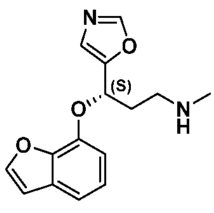

Ic-29

Except that (R)-3-chloro-1-(thiophen-2-yl)propyl-1-ol was replaced with (R)-3-chloro-1-(oxazol-5-yl)propyl-1-ol, the other required raw materials, reagents and preparation method were the same as Reference Example 8 and Example 9. 63 mg of the title compound as yellow oil was obtained. Yield: 21%.

1H NMR (500 MHz, $CDCl_3$) δ 7.83 (s, 1H), 7.62 (d, J = 2.1 Hz, 1H), 7.23 (dd, J = 7.8, 0.9 Hz, 1H), 7.08 (dd, J = 10.4, 5.3 Hz, 1H), 7.03 (s, 1H), 6.87 - 6.81 (m, 1H), 6.76 (d, J = 2.2 Hz, 1H), 5.74 (dd, J = 8.0, 5.4 Hz, 1H), 3.00 - 2.87 (m, 2H), 2.53 - 2.49 (m, 4H), 2.36 - 2.26 (m, 1H). MS (ESI, m/z): 272.76$(M+H)^+$.

**Example 39**

(S)-N-(3-(benzofuran-7-yloxy)-3-(thiophen-2-yl)propyl)acetamide (Compound $I_D$)

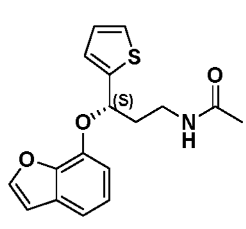

$I_D$

19 mg of acetic acid, 59 mg of 1-(3-dimethylaminopropyl)-3-ethylcarbodiimide hydrochloride, 38 mg of 4-dimethylaminopyridine and 52 mg of triethylamine were dissolved in 5 ml anhydrous dichloromethane, the mixture was stirred at room temperature for 1h, 5 ml of dichloromethane solution containing 70 mg of compound Ic-23 was added into the system, and the reaction was carried out at room temperature overnight. After the reaction was completed, water was added into the system, the mixture was extracted three times with ethyl acetate, washed with saturated brine, dried with anhydrous sodium sulfate, filtered, and concentrated. The residue was separated by column chromatography (methanol/dichloromethane=1:20) to afford 18 mg of the title compound as yellow oil. Yield: 22.29%.

$^1H$ NMR (500 MHz, $CDCl_3$) $\delta$ 7.64 (dd, $J$ = 9.8, 2.2 Hz, 1H), 7.24 (dd, $J$ = 5.0, 1.1 Hz, 1H), 7.21 (dd, $J$ = 7.8, 0.9 Hz, 1H), 7.07 - 7.02 (m, 1H), 7.01 - 6.98 (m, 1H), 6.96 - 6.91 (m, 1H), 6.78 (d, $J$ = 2.2 Hz, 1H), 6.75 (d, $J$ = 7.9 Hz, 1H), 6.31 (s, 1H), 5.78 - 5.70 (m, 1H), 3.69 - 3.59 (m, 1H), 3.55 - 3.44 (m, 1H), 2.42 - 2.24 (m, 2H), 1.99 (s, 3H). MS (ESI, m/z): 315.89$(M+H)^+$.

**Example 40**

((S)-3-(benzofuran-7-yloxy)-3-(thiophen-2-yl)propyl)-L-proline methyl ester (Compound $I_E$)

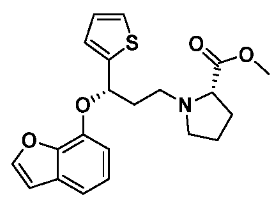

$I_E$

100 mg of Intermediate II-1, 113 mg of methyl L-proline ester hydrochloride, 283 mg of potassium carbonate and 10 mg of sodium iodide were dissolved in 5 ml of acetonitrile, and the reaction was carried out at 75°C overnight under nitrogen protection. After the reaction was completed, water was added into the system, the mixture was extracted with ethyl acetate three times, washed with water and saturated brine, dried over anhydrous sodium sulfate, filtered, and concentrated. The residue was separated by column chromatography (ethyl acetate/petroleum ether = 1: 5) to afford 16 mg of the title compound as colorless oil. Yield: 12.15%.

$^1H$ NMR (500 MHz, $CDCl_3$) δ 7.62 (d, $J$ = 2.1 Hz, 1H), 7.23 - 7.19 (m, 1H), 7.16 (dd, $J$ = 7.0, 5.7 Hz, 1H), 7.03 (ddd, $J$ = 7.8, 6.0, 1.5 Hz, 2H), 6.93 - 6.89 (m, 1H), 6.83 (t, $J$ = 8.9 Hz, 1H), 6.74 (t, $J$ = 2.1 Hz, 1H), 5.84 (dd, $J$ = 15.6, 7.6 Hz, 1H), 3.47 (s, 2H), 3.21 (s, 2H), 2.94 (d, $J$ = 53.0 Hz, 1H), 2.66 (s, 1H), 2.44 (s, 2H), 2.15 (d, $J$ = 49.5 Hz, 2H), 1.95 (s, 2H), 1.83 (s, 1H). MS (ESI, m/z): 386.13 $(M+H)^+$

**Example 41**

((S)-3-(benzofuran-7-yloxy)-3-(thiophen-2-yl)propyl)-L-proline (compound $I_F$)

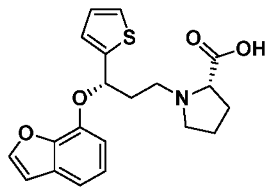

$I_F$

100 mg of compound $I_E$ and 50 mg of sodium hydroxide were dissolved in 15 ml mixed solution of water and tetrahydrofuran, and the reaction was carried out at 40°C overnight. After the reaction was completed, water was added into the system, and the reaction system was adjusted to pH=7 with diluted hydrochloric acid, extracted with ethyl acetate three times, washed with water and saturated brine, dried over anhydrous sodium sulfate, filtered, and concentrated to afford 33.7 mg of the title compound as colorless oil. Yield: 35%.

1H NMR (500 MHz, DMSO) $\delta$ 7.96 (d, J = 2.0 Hz, 1H), 7.47 (d, J = 4.9 Hz, 1H), 7.19 (t, J = 6.1 Hz, 2H), 7.06 (td, J = 7.8, 2.8 Hz, 1H), 6.94 (ddd, J = 9.9, 6.4, 2.9 Hz, 3H), 5.98 (dd, J = 12.9, 7.5 Hz, 1H), 3.45 - 3.42 (m, 1H), 3.09 (ddd, J = 20.6, 10.9, 5.1 Hz, 2H), 2.92 (s, 1H), 2.82 - 2.68 (m, 1H), 2.44 - 2.34 (m, 1H), 2.26 - 2.08 (m, 2H), 1.94 - 1.77 (m, 2H), 1.70 (dd, J = 18.3, 10.6 Hz, 1H). MS (ESI, m/z): 371.94 $(M+H)^+$.

**Comparative example 1**

**Preparation of compound of formula C1 was briefly described as follows:**

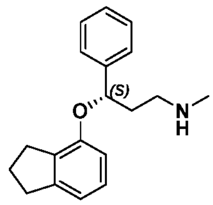

**C1**

Except that (R)-3-chloro-1-(thiophen-2-yl)propyl-1-ol was replaced with (R)-3-chloro-1-phenylpropane-1-ol, the other required raw materials, reagents and preparation method were the same as Example 18. 14 mg of compound C1 was obtained. Yield: 18%.

$^1H$ NMR (400 MHz, $CDCl_3$) $\delta$ 7.46 - 7.14 (m, 5H), 6.90 (t, J = 7.7 Hz, 1H), 6.77 (d, J = 7.4 Hz, 1H), 6.41 (d, J = 8.1 Hz, 1H), 5.33 (dd, J = 8.0, 4.4 Hz, 1H), 3.13 (dd, J = 13.5, 6.0 Hz, 2H), 2.91 (dt, J = 12.3, 7.5 Hz, 4H), 2.62 (s, 3H), 2.52 - 2.36 (m, 2H), 2.13 - 2.00 (m, 2H). MS (ESI, m/z): 282.20 $(M+H)^+$.

**Example 42**

TRPA1 inhibitory activity

The inhibitory activity of some compounds in the Examples of present invention on transient receptor potential channel protein TRPA1 was tested in this example. The compound of formula A (WO2010075353) was used as a positive control compound:

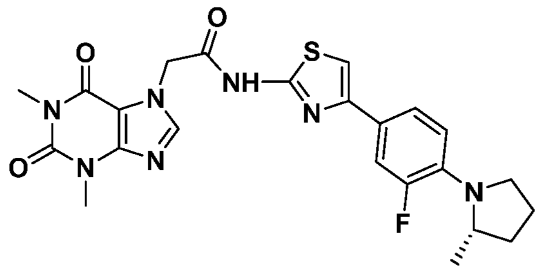

Compound of formula A

The method was as below:
IonWorks Barracuda (IWB) automated patch clamp detection was used as test method: HEK293 cells stably expressing TRPA1 were placed in DMEM medium containing 15 μg/mL Blasticidin S HCl, 200 μg/mL Hygromycin B and 10% FBS in the T175 culture flask, and cultured in 37°C, 5% $CO_2$ incubator. When the cell density reached about 80%, the culture medium was removed, rinsed with phosphate buffered saline (PBS) without calcium and magnesium. 3 mL of Trypsin was added to digest for 2 min, 7 mL of culture medium was added to terminate the digestion. The cells were collected to 15 mL centrifuge tube and centrifuged at 800 rpm for 3 min. After the supernatant was removed, the cells were added to appropriate volume of extracellular fluid for re-suspending, and the cell density was controlled at 2-3$\times 10^6$/mL for IWB experiment. Extracellular fluid formulation (in mM): 140 NaCl, 5 KCl, 1 $MgCl_2$, 10 HEPES, 0.5 EGTA, 10 Glucose (pH 7.4); intracellular fluid formulation (in mM): 140 CsCl, 10 HEPES, 5 EGTA, 0.1 $CaCl_2$, 1 $MgCl_2$ (pH 7.2). 28 mg/mL of amphotericin B was freshly prepared with DMSO on the day of experiment, and then final concentration of 0.1 mg/mL was prepared with intracellular fluid.

Population patch clamp (PPC) plate was used in IWB experiment. The entire detection process was automatically carried out by the instrument. Extracellular fluid was added into 384 wells of PPC plate, and 6 L of the intracellular fluid was added into plenum (under the PPC plate), 6 L of cell fluid was added for sealing test, and finally the intracellular fluid in plenum was replaced with amphotericin B-containing intracellular fluid to establish a whole-cell recording mode after perforating sealed cells. The sampling frequency for recording TPRA1 current was 10 kHz, the cells were clamped at 0 mV, the voltage stimulation command (channel protocol) was a ramp voltage from -100 mV to +100 mV for 300 ms. This voltage stimulation was applied every 10s, mTRPA1 current was induced by 300 M AITC.

Data recording and current amplitude measurement export were carried out by IWB software (version 2.5.3, Molecular Devices Corporation, Union City, CA). No statistics was recorded for holes with sealing impedance lower than 20 MΩ. The original current data was corrected by software for leakage reduction. TRPA1 current amplitude was measured at +100 mV. Each PPC plate in the experiment had a dose-effect data of HC030031 as a positive control. If $IC_{50}$ value of HC030031 exceeded 3 times the average $IC_{50}$ value previously obtained on each plate, it would be retested. The dose-effect curve of compounds and $IC_{50}$ were fitted and calculated by GraphPad Prism 5.02 (GraphPad Software, San Diego, CA).

**Experimental results**

Some compounds of present invention were detected by IonWorks Barracuda (IWB) automated patch clamp detection method for $IC_{50}$ inhibitory activity. The activity data was shown in Table 2, and the dose-effect relationship of some representative compounds in inhibiting TRPA1 activity was shown in Figs. 1A -1E.

Table 2. Inhibitory activity data ($IC_{50}$, μM) of some compounds of the present invention on TRPA1 in the patch clamp detection test

| NO. | $IC_{50}$(μM) | NO. | $IC_{50}$(μM) | NO. | $IC_{50}$(μM) |
|---|---|---|---|---|---|
| **Ic-1** | +++++ | **Ic-2** | ++++ | **Ic-3** | +++++ |
| **Ic-4** | +++++ | **Ic-8** | +++++ | **Ic-10** | ++++ |
| **Ic-23** | +++++ | **Ic-24** | +++++ | **Ic-25** | +++++ |
| **Ic-26** | ++++ | **Ic-29** | ++++ | Compound C1 | ++ |

(continued)

| NO. | $IC_{50}$(μM) | NO. | $IC_{50}$(μM) | NO. | $IC_{50}$(μM) |
|---|---|---|---|---|---|
| Compound of formula A | + | Duloxetine (S configuration) | +++ | Duloxetine (R configuration) | ++ |
| Activity: $IC_{50}$ (μM):<br>50-100: +<br>20-50: ++<br>10-20: +++<br>5-10: ++++<br>1-5: +++++ | | | | | |

The results showed that the compounds of present invention exhibited potent inhibitory activity on TRPA1, wherein the $IC_{50}$ values of 11 compounds on TRPA1 were 1-10 μM. It could be seen from Figs. 1A-1E that the $IC_{50}$ values of compounds of Ic-3, Ic-4, Ic-8, Ic-23, and Ic-24 on TRPA1 were <5 μM. Therefore, it could be concluded that the compounds of formula I of present invention had potent inhibitory activity on TRPA1.

In addition, the test results of S configuration of compound $I_C$-10 and the corresponding R configuration of compound $I_C$-10, i.e., (R)-3-(2,3-dihydro- 1H-inden-4-yl)oxy)-N-methyl-3-(2-thienyl)- 1-amine, and S configuration of compound Ic-23 and the corresponding R configuration of compound, i.e., (R)-3-(benzofuran-7-yloxy)-3-(thiophen-2-yl) propyl-1-amine showed that the ratio of $IC_{50}$ of R configuration of compound to that of S configuration of compound was ≥2, which suggested that compounds of the present invention in S configuration had higher inhibitory activity on TRPA1.

In addition, the activity ratio of compound Ic-10 (containing heteroaryl

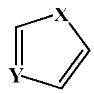

) to compound C1 (containing phenyl), i.e., the $IC_{50}$ of compound Cl/the $IC_{50}$ of compound Ic-10, was about 2.5-fold, which suggested that the compounds of present invention containing heteroaryl (such as compound Ic-10) had higher inhibitory activity on TRPA1.

In addition, compared with compounds with naphthalene ring as A group, such as duloxetine, the $IC_{50}$ values of compound Ic-10 with benzoalicyclic ring as A group, compound Ic-3, compound Ic-23, and compound Ic-1 with benzoheteroaryl as A group were significantly decreased. Specifically, the ratio of the $IC_{50}$ of S configuration of duloxetine to the $IC_{50}$ of any of compound Ic-10, compound Ic-3, compound Ic-23 or compound Ic-1 was about 2.8-6.8 (note: the $IC_{50}$ of R configuration of duloxetine was 48.5μM, and the $IC_{50}$ of S configuration of duloxetine was 24.74μM). The results suggested that the compounds of present invention with benzoalicyclyl or heteroaryl as A group had higher (about 2.8-6.8 times higher) inhibitory activity on TRPA1.

Similarly, the inventors also measured the TRPA1 inhibitory activity of S configuration of duloxetine and compound Ic-10 by manual patch clamp detection. Similar to the test results of automatic patch clamp detection method, in the manual patch clamp detection, the ratio of $IC_{50}$ of the S configuration of duloxetine to the $IC_{50}$ of the compound Ic-10 was 4.36/1.12=3.9.

Similarly, the inventors also measured the TRPA1 inhibitory activity of compound Ic-1 by manual patch clamp detection. The method was as follows:

The HEK293 cell line stably expressing human TRPA1 channel was placed in T75 culture flask with DMEM medium containing 15 μg/mL Blasticidin S HCl, 200 μg/mL Hygromycin B and 10% FBS, and cultured in incubator at 37°C and 5% $CO_2$. When the cell density reached about 80%, the culture medium was removed, the residue was rinsed with phosphate buffered saline (PBS) without calcium and magnesium. 2 mL of trypsin was added to digest for 2 minutes, and 8 mL of culture medium was added to terminate the digestion. The cells were collected to 15 mL centrifuge tube and centrifuged at 800 rpm for 3 min. After the supernatant was removed, an appropriate volume of extracellular fluid was added to resuspend the cells.

In manual patch clamp detection, HEKA system (Patch Master software) was used together with EPC-10 amplifier to record the whole cell current of TRPA1 stably transfected cell line at room temperature. intracellular fluid formulation for whole cell recording was as follows(mM): 140 CsCl, 10 HEPES, 5 EGTA, 0.1 $CaCl_2$, 1 $MgCl_2$ (pH 7.2, osmotic pressure 295-300 mOsm); $Ca^{2+}$-free extracellular fluid formulation for recording was as follows (mM): 140 NaCl, 5 KCl, 0.5 EGTA, 1 $MgCl_2$, 10 Glucose, 10 HEPES (pH 7.4, osmotic pressure 300-310 mOsm). Glass microelectrode resistance used for patch clamp recording was 2-4 MΩ, the sampling frequency was 10 kHz, the filter frequency was 2.9 kHz, the cell clamp was 0 mV, and the voltage stimulation command (channel protocol) was a linear voltage from -100 mV

to +100 mV for 300 ms, then restored to 0 mV clamping potential. The recording was performed every 2s. The hTRPA1 current was induced by 100 μM AITC. To ensure the accuracy of current recording, the series resistance was used for 60% compensation during recording.

Data recording and current amplitude measurement export were completed by Patch Master software. Cells with sealing impedance lower than 500 MΩ w were not included in the statistics. The original current data were corrected by software for leakage reduction, and the hTRPA1 current amplitude was measured at +100 mV. The compound dose-effect curve and $IC_{50}$ were fitted and calculated by GraphPad Prism 5.02 (GraphPad Software, San Diego, CA).

The results of manual patch clamp detection

Similar to the results of the automatic patch clamp detection, the ratio of $IC_{50}$ of S configuration of duloxetine to that of compound Ic-lwas 4.36 μM /0.43 μM = 10.14 in the manual patch clamp detection.

Example 43

Cytotoxicity test

In this example, the method for determining hepatotoxicity and neurotoxicity of compound Ic-10 and compound Ic-1 were as follows:

HepG-2 and SH-SY5Y cells were placed in 10cm dish and cultured at 37°C, 5% $CO_2$ in a cell incubator. Trypsin was used to digest and resuspend cells and cells were counted. The cells were transferred to 96-well plate with 8000 cells in a well (100μl/well). A serial of gradient concentration dilutions of compound with 2-fold dilution were prepared, and the system was 100 μL/well. The supernatant of cell culture system in the 96-well plate was removed on the first day, and fresh-prepared drug concentration system was add into culture plate wells (duplicate wells were set). The cells were cultured at 37°C, 5% $CO_2$ in a cell incubator for 72 h. After the cell culture was completed, the supernatant of cell culture system was removed from 96-well plate, 100μl of detection solution containing 10% CCK-8 medium was added into each well, and the cells were cultured at 37°C, 5% $CO_2$ in a cell incubator for 1 h. Microplate reader was used to measure the absorbance at 450 nm. Data was processed to calculate cytotoxicity, and $IC_{50}$ was calculated by GraphPad Prism. The cytotoxicity calculation formula was as follows: Cytotoxicity (%)=[A(0 Dosing)-A(Dosing)]/[A(0 Dosing)-A (control)) x100

A (Dosing): the absorbance of wells containing cells, CCK-8 solution and drug solution.

A (control): the absorbance of wells containing medium and CCK-8 solution and without cells.

A (0 Dosing): the absorbance of wells containing cells and CCK-8 solution but without drug solution.

Experimental result

The results of hepatotoxicity (HepG2 cell) and neurotoxicity (SH-SY5Y) of compound Ic-10 and compound Ic-1 showed: the hepatotoxicity and neurotoxicity of duloxetine were 33 μM and 28 μM ($IC_{50}$, μM), respectively, while the hepatotoxicity and neurotoxicity of compound Ic-1 and compound Ic-10 of the present invention were about 60-120 μM ($IC_{50}$, μM). It suggested that the toxicity and side effects of compounds of the present invention were significantly lower, and were about 1/2 or 1/3 of toxicity and side effects of duloxetine. The results showed the compounds of the present invention had excellent safety.

Example 44

Analgesic activity test experiment

In this example, analgesic activity of compound Ic-10 of present invention was evaluated by mice formalin pain model. The method was as follows:

30 C57BL/6 mice (male, 9-week aged) were randomly divided into 3 groups: solvent control group (vehicle, saline), duloxetine group (duloxetine, 5-HT reuptake and NE reuptake inhibitor) and Ic-10 group (compound Ic-10 of the present invention). Before the start of experiment, the mice were allowed to adapt to the experimental environment for 72 h without fasting. The test drug was administrated by intraperitoneal injection at a dose of 20 mg/kg, and then the mice were placed in a transparent, ventilated plexiglass cylinder for 1 h, and then 20 μl of 4% formalin solution was injected into the left hind plantar of mice of each group by microinjector. The claw pain response of mice was real-time recorded by miniature camera. The number of times of lifting (1 min/time), shaking (2 min/time), and licking (3 min/time) left claw and the time length of licking left claw were used as indicators of pain response, the cumulative scoring and licking time in two stages of

0-10 min (phase I, acute pain phase) and 10-60 min (phase II, inflammatory pain phase) were observed and recorded, and the statistical analysis was conducted.

Experimental results

The results of analgesic activity of compound Ic-10 of the present invention in mice formalin pain model were shown in Fig. 2. The results showed that in the statistical detection indicator of licking time, the compound Ic-10 of the present invention had showed significant analgesic activity in both phase I (0-10 min) and phase II (10-60 min) at a dose of 20 mg/kg, and almost completely inhibited licking claw behavior caused by pain as compared with saline group, and had similar analgesic activity to clinical drug duloxetine.

**Example 45**

**Mice hot plate pain test experiment**

In this example, the analgesic activity of compound Ic-23, compound Ic-10, compound Ic-1 and other compounds of the present invention were evaluated by C57 mice hot plate pain model. The method was as follows:

1 Animal screening

SPF-grade C57 male mice were placed at hot plate with constant 55±0.1 °C. Mice with painful response such as licking claw within 10-30s were selected (the mice which evade and jump were abandoned). If the pain reaction of mice was observed, the mice were taken out immediately to prevent mice from scalding.

2 Animal groups

The 40 selected animals were weighed and randomly divided into 4 groups: saline control group (blank control), duloxetine group (positive control group), gabapentin group (positive control group) and Ic-23 group (compound of the present invention).

3 Sample preparation

The test compounds were freshly prepared on the day of administration. 0.9% NaCl physiological saline solution was prepared as solvent for later use. Appropriate amount of test compounds were added into required volume of physiological saline and fully suspended, and the concentration of the drug compound in preparation was 1 mg/ml. The standard volume of administration to mice was 10 ml/kg (or 0.1 ml/10g ).

**4 Animal administration**

The administration mode was intraperitoneal administration. animals did not need to fast before administration. The administration volume was 10 ml/kg. The dosage of duloxetine and Ic-23 was 10 mg/kg, and the dosage of gabapentin was 100 mg/kg.

**5 Hot plate experimental observation**

Hot plate observation index: the reaction time of mice on the hot plate at 55±0.1°C (Time latency). Measurement and recording were conducted at 3 h before administration and 15 min, 30 min, and 60 min after administration.

**6. Data Statistics and Analysis**

Maximum possible effect (MPE) % was used to evaluate analgesic effect of each compound, i.e., MPE%=[(Post drug latency-baseline latency)/(30-baseline latency)]x100. MPE% at different time points was recorded. The higher the value of MPE% was, the stronger the analgesic effect of the compound was.

**Experimental results**

The results of analgesic activity of compound Ic-23 of the present invention in the mice hot plate pain model were shown in Fig. 3. The results showed that compared with the saline control group, the compound Ic-23 of the present

invention showed very potent analgesic effect at a dose of 10 mg/kg with a significant difference. Compared with the positive control group, the analgesic activity of the compound Ic-23 of the present invention was significantly stronger than 100 mg/kg of gabapentin, and stronger than 10 mg/kg of duloxetine within 60 minutes.

Moreover, the analgesic activity of compound Ic-10 and compound Ic-1 were stronger than those of gabapentin and duloxetine at the same dose (10 mg/kg),.

The hot plate pain model was a classic model for evaluating the efficacy of drugs on acute pain. Therefore, the compounds of the present invention had excellent therapeutic effect on acute pain.

**Example 46**

**Pharmacokinetic test of compound Ic-1**

In this example, the pharmacokinetic properties of compounds such as duloxetine and compound Ic-1 were tested in rat. The method was as follows:

Test method:

A certain amount of sample was weighed and dissolved in deionized water to prepare 1 mg/mL of solution. Male SD rats were used as test animals. The dose of single intravenous (IV) injection was 2 mg/kg, and the dose of oral (PO) administration was 10 mg/kg. Each group had three rats. The rats in oral group were fasted for 10-14 hours before administration, and were fed 4 hours after administration. Animal blood collection time points were as follows: before administration, and 5 min, 15 min, 30 min, 1 h, 2 h, 4 h, 6 h, 8 h, and 24 h after administration for intravenous administration; before administration, and 15 min, 30 min, 1 h, 2 h, 4 h, 6 h, 8 h and 24 h after administration for oral administration. About 0.25 mL of blood was collected via the jugular vein from each animal, and heparin sodium was used for anticoagulation. After collecting the blood samples, the blood samples were placed on ice and centrifuged to separate the plasma (centrifugation conditions: 8000 rpm, 6 min, 4 °C). The collected plasma was stored at -80 °C before analysis. 50 $\mu$L of plasma sample was taken into 1.5 mL of centrifuge tube, 250 $\mu$L of internal standard solution was added, and the same volume of methanol rather than internal standard solution was added in the blank group. The mixture was vortexed and mixed, centrifuged at 14,000 rpm for 5 min, 200 $\mu$L of supernatant was taken into 96-well sample plate, and the LC-MS/MS was used for sample analysis. In the linear regression analysis, the peak area was taken as the y-axis and the drug concentration was taken as the x-axis. The linear relationship between the peak area ratio and the concentration was expressed by the correlation coefficient (R) obtained from the regression equation of the compound. According to the blood concentration data of the drug, the pharmacokinetic calculation software WinNonlin7.0 non-compartmental model was used to calculate the pharmacokinetic parameters of the test drug.

Internal standard working solution: a certain amount of tolbutamide internal standard stock solution with a concentration of 490,000 ng/mL was taken into a volumetric flask with a certain volume, diluted to a desired volume with methanol and mixed well to obtain the internal standard working solution with a concentration of 200 ng/mL.

Experimental results

According to average blood concentration data of drug in each group, the pharmacokinetic calculation software WinNonlin7.0 non-compartmental model was used to calculate pharmacokinetic parameters of compound in each group. The results were shown in Table 3.

Table 3 Main pharmacokinetic parameters of compound Ic-1 and duloxetine

| Compound | Administration mode | Administration dose | Tmax (h) | Cmax (ng/ml) | Tl/2 (h) | AUC(0-∞) (h*ng/mL) | bioavailability |
|---|---|---|---|---|---|---|---|
| $I_C$-1 | IV | 2 mg/kg | 0.083 | 170 | 2.97 | 449 | 179% |
| | PO | 10 mg/kg | 3 | 266 | 5.69 | 4016 | |
| Duloxetine (S configuration) | IV | 2 mg/kg | 0.083 | 177 | 1.77 | 449 | 9.9% |
| | PO | 10 mg/kg | 0.833 | 76 | 1.81 | 222 | |

Table 3 showed that after compound Ic-1 was injected intravenously at a dose of 2 mg/kg, the peak concentration ($C_{max}$, 170 ng/mL) was reached at the first time point of sampling (0.083h), the elimination half-life (T1/2) ) was 2.97 h, AUC(0-∞) was 449 h*ng/mL; after compound Ic-1 was orally administered at a 5-fold dose (10mg/kg), the peak

concentration (Cmax, 266 ng/mL) was reached at 3 h, the elimination half-life (T1/2) was 5.69 h, and AUC(0-∞) was 4016 h*ng/mL. Based on the calculation on AUC (0-∞), the oral bioavailability was 179%.

After duloxetine was injected intravenously at a dose of 2 mg/kg, the peak concentration (Cmax, 177 ng/mL) was reached at 0.083h, the elimination half-life (T1/2) was 1.77 h, and the AUC (0-∞) was 449 h*ng/mL; after duloxetine was orally administered at a 5-fold dose (10 mg/kg), the peak concentration (Cmax, 76 ng/mL) was reached at 0.83 h, the elimination half-life (T1/2) was 1.81 h, and the AUC ( 0-∞) was 222 h*ng/mL. Based on the calculation on AUC (0-∞), the oral bioavailability was 9.9%.

The results showed that, compared with duloxetine, the compounds of formula I of the present invention (such as compound Ic-1) had more excellent pharmacokinetic properties with a longer half-life, higher exposure in plasma, and better bioavailability, so that it was suitable for development into a medicine for oral administration, and had a good prospect of medicine.

**Example 47**

Evaluation of therapeutic effect of compound Ic-1 on fibromyalgia in mice ICS model

**Experimental method**

1. Experiment group

The experimental groups were solvent control group, 10 mg/kg duloxetine group (positive control group) and 10 mg/kg compound Ic-1 group (the compound prepared in Example 9)

2. Compound preparation

10 mg/kg duloxetine: 17 mg of duloxetine was weighed, dissolved in saline and diluted to 8.5 mL. After completely dissolved, the duloxetine was administered orally. The volume of administration was 5 ml/kg.

10 mg/kg compound Ic-1: 17 mg of compound Ic-1 was weighed, dissolved in saline and diluted to 8.5 mL. After completely dissolved, the compound Ic-1 was administered orally. The volume of administration was 5 ml/kg.

3. Animals

Male C57BL/6 mice weighed 18-22 g were selected at the beginning of experiment. Each cage had 4 mice which were allowed to feed and drink water freely. Each experimental group had 12 mice, and the experimental mice were labeled by tail labeling method.

4. Experimental method

4.1 Model establishment

Day 0: At 4:30 pm, the mice were put in a plexiglass box with stainless steel mesh, and then the plexiglass box was put in cold environment (4±2°C) overnight. The mice were allowed to feed freely and drink, and agar was used to replace water.

Day 1: At 10:00 am, the mice were delivered to room temperature (24±2°C) environment for 30 min, and then the mice were delivered to cold environment for 30 min. The above steps were repeated until 4:30 pm, and the mice were put in cold environment overnight.

Day 2: the operation on day 1 was repeated.

Day 3: the mice were taken out from cold environment at 10:00 am.

4.2 Administration

The compounds were administered orally according to the schedule of experiment, and the dosage was 10 mg/kg.

4.3 Mechanical allodynia test

On the fourth day after modeling, the pre-administration mechanical allodynia test was performed on the animals. Animals with PWT value greater than 0.5 g were excluded and were not used for experiments. On the fifth day after

modeling, the animals were tested for mechanical allodynia at 0.5 h, 1 h, and 2 h after the administration of compounds.

The test method for mechanical allodynia was as follows:

The mice was individually placed in a plexiglass box with a grid on the bottom of the box to ensure that the mice claw could be tested. The mice were allowed to adapt for 15 min before the test. After the adaptation was completed, the test fiber was used to test the center of the left hind claw of mice. The test fiber comprised 8 test strengths: 2.36(0.02g), 2.44(0.04g), 2.83(0.07 g), 3.22(0.16 g), 3.61(0.4 g), 3.84(0.6 g), 4.08(1 g), and 4.17(1.4 g). During the test, the test fiber was pressed vertically against the skin and forced to bend for 6-8 s with a 5s interval of test.

During the test, rapid withdrawal of animal claw was recorded as pain response. When the test fiber was removed from animal skin, the withdrawal of animal claw was also recorded as pain response. If animal moved, the pain response was not recorded, and the test was repeated. In the test, 3.22(0.16 g) was used firstly. If animal responded to pain, the test fiber with lower strength was used in next test; if the animal did not respond to pain, test fiber with higher strength was used in next test (Chaplan et al. 1994). The maximum strength of tested fiber was 4.17(1.4 g).

The test results were recorded in Table 4 below, wherein pain response was recorded as X
and no pain response was recorded as O.

Table 4

| | | | |
|---|---|---|---|
| 2.36 | | | |
| 2.44 | | | |
| 2.83 | | | |
| 3.22 | O | | |
| 3.61 | O | O | O |
| 3.84 | X | X | X |
| 4.08 | | | |
| 4.17 | | | |

Mechanical allodynia was calculated by using the following formula:

$$50\% \text{ response threshold (g)} = (10^{(Xf+k\ \delta\ )})/10{,}000;$$

Xf = the final test fiber value used in the test;
k = table value (Chaplan *et al.,* 1994, page 62);
δ = mean difference

5. Data collection and analysis

Excel software was used to collect data, and prism software was used to analyze data. The larger the withdrawal threshold (PWT) was, the stronger the analgesic effect of the compound was.

Experimental result

The results of analgesic activity of compound Ic-1 in the mice ICS model were shown in Table 5 and Fig. 4.

Table 5 Statistical data of mechanical allodynia test results (PWT) of compound Ic-1 and duloxetine at different times after administration in mice ICS model

| Compound | Dosage | PWT(g) Mean ± SD | | |
|---|---|---|---|---|
| | | 0.5 h | 1 h | 2h |
| Duloxetine | 10 mg/kg | 0.416 ± 0.140 | 0.648 ± 0.155 | 0.530 ± 0.235 |
| **Ic-1** | 10 mg/kg | 0.482 ± 0.199 | 0.773 ± 0.261 | 0.607 ± 0.160 |
| Solvent control group | | 0.369 ± 0.149 | 0.366 ± 0.159 | 0.367 ± 0.095 |

The Table 5 and Fig. 4 showed that compound Ic-1 of the present invention had very potent analgesic effect at a dose of 10 mg/kg, and could inhibit mechanical allodynia 1 hour and 2 hours after oral administration in ICS model. Compared with the positive control group, compound Ic-1 had stronger analgesic effect than that of duloxetine at 0.5 h, 1 h and 2 h. The mice ICS model was classic model for evaluating the efficacy of drug in the treatment of fibromyalgia. Therefore, the compound Ic-1 of the present invention had excellent therapeutic effect on fibromyalgia.

**Example 48**

Evaluation of therapeutic effect of compound Ic-1 on visceral pain and inflammatory pain in mice acetic acid writhing pain model

**Experimental method**

Male ICR mice, weighed 22-25g, were fasted but allowed to drink water freely for 2 h before administration. All ICR mice were weighed and grouped randomly, and the number of animals in each group was >10. The negative control group was saline group (vehicle, blank control), and the positive control group was administrated with 10 mg/kg indomethacin (a non-steroidal anti-inflammatory drug), 10 mg/kg anisodamine (an antispasmodic drug with clinically analgesic activity), 10 mg/kg duloxetine and 20 mg/kg duloxetine. The test compound was Ic-1 (the compound prepared in Example 9), and the administration dosages were 5 mg/kg and 10 mg/kg. The drug was administrated by gavage based on the weight of mice. 1.5% acetic acid solution (0.1ml/10g) was injected intraperitoneally 1 hour after administration, and the number of times of visceral pain in each group was observed within 30 min. When concave abdomen, stretched trunk and hind claw, and high buttocks appeared, one number point was recorded. Finally, the number of appearance of the above phenomenon was counted within 30 minutes. After administration, the fewer visceral pains in the mice were, the stronger the analgesic effect of the compound was.

Experimental result

The mice acetic acid writhing pain model test results were shown in Fig 5. Fig 5 showed that the compound Ic-1 (5 mg/kg and 10 mg/kg) of the present invention could significantly reduce the number of appearance of writhing reaction in mice caused by acetic acid with a significant difference as compared with the number of appearance of writhing reaction in mice in the saline group (vehicle, blank control) (49 times). At a dosage of 5 mg/kg of compound Ic-1, the number of appearance of writhing reaction in mice was 20 times, which was 50% lower than that (49 times) in the saline control group, suggesting that the half effective dose ($ED_{50}$) of compound Ic-1 was less than 5 mg/kg in the model. The analgesic effect of compound Ic-1 at a dosage of 10 mg/kg (17 times) was stronger than that of positive drug indomethacin (28 times), anisodamine (27 times) and duloxetine (27 times) at the same dosage. The analgesic effect of compound Ic-1 at a dosage of 5 mg/kg (20 times) was equivalent to that of duloxetine at a dosage of 20 mg/kg (21 times).

This experiment showed that the analgesic activity of compound Ic-1 of the present invention was significantly stronger than positive control drug in the mice acetic acid writhing pain model. The mice acetic acid writhing pain model was a classical model for evaluating the efficacy of drug in treating visceral pain and inflammatory pain. Therefore, the compound Ic-1 of the present invention had excellent therapeutic effect on visceral pain and inflammatory pain.

**Example 49**

Evaluation of therapeutic effect of compound Ic-1 on nerve pain in rat SNL model

**Experimental method**

Male SPF grade SD rats, weighed 150-180g, were selected. Aseptic operation during surgery was performed. The animals were anesthetized with sodium pentobarbital (50 mg/kg, intraperitoneal injection). The surgical area of animal waist was shaved and the skin was disinfected three times with iodophor and 70% ethanol. After the skin was dried, the operation was started. Surgical knife was used to make a longitudinal incision at the back of the sacrum of the animal waist to expose the left paraspinal muscles, and stretcher was used to separate muscle tissue to expose the spine. The left spinal nerves L5 and L6 were separated and ligated with a 6-0 silk thread, and the wound was sutured. After the operation, the animals were placed on electrothermal pad, and 5 mL of saline was injected subcutaneously to prevent dehydration. After the animals were fully awakened and could move around freely, the animals were put back in the cage.

After the operation, the animals were adapted in the experimental environment for 15 min/day for 3 days. One day before the administration, the rats were subjected to mechanical allodynia baseline test, and the animals that did not exhibit mechanical allodynia (the withdrawal threshold was greater than 5g) were eliminated and the remaining rats were

randomly divided into one control group and two experimental groups.

The animals were weighed to calculate the dosage. The rats in two experimental groups were administrated with 100 mg/kg gabapentin (gabapentin was currently the first-line drug for the treatment of neuralgia) and 10 mg/kg compound Ic-1 (the compound prepared in Example 9), and the rats in control group were administrated with equal volume of saline orally. 1 h after administration, mechanical allodynia test was performed. The rat was individually placed in a plexiglass box with a grid on the bottom of the box to ensure that the rat claw could be tested. The rats were allowed to adapt the environment for 15 min before test. After the adaptation was completed, the test fiber was used to test the center of the left hind claw of the rat. The test fiber comprises 8 test strengths: 3.61 (0.4g), 3.84 (0.6g), 4.08 (1g), 4.31 (2g), 4.56 (4g), 4.74 (6g), 4.93 (8g), 5.18 (15g). During the test, the test fiber was pressed vertically against the skin and forced to bend the fiber for 6-8 s with a 5s interval of test. During the test, rapid withdrawal of animal claw was recorded as pain response. When the test fiber was removed from animal skin, the withdrawal of animal claw was also recorded as pain response. If animal moved, the pain response was not recorded and the test was repeated. In the test, 4.31 (2 g) was used firstly. If animal responded to pain, the test fiber with lower strength was used in next test; if the animal did not respond to pain, test fiber with higher strength was used in next test. The maximum strength of tested fiber was 5.18 (15 g).

Mechanical allodynia was expressed as withdrawal threshold (PWT) in rat behavioral test, which was calculated with the following formula:

$$50\%\ \text{response threshold (g)} = (10^{(Xf+k\ \delta\ )})/10{,}000;$$

Xf = the final test fiber value used in the test;
k = table value;
δ = mean difference

Excel software was used to collect data, and Prism 6.01(Graph pad software, Inc.) software was used to analyze data. The larger the withdrawal threshold (PWT) was, the stronger the analgesic effect of the compound was.

**Experimental result**

The results of analgesic activity in the rat SNL model were shown in Table 6 and Fig. 6.

Table 6 The statistical data of claw withdrawal threshold (PWT) 1 hour after administration of the compound Ic-1 and gabapentin in the rat SNL model

| Compound | Dose | PWT(g) |
|---|---|---|
| Control group | | 3.967 ± 0.775 |
| Compound Ic-1 | 10 mg/kg | 7.869 ± 2.846 |
| Gabapentin | 100 mg/kg | 6.352 ± 1.897 |

Table 6 and Fig. 6 showed that, compared with the saline control group, the compound IC-1 of the present invention had very potent analgesic effect at a dosage of 10 mg/kg with a significant difference. Compared with the positive control group, the analgesic activity of compound Ic-1 of the present invention was equivalent to the analgesic effect of 100 mg/kg gabapentin within 1h after administration. The rat SNL model was a classical model for evaluating the efficacy of drug in the treatment of nerve pain. Therefore, the compound Ic-1 of the present invention had excellent therapeutic effect on nerve pain.

**Example 50**

Evaluation of therapeutic effect of compound IC-1 on acute pain and inflammatory pain in mice formalin pain model

**Experimental method**

100 male C57BL/6 mice (9-week aged), were randomly divided into 10 groups to evaluate analgesic activity of two compounds in the mice formalin pain model, and each group had 10 mice. The experimental groups were duloxetine group and the compound Ic-1 group (the compound prepared in Example 9), respectively. Before the start of experiment, the mice were allowed to adapt experimental environment for 72 h with free feeding and drinking water. The test drug was

administrated intraperitoneally, and the dosage was as follows:

Duloxetine group: blank vehicle (blank saline control), 1 mg/kg, 5 mg/kg, 10 mg/kg and 20 mg/kg;
Compound Ic-1 group: blank vehicle (blank saline control, similar to that in Duloxetine group), 0.1 mg/kg, 0.5 mg/kg, 1 mg/kg, 5 mg/kg and 10 mg/kg.

After administration, the mice were placed in a transparent, ventilated plexiglass cylinder, and 1 h later, 20 μl of 4% formalin solution was injected into the left hind plantar of mice in each group by micro-injector, and claw pain response in mice was recorded in real time by a mini-camera. The time length of licking left claw was used as an indicator of pain response, licking time in 0-10 min (phase I) and 10-60 min (phase II) was observed and recorded, and the statistical analysis was conducted. The half effective dose ($ED_{50}$) of three compounds was calculated: $ED_{50}$ referred to the dose of the drug that decreased licking time by half as compared with the blank control group. The smaller the $ED_{50}$ value was, the lower the effective analgesic dose of the compound was and the stronger the analgesic effect was.

**Experimental results**

The claw licking time statistical results of compound Ic-1 and duloxetine in the mice formalin model at different dosages (10-60min) were shown in Table 7 and Fig. 7.

Table 7 The licking time statistical results of compound Ic-1 and duloxetine in the mice formalin model at different dosages (Phase II, 10-60min)

| Dose | Licking time(s) | |
|---|---|---|
| | Duloxetine | Compound Ic-1 |
| Blank Vehicle | 349.55±43.09 | 349.55±43.09 |
| 0.1 mg/kg | - | 339.35±27.90 |
| 0.5 mg/kg | - | 368.16±44.46 |
| 1 mg/kg | 259.97±64.44 | 171.57±39.69 |
| 5 mg/kg | 230.49±41.31 | 158.02±25.18 |
| 10 mg/kg | 162.83±36.27 | 35.68±11.77 |
| 20 mg/kg | 72.27±17.29 | -- |

Table 7 and Fig. 7 showed that the licking time of compound Ic-1 of the present invention in phase II (10-60min) at a dosage of 1 mg/kg had decreased by more than 50% as compared with that of blank Vehicle. The analgesic effect ($ED_{50}$) in phase II pain was 2.22 mg/kg, while the $ED_{50}$ of duloxetine in phase II pain was 8.00 mg/kg. The analgesic activity of compound Ic-1 of the present invention was significantly stronger than that of duloxetine at the same dosage. From the above data, it could be seen that the compound Ic-1 of the present invention showed very strong analgesic activity in the mice formalin pain model. The mice formalin model was a classical model for evaluating drug effect on acute pain and inflammatory pain. Therefore, the compound Ic-1 of the present invention had excellent therapeutic effect on acute pain and inflammatory pain.

It should be understood that those skilled in the art will be able to make various changes or modifications to the present invention after reading the teachings of the present invention, as long as they fall within the scope of the claims .

## Claims

1. A compound of formula I, or a pharmaceutically acceptable salt thereof for use in preventing and/or treating a disease caused by transient receptor potential channel protein (TRP), wherein the disease is selected from the group consisting of pain, epilepsy, inflammation, respiratory disorder, pruritus, urinary tract disorder and inflammatory bowel disease;

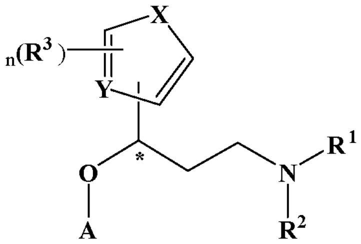

I

wherein,

A is benzofuranyl or indanyl;
$R^1$ and $R^2$ are each independently hydrogen, substituted or unsubstituted $C_1$-$C_6$ alkyl; X is oxygen atom, or sulfur atom;
Y is CH or nitrogen atom;
$R^3$ is hydrogen, halogen, substituted or unsubstituted $C_1$-$C_6$ alkyl;
n is 1;
"*" represents a chiral carbon atom, and the absolute configuration of the chiral carbon atom is S type;
wherein, the "substituted" means that 1-4 hydrogen atoms on the group are substituted by a substituent selected from the group consisting of $C_1$-$C_6$ alkyl, $C_3$-$C_7$ cycloalkyl, $C_1$-$C_3$ haloalkyl, halogen, nitro, cyano, hydroxyl, $C_1$-$C_4$ carboxyl, $C_2$-$C_4$ ester group, $C_2$-$C_4$ amide group, $C_1$-$C_6$ alkoxyl, $C_1$-$C_6$ haloalkoxy, benzyl, 5- or 6- membered aryl or heteroaryl.

**2.** The compound or salt for use according to claim 1, wherein the compound comprises one or more features selected from the following group:

$R^1$ and $R^2$ are each independently hydrogen or $C_1$-$C_3$ alkyl; and/or
$R^3$ is hydrogen, halogen, substituted or unsubstituted $C_1$-$C_3$ alkyl.

**3.** The compound or salt for use according to claim 1, wherein the compound comprises one or more features selected from the following group:

$R^1$ and $R^2$ are each independently hydrogen, methyl; and/or
$R^3$ is hydrogen, chlorine atom or methyl.

**4.** A compound or a pharmaceutically acceptable salt thereof for use in preventing and/or treating a disease caused by transient receptor potential channel protein (TRP), wherein the disease is selected from the group consisting of pain, epilepsy, inflammation, respiratory disorder, pruritus, urinary tract disorder and inflammatory bowel disease, and the compound is selected from the following group:

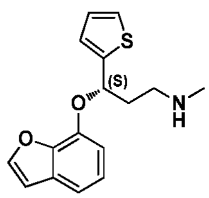

Ic-1

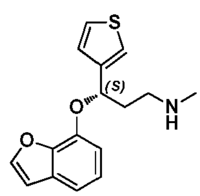

Ic-2

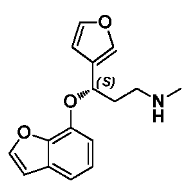

Ic-3

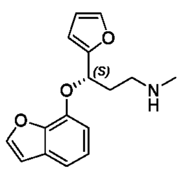

Ic-4

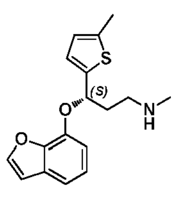

Ic-5

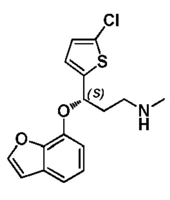

Ic-6

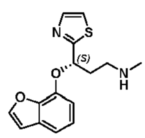

Ic-7

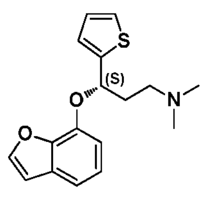

Ic-8

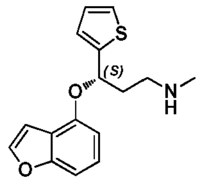

$I_C$-9

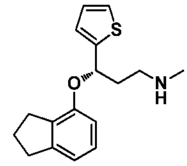

$I_C$-10

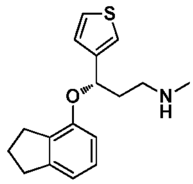

$I_C$-11

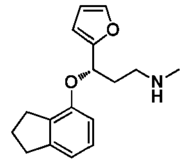

$I_C$-12

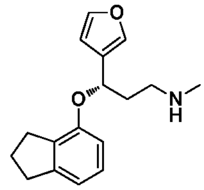

$I_C$-13

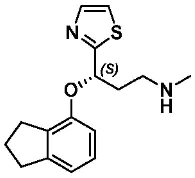

$I_C$-14

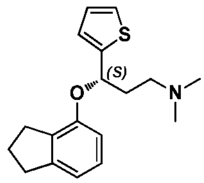

$I_C$-15

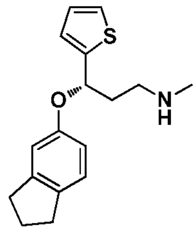

$I_C$-16

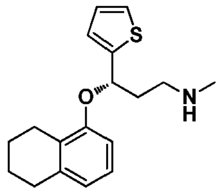

$I_C$-17

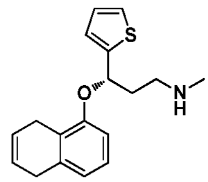

$I_C$-18

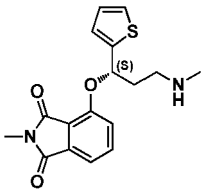

$I_C$-19

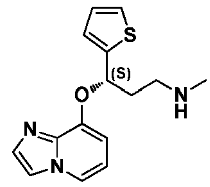

$I_C$-20

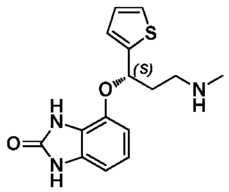

$I_C$-21

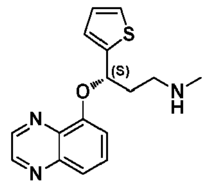

$I_C$-22

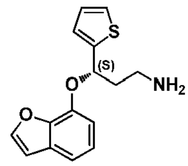

$I_C$-23

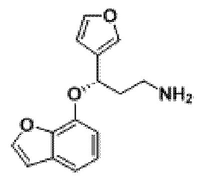

$I_C$-24

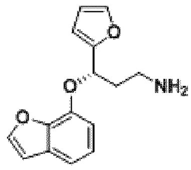

$I_C$-25

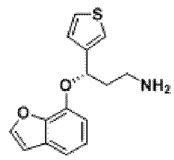

$I_C$-26

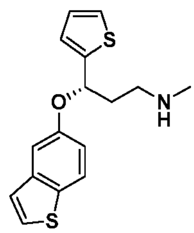

$I_C$-27

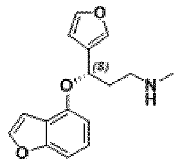

$I_C$-28

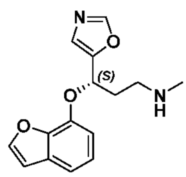

$I_C$-29

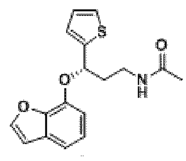

$I_D$

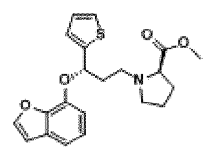

$I_E$

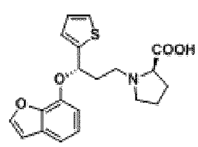

$I_F$

**5.** The compound or salt for use according to claim 1 or 4, wherein the transient receptor potential channel protein is TRPA1.

**6.** The compound or salt for use according to claim 1 or 4, wherein the disease related to transient receptor potential channel protein is pain.

**7.** The compound or salt for use according to claim 1 or 4, wherein the pain comprises acute inflammatory pain, chronic inflammatory pain, visceral pain, neurogenic pain, fibromyalgia, headache, neuralgia or pain caused by cancer.

**8.** A compound of formula I, or a pharmaceutically acceptable salt thereof

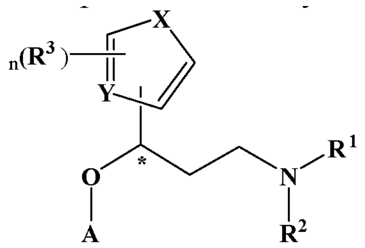

I

wherein,

A is benzofuranyl or indanyl;
$R^1$ and $R^2$ are each independently hydrogen, substituted or unsubstituted $C_1$-$C_6$ alkyl;
X is oxygen atom, or sulfur atom;
Y is CH or nitrogen atom;
$R^3$ is hydrogen, halogen, substituted or unsubstituted $C_1$-$C_6$ alkyl;
n is 1;
"*" represents a chiral carbon atom, and the absolute configuration of the chiral carbon atom is S type;
wherein, the "substituted" means that 1-4 hydrogen atoms on the group are substituted by a substituent selected from the group consisting of $C_1$-$C_6$ alkyl, $C_3$-$C_7$ cycloalkyl, $C_1$-$C_3$ haloalkyl, halogen, nitro, cyano, hydroxyl, $C_1$-$C_4$ carboxyl, $C_2$-$C_4$ ester group, $C_2$-$C_4$ amide group, $C_1$-$C_6$ alkoxyl, $C_1$-$C_6$ haloalkoxy, benzyl, 5- or 6- membered aryl or heteroaryl.

**9.** The compound of claim 8, wherein the compound comprises one or more features selected from the following group:

$R^1$ and $R^2$ are each independently hydrogen or $C_1$-$C_3$ alkyl; and/or
$R^3$ is hydrogen, halogen, substituted or unsubstituted $C_1$-$C_3$ alkyl.

**10.** The compound of claim 8, wherein the compound comprises one or more features selected from the following group:

$R^1$ and $R^2$ are each independently hydrogen or methyl; and/or
$R^3$ is hydrogen, chlorine atom or methyl.

**11.** A compound selected from the following group, or a pharmaceutically acceptable salt thereof:

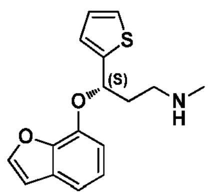

Ic-1

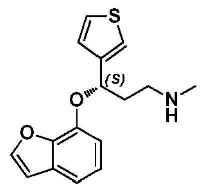

Ic-2

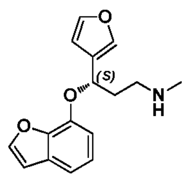

Ic-3

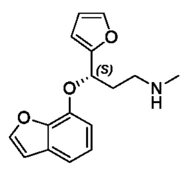

Ic-4

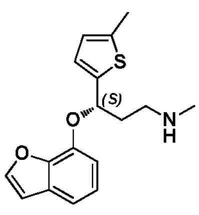

Ic-5

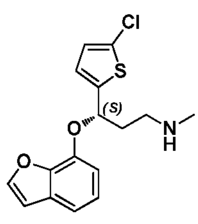

Ic-6

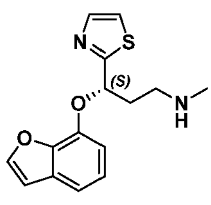

Ic-7

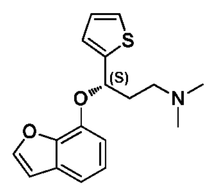

Ic-8

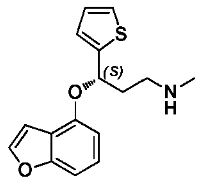
Ic-9

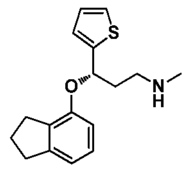
Ic-10

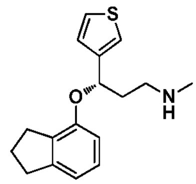
Ic-11

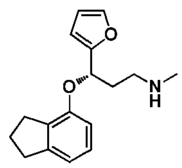
Ic-12

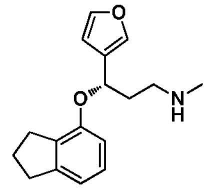
Ic-13

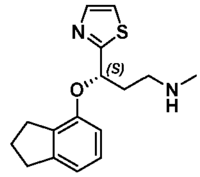
Ic-14

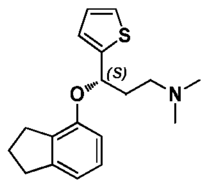
Ic-15

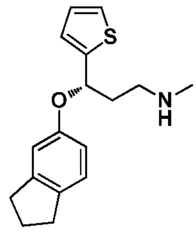
Ic-16

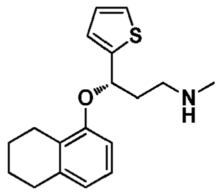
Ic-17

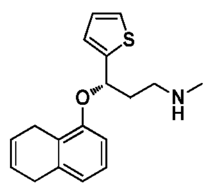
Ic-18

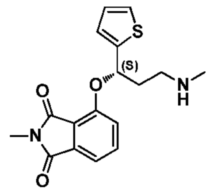
Ic-19

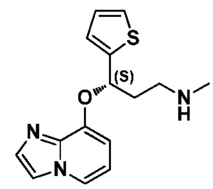
Ic-20

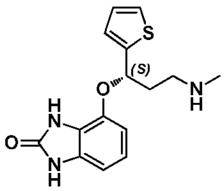
Ic-21

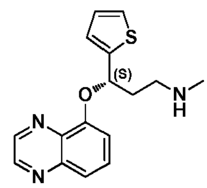
Ic-22

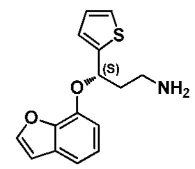
Ic-23

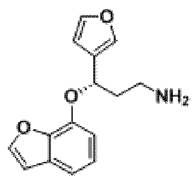
Ic-24

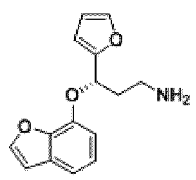
Ic-25

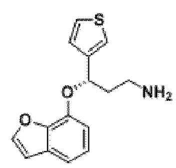
Ic-26

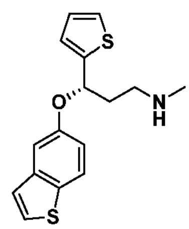
Ic-27

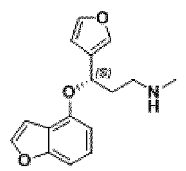
Ic-28

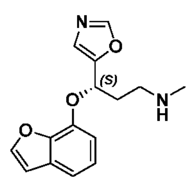
Ic-29

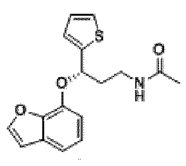
ID

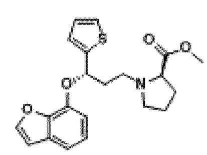
IE

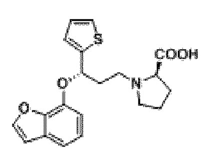
IF

**12.** A pharmaceutical composition which comprises a compound, or a pharmaceutically acceptable salt of claim 8 or 11; and a pharmaceutically acceptable carrier.

**13.** A method for preparing a compound, or a pharmaceutically acceptable salt of claim 8 or 11, wherein the method comprises: in an inert solvent, reacting an intermediate of formula II with $R^1$-NH-$R^2$, thereby forming a compound of formula I:

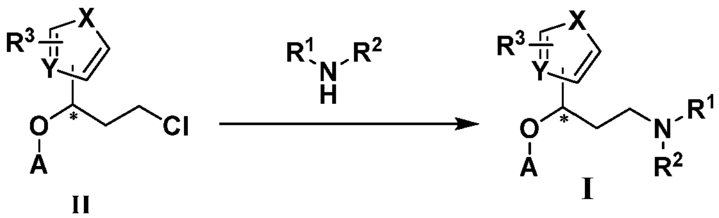

II

I

wherein X, Y, A, $R^1$, $R^2$, $R^3$ and "*" are as defined in claim 8.

**14.** A method for non-therapeutically and non-diagnostically inhibiting activity of a transient receptor potential channel protein (TRP) *in vitro,* which comprises contacting the transient receptor potential channel protein or cells expressing the TRP protein with a compound, or a pharmaceutically acceptable salt of claim 8 or 11 to inhibit the activity of transient receptor potential channel protein.

**Patentansprüche**

**1.** Verbindung der Formel I oder ein pharmazeutisch annehmbares Salz davon zur Verwendung bei der Vorbeugung gegen eine und/oder Behandlung einer Erkrankung, die durch ein transientes Rezeptorpotential-Kanalprotein (TRP) verursacht wird, wobei die Erkrankung aus der aus Schmerzen, Epilepsie, einer Entzündung, Atemstörungen, Pruritus, einer Harnwegsstörung und einer chronisch-entzündlichen Darmerkrankung bestehenden Gruppe ausgewählt ist;

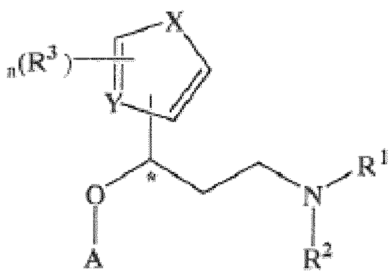

I

worin

A Benzofuranyl oder Indanyl ist;
$R^1$ und $R^2$ jeweils unabhängig voneinander Wasserstoff, substituiertes oder unsubstituiertes $C_1$-$C_6$-Alkyl sind;
X ein Sauerstoffatom oder ein Schwefelatom ist;
Y CH oder ein Stickstoffatom ist;
$R^3$ Wasserstoff, Halogen, substituiertes oder unsubstituiertes $C_1$-$C_6$-Alkyl ist;
n = 1 ist;
"*" für ein chirales Kohlenstoffatom steht und die absolute Konfiguration des chiralen Kohlenstoffatoms der S-Typ ist;
wobei "substituiert" bedeutet, dass 1 bis 4 Wasserstoffatome der Gruppe durch einen Substituenten ersetzt sind, der aus der aus $C_1$-$C_6$-Alkyl, $C_3$-$C_7$-Cycloalkyl, $C_1$-$C_3$-Halogenalkyl, Halogen, Nitro, Cyano, Hydroxyl, $C_1$-$C_4$-Carboxyl, einer $C_2$-$C_4$-Estergruppe, einer $C_2$-$C_4$-Amidgruppe, $C_1$-$C_6$-Alkoxyl, $C_1$-$C_6$-Halogenalkoxy, Benzyl, 5- oder 6-gliedrigem Aryl und Heteroaryl bestehenden Gruppe ausgewählt ist.

**2.** Verbindung oder Salz zur Verwendung nach Anspruch 1, wobei die Verbindung ein oder mehrere Merkmale aufweist, die aus der folgenden Gruppe ausgewählt sind:

$R^1$ und $R^2$ sind jeweils unabhängig voneinander Wasserstoff oder $C_1$-$C_3$-Alkyl; und/oder
$R^3$ ist Wasserstoff, Halogen, substituiertes oder unsubstituiertes $C_1$-$C_3$-Alkyl.

**3.** Verfahren oder Salz zur Verwendung nach Anspruch 1, wobei die Verbindung ein oder mehrere Merkmale aufweist,

die aus der folgenden Gruppe ausgewählt sind:

$R^1$ und $R^2$ sind jeweils unabhängig voneinander Wasserstoff, Methyl; und/oder
$R^3$ ist Wasserstoff, ein Chloratom oder Methyl.

**4.** Verbindung oder ein pharmazeutisch annehmbares Salz davon zur Verwendung bei der Vorbeugung und/oder Behandlung einer Erkrankung, die durch ein transientes Rezeptorpotential-Kanalprotein (TRP) verursacht wird, wobei die Erkrankung aus der aus Schmerzen, Epilepsie, einer Entzündung, Atemstörungen, Pruritus, einer Harnwegsstörung und chronisch-entzündlicher Darmerkrankung bestehenden Gruppe ausgewählt ist und die Verbindung aus der folgenden Gruppe ausgewählt ist:

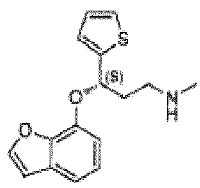
$I_C$-1

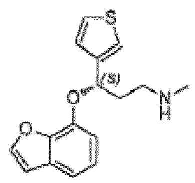
$I_C$-2

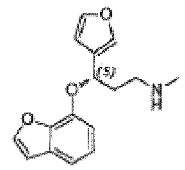
$I_C$-3

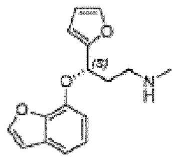
$I_C$-4

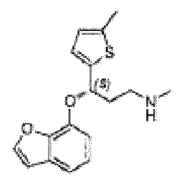
$I_C$-5

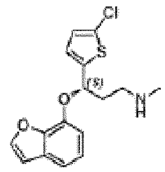
$I_C$-6

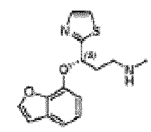
$I_C$-7

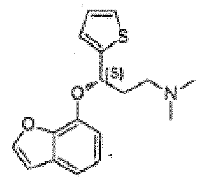
$I_C$-8

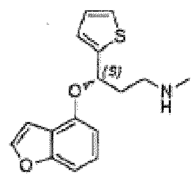
$I_C$-9

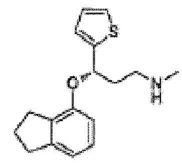
$I_C$-10

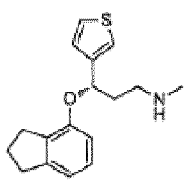
$I_C$-11

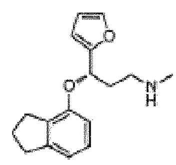
$I_C$-12

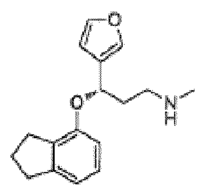
$I_C$-13

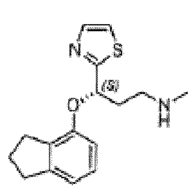
$I_C$-14

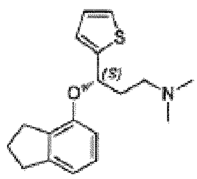
$I_C$-15

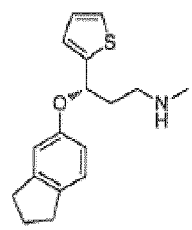
$I_C$-16

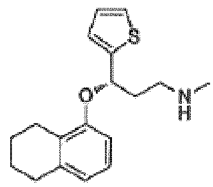
$I_C$-17

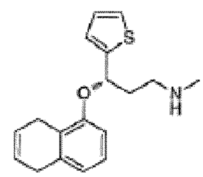
$I_C$-18

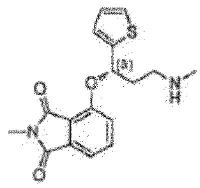
$I_C$-19

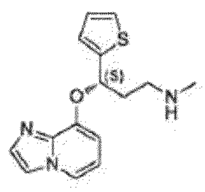
$I_C$-20

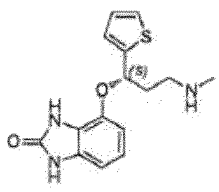
$I_C$-21

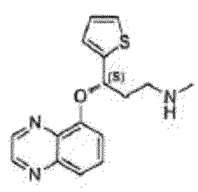
$I_C$-22

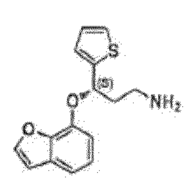
$I_C$-23

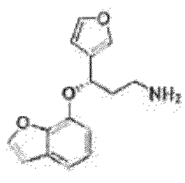
$I_C$-24

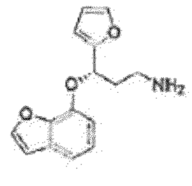
$I_C$-25

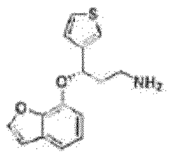
$I_C$-26

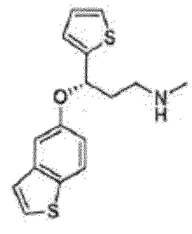
$I_C$-27

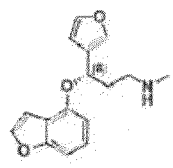
$I_C$-28

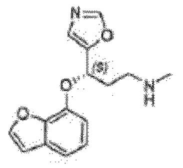
$I_C$-29

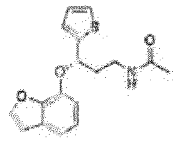
$I_D$

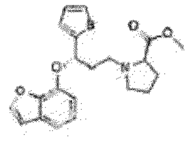
$I_E$

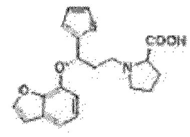
$I_F$

**5.** Verbindung oder Salz zur Verwendung nach einem der Ansprüche 1 oder 4, wobei das transiente Rezeptorpotential-Kanalprotein (TRP) TRPA1 ist.

**6.** Verbindung oder Salz zur Verwendung nach einem der Ansprüche 1 oder 4, wobei die mit dem transienten Rezeptorpotential-Kanalprotein zusammenhängende Erkrankung Schmerzen sind.

**7.** Verbindung oder Salz zur Verwendung nach einem der Ansprüche 1 oder 4, wobei die Schmerzen akute Entzündungsschmerzen, chronische Entzündungsschmerzen, viszerale Schmerzen, neurogene Schmerzen, Fibromyalgie, Kopfschmerzen, Neuralgie oder durch Krebs verursachte Schmerzen sind.

**8.** Verbindung der Formel I oder ein pharmazeutisch annehmbares Salz davon

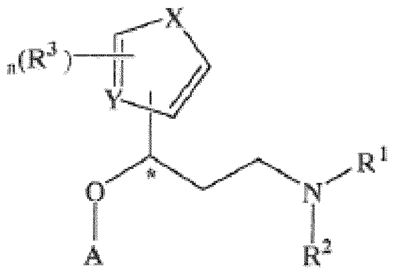
I

worin

A Benzofuranyl oder Indanyl ist;
$R^1$ und $R^2$ jeweils unabhängig voneinander Wasserstoff, substituiertes oder unsubstituiertes $C_1$-$C_6$-Alkyl sind;
X ein Sauerstoffatom oder ein Schwefelatom ist;
Y CH oder ein Stickstoffatom ist;
$R^3$ Wasserstoff, Halogen, substituiertes oder unsubstituiertes $C_1$-$C_6$-Alkyl ist;
n = 1 ist;
"*" für ein chirales Kohlenstoffatom steht und die absolute Konfiguration des chiralen Kohlenstoffatoms der S-Typ ist;
wobei "substituiert" bedeutet, dass 1 bis 4 Wasserstoffatome der Gruppe durch einen Substituenten ersetzt sind, der aus der aus $C_1$-$C_6$-Alkyl, $C_3$-$C_7$-Cycloalkyl, $C_1$-$C_3$-Halogenalkyl, Halogen, Nitro, Cyano, Hydroxyl, $C_1$-$C_4$-Carboxyl, einer $C_2$-$C_4$-Estergruppe, einer $C_2$-$C_4$-Amidgruppe, $C_1$-$C_6$-Alkoxyl, $C_1$-$C_6$-Halogenalkoxy, Benzyl, 5- oder 6-gliedrigem Aryl und Heteroaryl bestehenden Gruppe ausgewählt ist.

**9.** Verbindung nach Anspruch 8, wobei die Verbindung ein oder mehrere Merkmale aufweist, die aus der folgenden

Gruppe ausgewählt sind:

$R^1$ und $R^2$ sind jeweils unabhängig voneinander Wasserstoff oder $C_1$-$C_3$-Alkyl; und/oder
$R^3$ ist Wasserstoff, Halogen, substituiertes oder unsubstituiertes $C_1$-$C_3$-Alkyl.

**10.** Verbindung nach Anspruch 8, wobei die Verbindung ein oder mehrere Merkmale aufweist, die aus der folgenden Gruppe ausgewählt sind:

$R^1$ und $R^2$ sind jeweils unabhängig voneinander Wasserstoff, Methyl; und/oder
$R^3$ ist Wasserstoff, ein Chloratom oder Methyl.

**11.** Verbindung, die aus der folgenden Gruppe ausgewählt ist, oder ein pharmazeutisch annehmbares Salz davon:

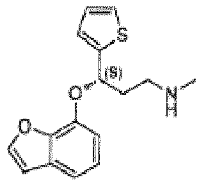
$I_C$-1

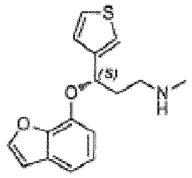
$I_C$-2

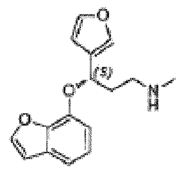
$I_C$-3

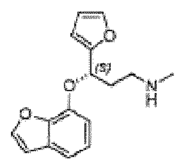
$I_C$-4

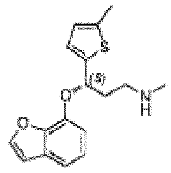
$I_C$-5

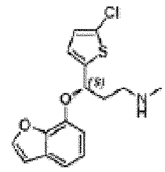
$I_C$-6

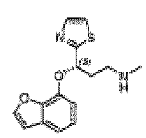
$I_C$-7

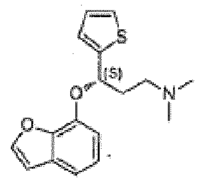
$I_C$-8

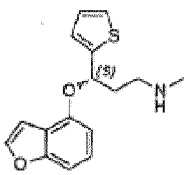
$I_C$-9

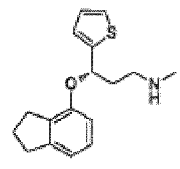
$I_C$-10

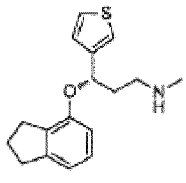
$I_C$-11

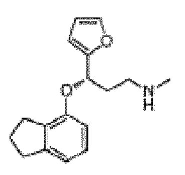
$I_C$-12

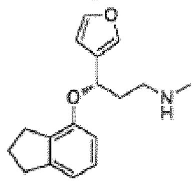
$I_C$-13

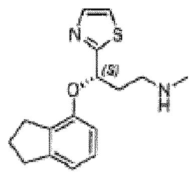
$I_C$-14

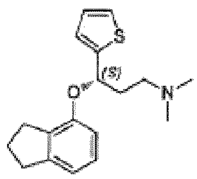
$I_C$-15

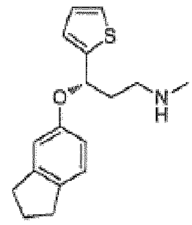
$I_C$-16

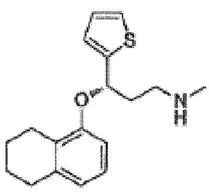
$I_C$-17

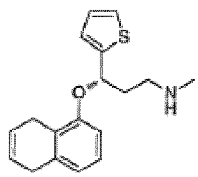
$I_C$-18

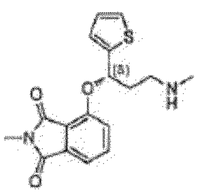
$I_C$-19

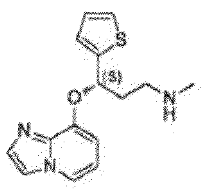
$I_C$-20

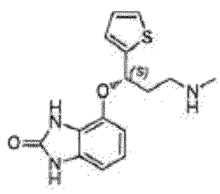
$I_C$-21

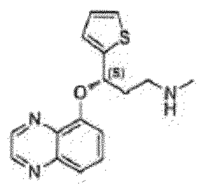
$I_C$-22

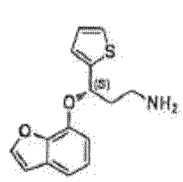
$I_C$-23

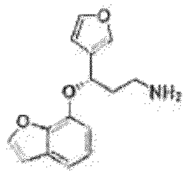
Ic-24

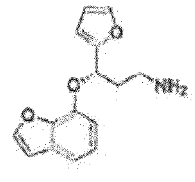
Ic-25

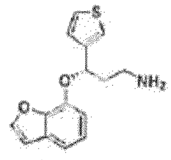
Ic-26

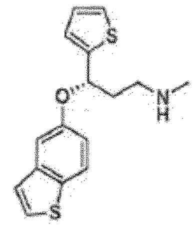
Ic-27

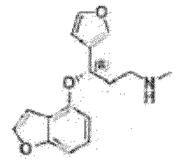
Ic-28

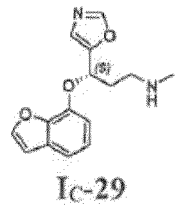
Ic-29

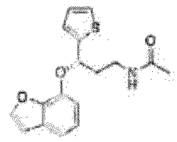
ID

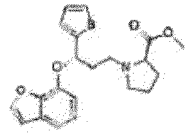
IE

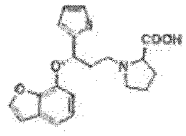
IF

**12.** Pharmazeutische Zusammensetzung, die eine Verbindung oder ein pharmazeutisch annehmbares Salz nach Anspruch 8 oder 11 und einen pharmazeutisch annehmbaren Träger umfasst.

**13.** Verfahren zur Herstellung einer Verbindung oder eines pharmazeutisch annehmbaren Salzes nach Anspruch 8 oder 11, wobei das Verfahren Folgendes umfasst: das Umsetzen eines Zwischenprodukts der Formel II mit $R^1$-NH-$R^2$ in einem inerten Lösungsmittel, um eine Verbindung der Formel I zu bilden:

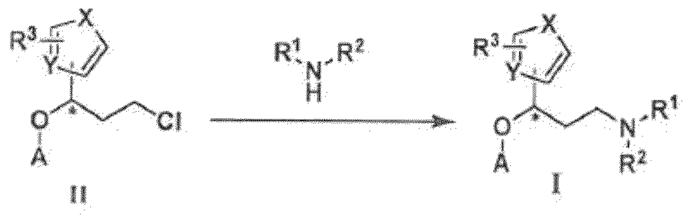
II I

worin X, Y, A, $R^1$, $R^2$, $R^3$ und "*" wie in Anspruch 8 definiert sind.

**14.** Verfahren zur nicht-therapeutischen und nicht-diagnostischen Hemmung der Aktivität eines transienten Rezeptorpotential-Kanalproteins (TRP) in vitro, welches das In-Kontakt-Bringen des transienten Rezeptorpotential-Kanalproteins oder von Zellen, die das TRP-Protein exprimieren mit einer Verbindung oder einem pharmazeutisch annehmbaren Salz nach Anspruch 8 oder 11 umfasst, um die Aktivität eines transienten Rezeptorpotential-Kanalproteins zu hemmen.

## Revendications

**1.** Composé de formule I, ou un sel pharmaceutiquement acceptable de celui-ci, à utiliser dans la prévention et/ou le traitement d'une maladie causée par une protéine du canal à potentiel de récepteur transitoire (TRP), dans lequel la maladie est choisie dans le groupe comprenant la douleur, l'épilepsie, l'inflammation, un trouble respiratoire, le prurit, un trouble des voies urinaires et une maladie inflammatoire de l'intestin ;

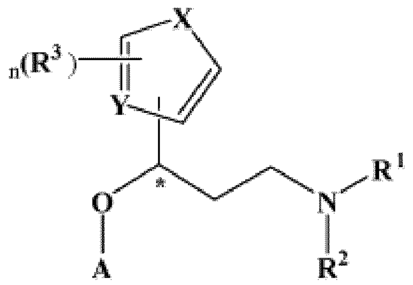

I

dans lequel,

A est un benzofuranyle ou un indanyle ;
$R^1$ et $R^2$ sont chacun indépendamment un hydrogène, un alkyle en Ci-Ce substitué ou non substitué ;
X est un atome d'oxygène ou un atome de soufre ;
Y est CH ou un atome d'azote ;
$R^3$ est un hydrogène, un halogène, un alkyle en Ci-Ce substitué ou non substitué ;
n est égal à 1 ;
« * » représente un atome de carbone chiral, et la configuration absolue de l'atome de carbone chiral est de type S ;
dans lequel, le terme « substitué » signifie que 1 à 4 atomes d'hydrogène sur le groupe sont substitués par un substituant choisi dans le groupe constitué d'un alkyle en Ci-Ce, d'un cycloalkyle en $C_3$-$C_7$, d'un haloalkyle en $C_1$-$C_3$, d'un halogène, d'un nitro, d'un cyano, d'un hydroxyle, d'un carboxyle en $C_1$-$C_4$, d'un groupe ester en $C_2$-$C_4$, d'un groupe amide en $C_2$-$C_4$, d'un alcoxyle en Ci-Ce, d'un haloalcoxy en $C_1$-$C_6$, d'un benzyle, d'un aryle à 5 ou 6 chaînons ou d'un hétéroaryle.

**2.** Composé ou sel à utiliser selon la revendication 1, dans lequel le composé comprend une ou plusieurs caractéristiques choisies dans le groupe suivant :

$R^1$ et $R^2$ sont chacun indépendamment un hydrogène ou un alkyle en $C_1$-$C_3$ ; et/ou
$R^3$ est un hydrogène, un halogène, un alkyle en $C_1$-$C_3$ substitué ou non substitué.

**3.** Composé ou sel à utiliser selon la revendication 1, dans lequel le composé comprend une ou plusieurs caractéristiques choisies dans le groupe suivant :

$R^1$ et $R^2$ sont chacun indépendamment un hydrogène, un méthyle ; et/ou
$R^3$ est un hydrogène, un atome de chlore ou un méthyle.

**4.** Composé ou un sel pharmaceutiquement acceptable de celui-ci à utiliser dans la prévention et/ou le traitement d'une maladie causée par une protéine du canal à potentiel de récepteur transitoire (TRP), dans lequel la maladie est choisie dans le groupe comprenant la douleur, l'épilepsie, l'inflammation, un trouble respiratoire, le prurit, un trouble des voies urinaires et une maladie inflammatoire de l'intestin, et le composé est choisi dans le groupe suivant :

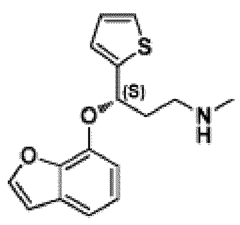

$I_C$-1

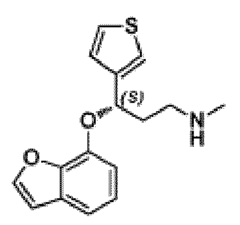

$I_C$-2

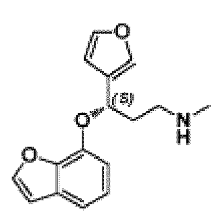

$I_C$-3

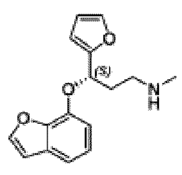

Ic-4

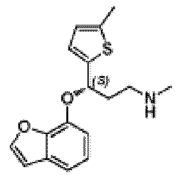

Ic-5

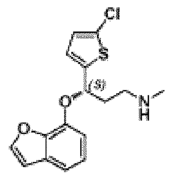

Ic-6

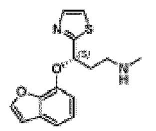

Ic-7

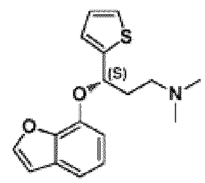

Ic-8

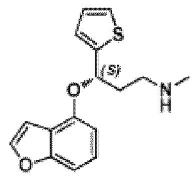

Ic-9

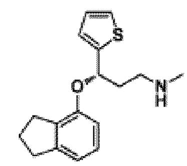

Ic-10

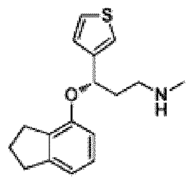

Ic-11

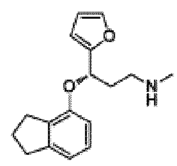

Ic-12

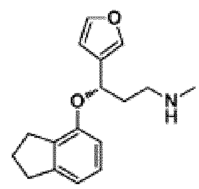

Ic-13

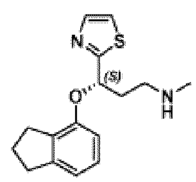

Ic-14

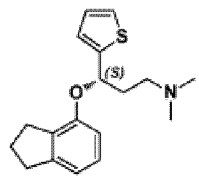

Ic-15

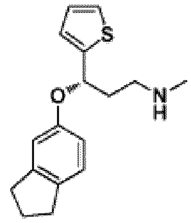

Ic-16

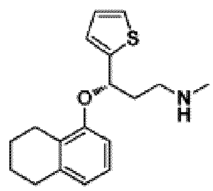

Ic-17

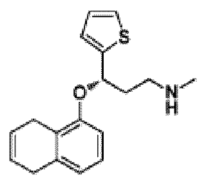

Ic-18

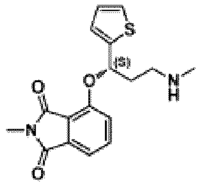

Ic-19

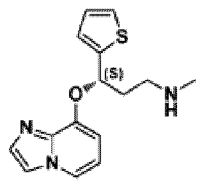

Ic-20

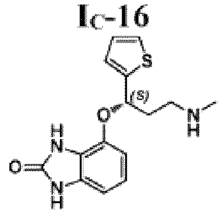

Ic-21

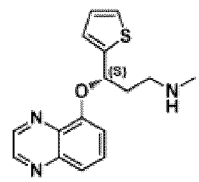

Ic-22

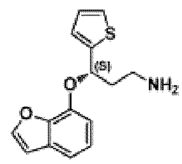

Ic-23

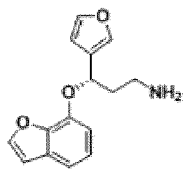

Ic-24

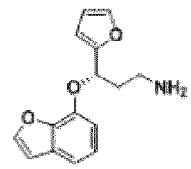

Ic-25

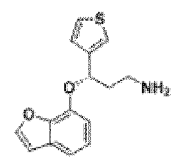

Ic-26

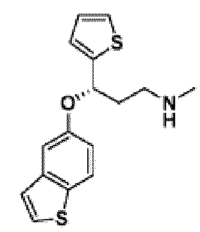

Ic-27

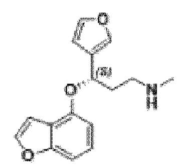

Ic-28

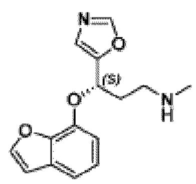

Ic-29

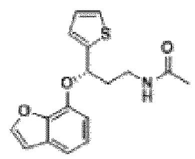

ID

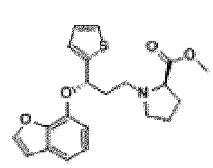

IE

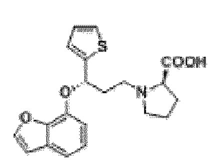

IF

**5.** Composé ou sel à utiliser selon la revendication 1 ou 4, dans lequel la protéine du canal à potentiel de récepteur transitoire est TRPA1.

**6.** Composé ou sel à utiliser selon la revendication 1 ou 4, dans lequel la maladie liée à la protéine du canal à potentiel de récepteur transitoire est la douleur.

**7.** Composé ou sel à utiliser selon la revendication 1 ou 4, dans lequel la douleur comprend une douleur inflammatoire aiguë, une douleur inflammatoire chronique, une douleur viscérale, une douleur neurogène, une fibromyalgie, un mal de tête, une névralgie ou une douleur causée par un cancer.

**8.** Composé de formule I, ou sel pharmaceutiquement acceptable de celui-ci

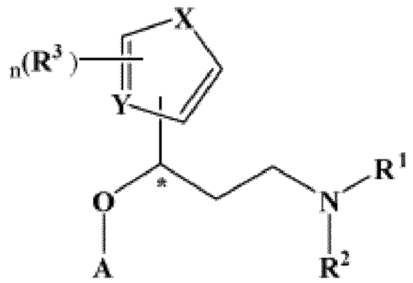

I

dans lequel,

A est un benzofuranyle ou un indanyle ;
$R^1$ et $R^2$ sont chacun indépendamment un hydrogène, un alkyle en Ci-Ce substitué ou non substitué ;
X est un atome d'oxygène ou un atome de soufre ;
Y est CH ou un atome d'azote ;
$R^3$ est un hydrogène, un halogène, un alkyle en Ci-Ce substitué ou non substitué ;
n est égal à 1 ;
« * » représente un atome de carbone chiral, et la configuration absolue de l'atome de carbone chiral est de type S ;
dans lequel, le terme « substitué » signifie que 1 à 4 atomes d'hydrogène sur le groupe sont substitués par un substituant choisi dans le groupe constitué d'un alkyle en Ci-Ce, d'un cycloalkyle en $C_3$-$C_7$, d'un haloalkyle en $C_1$-$C_3$, d'un halogène, d'un nitro, d'un cyano, d'un hydroxyle, d'un carboxyle en $C_1$-$C_4$, d'un groupe ester en $C_2$-$C_4$, d'un groupe amide en $C_2$-$C_4$, d'un alcoxyle en Ci-Ce, d'un haloalcoxy en $C_1$-$C_6$, d'un benzyle, d'un aryle à 5 ou 6 chaînons ou d'un hétéroaryle.

**9.** Composé selon la revendication 8, dans lequel le composé comprend une ou plusieurs caractéristiques choisies dans le groupe suivant :

$R^1$ et $R^2$ sont chacun indépendamment un hydrogène ou un alkyle en $C_1$-$C_3$ ; et/ou
$R^3$ est un hydrogène, un halogène, un alkyle en $C_1$-$C_3$ substitué ou non substitué.

**10.** Composé selon la revendication 8, dans lequel le composé comprend une ou plusieurs caractéristiques choisies dans le groupe suivant :

$R^1$ et $R^2$ sont chacun indépendamment un hydrogène ou un méthyle ; et/ou
$R^3$ est un hydrogène, un atome de chlore ou un méthyle.

**11.** Composé choisi dans le groupe suivant, ou sel pharmaceutiquement acceptable de celui-ci :

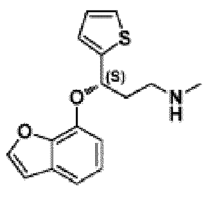

$I_C$-1

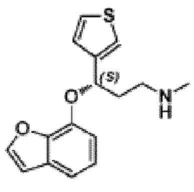

$I_C$-2

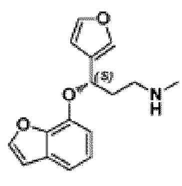

$I_C$-3

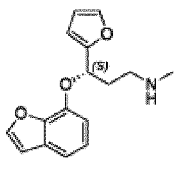

$I_C$-4

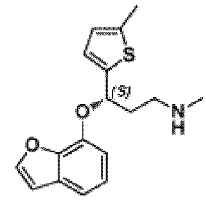

$I_C$-5

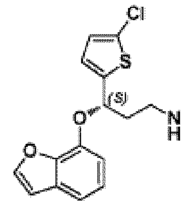

$I_C$-6

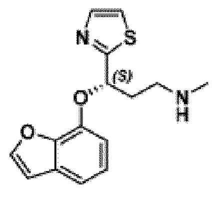

$I_C$-7

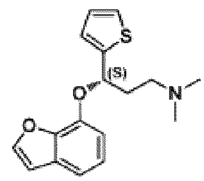

$I_C$-8

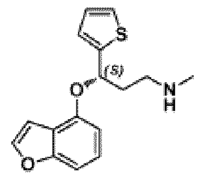

$I_C$-9

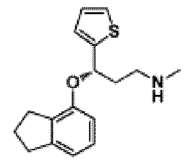

$I_C$-10

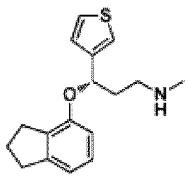

$I_C$-11

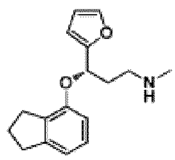

$I_C$-12

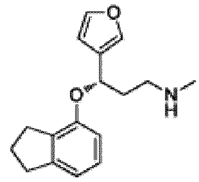

$I_C$-13

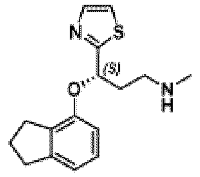

$I_C$-14

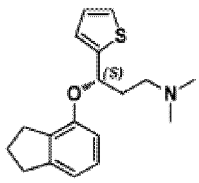

$I_C$-15

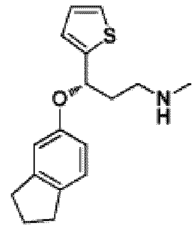

$I_C$-16

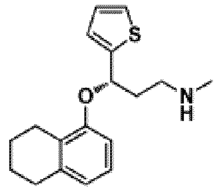

$I_C$-17

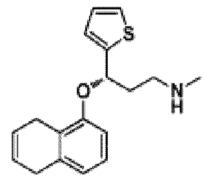

$I_C$-18

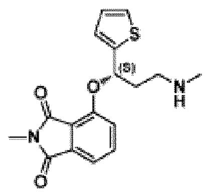

$I_C$-19

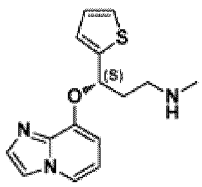

$I_C$-20

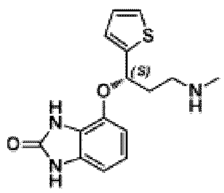

$I_C$-21

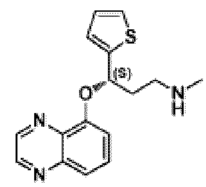

$I_C$-22

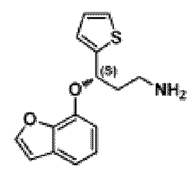

$I_C$-23

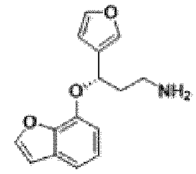

$I_C$-24

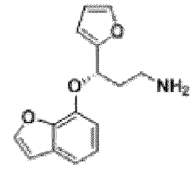

$I_C$-25

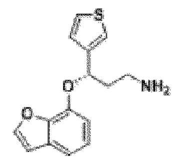

$I_C$-26

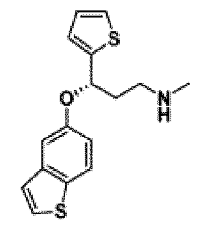

$I_C$-27

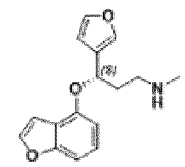

$I_C$-28

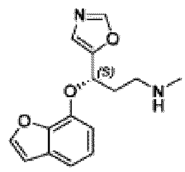

$I_C$-29

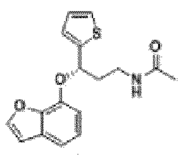

$I_D$

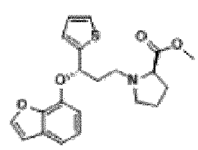

$I_E$

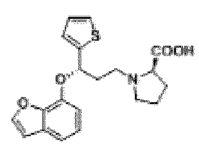

$I_F$

**12.** Composition pharmaceutique qui comprend un composé ou un sel pharmaceutiquement acceptable selon la revendication 8 ou 11 ; et
un support pharmaceutiquement acceptable.

**13.** Procédé de préparation d'un composé, ou d'un sel pharmaceutiquement acceptable selon la revendication 8 ou 11, dans lequel le procédé comprend l'étape consistant à :

dans un solvant inerte, faire réagir un intermédiaire de formule II avec $R^1$-NH-$R^2$, en formant ainsi un composé de formule I :

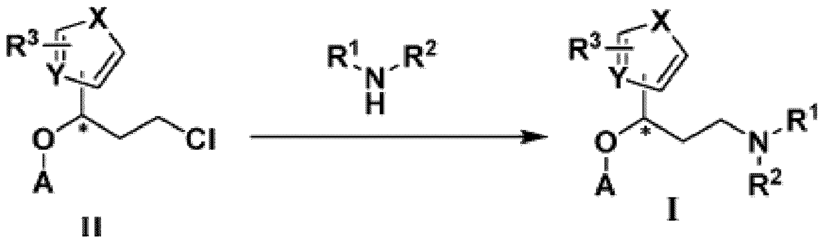

dans lequel X, Y, A, $R^1$, $R^2$, $R^3$ et « * » sont tels que définis dans la revendication 8.

**14.** Procédé pour inhiber de manière non thérapeutique et non diagnostique l'activité d'une protéine du canal à potentiel de récepteur transitoire (TRP) *in vitro,* qui comprend une mise en contact de la protéine ou des cellules du canal à potentiel de récepteur transitoire exprimant la protéine TRP avec un composé, ou un sel pharmaceutiquement acceptable selon la revendication 8 ou 11 pour inhiber l'activité de la protéine du canal à potentiel de récepteur transitoire.

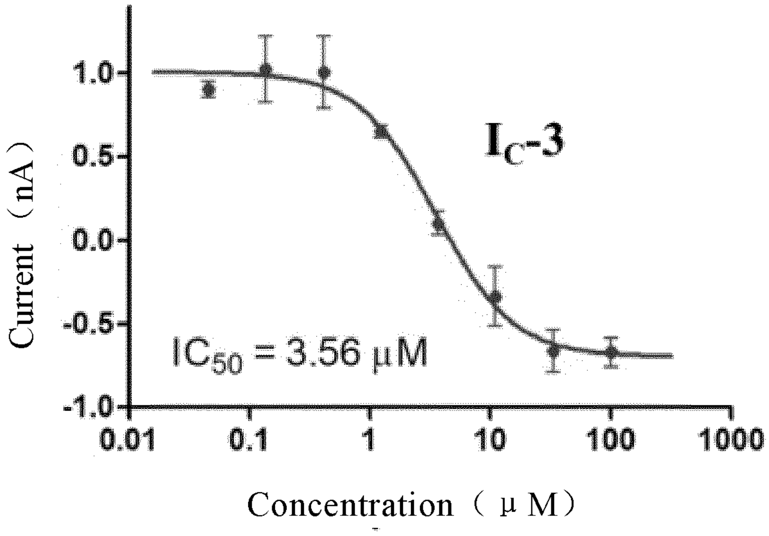
1.0
0.5
0.0
-0.5
-1.0
Current （nA）
$I_C$-3
$IC_{50}$ = 3.56 μM
0.01
0.1
1
10
100
1000
Concentration （μM）

Fig. 1A

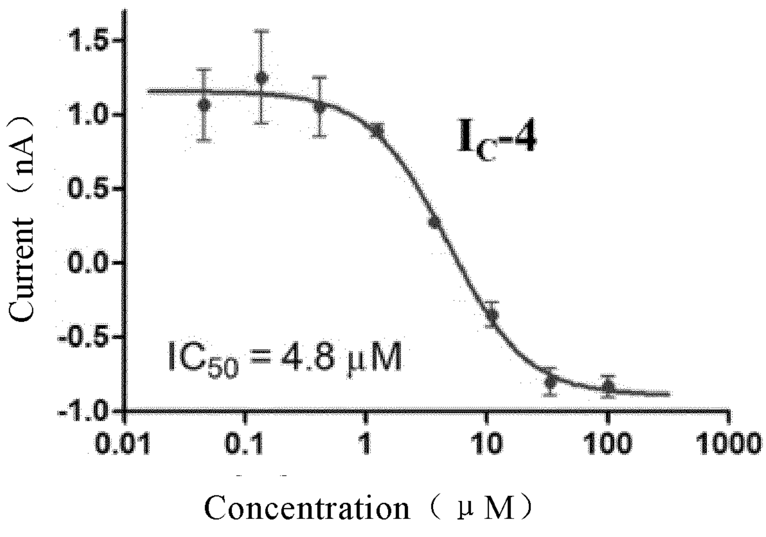
1.5
1.0
0.5
0.0
-0.5
-1.0
Current （nA）
$I_C$-4
$IC_{50}$ = 4.8 μM
0.01
0.1
1
10
100
1000
Concentration （μM）

Fig. 1B

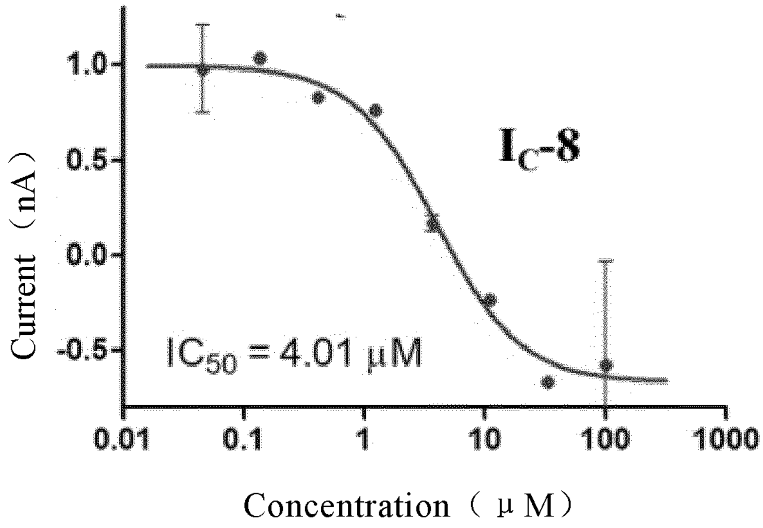
1.0
0.5
0.0
-0.5
Current （nA）
IC50 = 4.01 μM
$I_C$-8
0.01
0.1
1
10
100
1000
Concentration（ μ M）

Fig. 1C

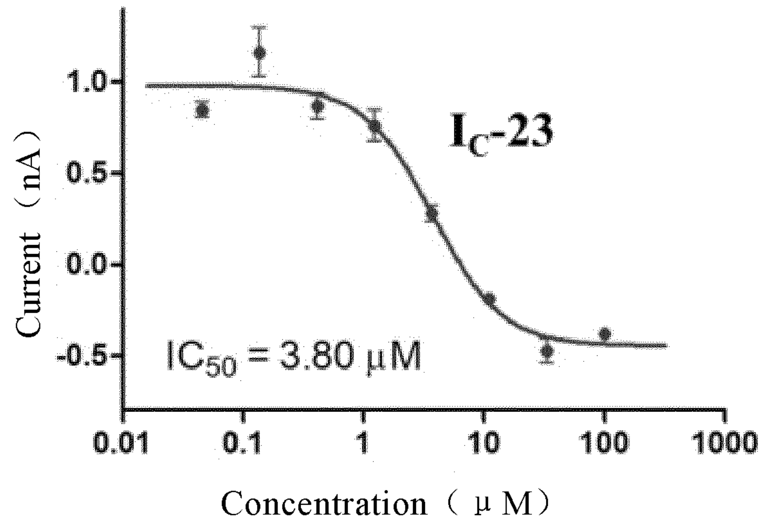
1.0
0.5
0.0
-0.5
Current （nA）
IC50 = 3.80 μM
$I_C$-23
0.01
0.1
1
10
100
1000
Concentration（ μ M）

Fig. 1D

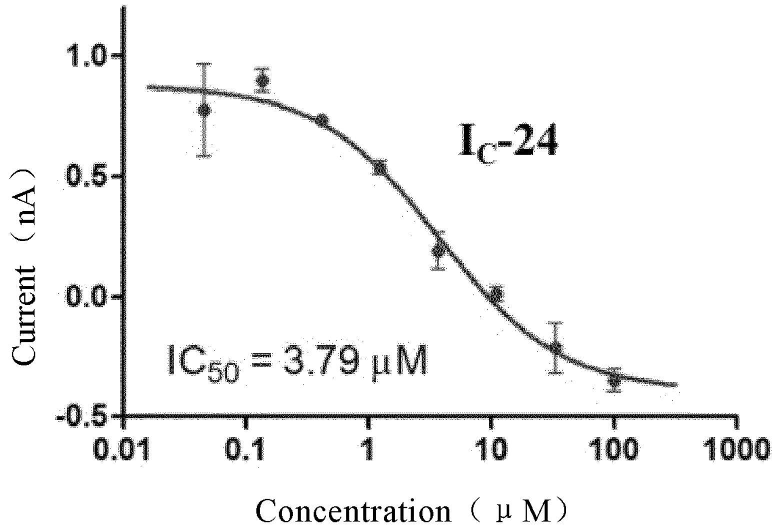
1.0
0.5
0.0
-0.5
Current （nA）
IC-24
IC50 = 3.79 μM
0.01
0.1
1
10
100
1000
Concentration （μM）

Fig. 1E

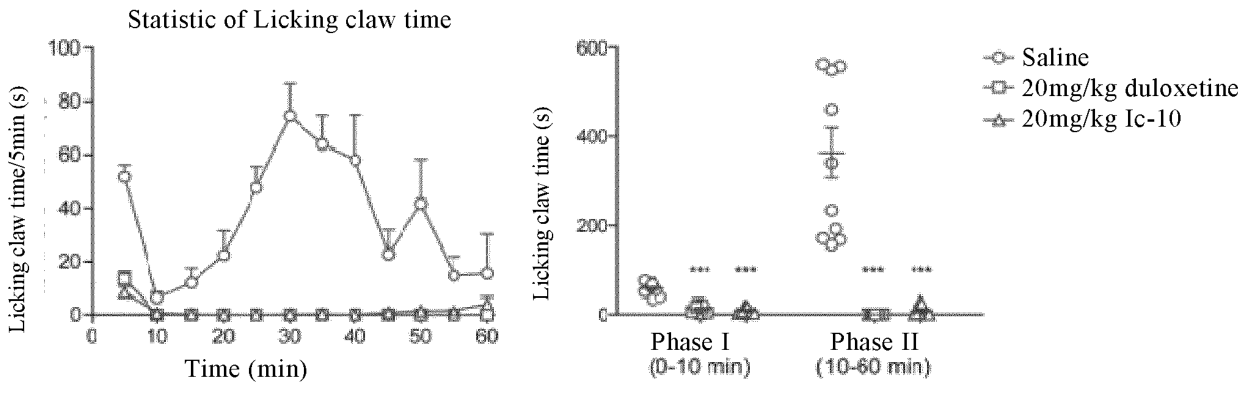
Statistic of Licking claw time
Licking claw time/5min (s)
Time (min)
Licking claw time (s)
Phase I
(0-10 min)
Phase II
(10-60 min)
Saline
20mg/kg duloxetine
20mg/kg Ic-10

Fig. 2

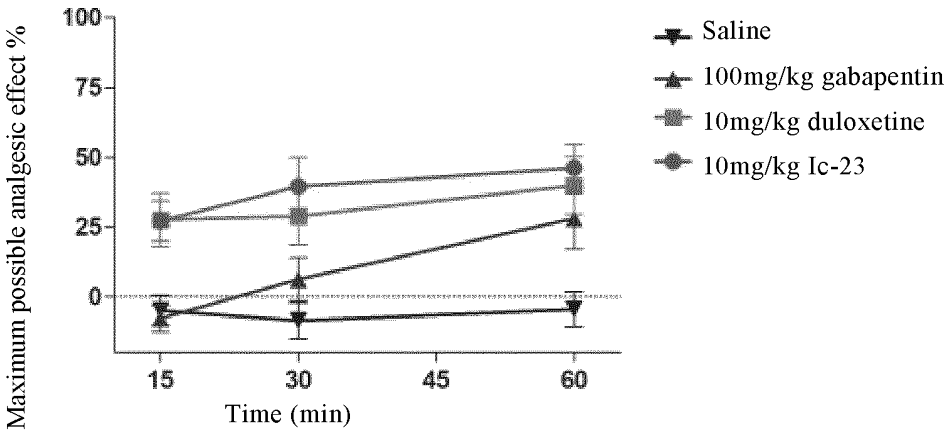
Maximum possible analgesic effect %
100
75
50
25
0
15
30
45
60
Time (min)
Saline
100mg/kg gabapentin
10mg/kg duloxetine
10mg/kg Ic-23

Fig. 3

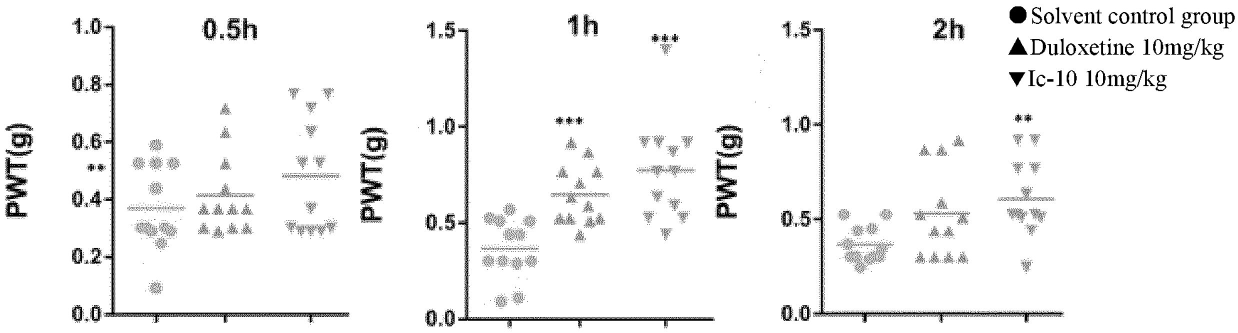
0.5h
1h
2h
PWT(g)
1.0
0.8
0.6
0.4
0.2
0.0
1.5
0.5
**
***
Solvent control group
Duloxetine 10mg/kg
Ic-10 10mg/kg

Fig. 4

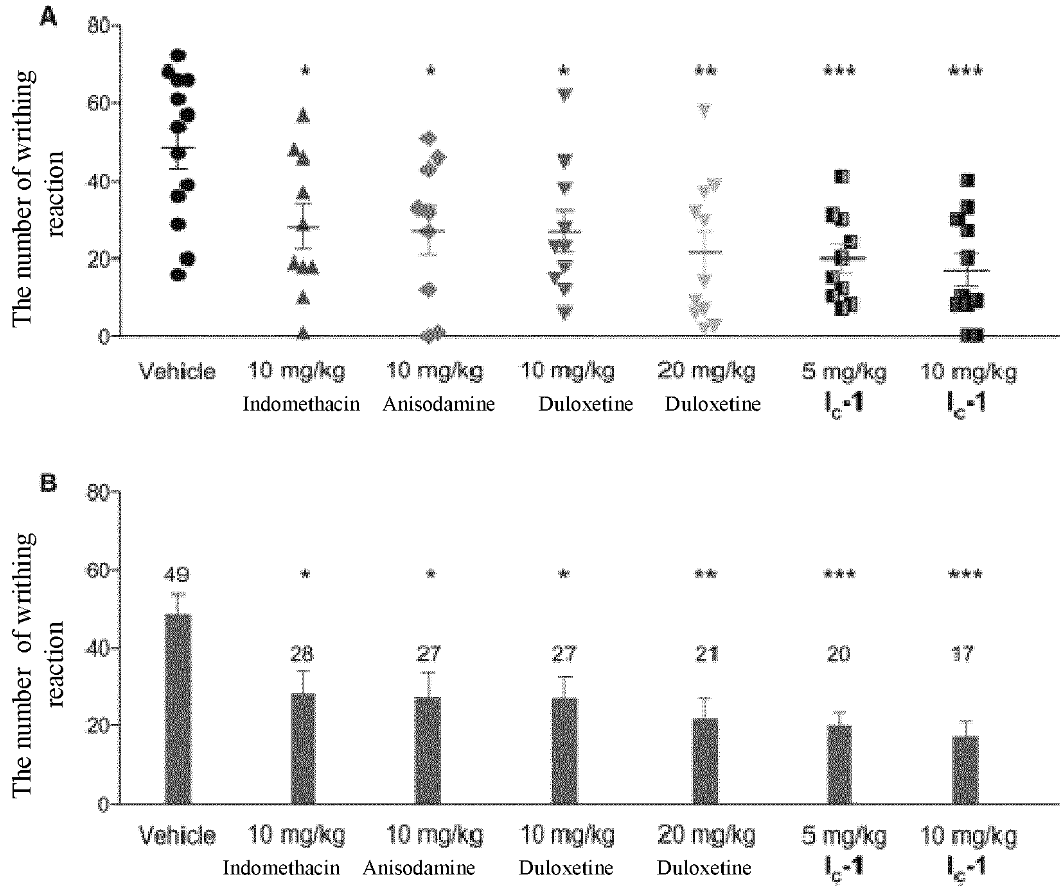
A
The number of writhing reaction
80
60
40
20
0
*
*
*
**
***
***
Vehicle
10 mg/kg Indomethacin
10 mg/kg Anisodamine
10 mg/kg Duloxetine
20 mg/kg Duloxetine
5 mg/kg Ic-1
10 mg/kg Ic-1
B
The number of writhing reaction
80
60
40
20
0
49
28
27
27
21
20
17
*
*
*
**
***
***
Vehicle
10 mg/kg Indomethacin
10 mg/kg Anisodamine
10 mg/kg Duloxetine
20 mg/kg Duloxetine
5 mg/kg Ic-1
10 mg/kg Ic-1

Fig. 5

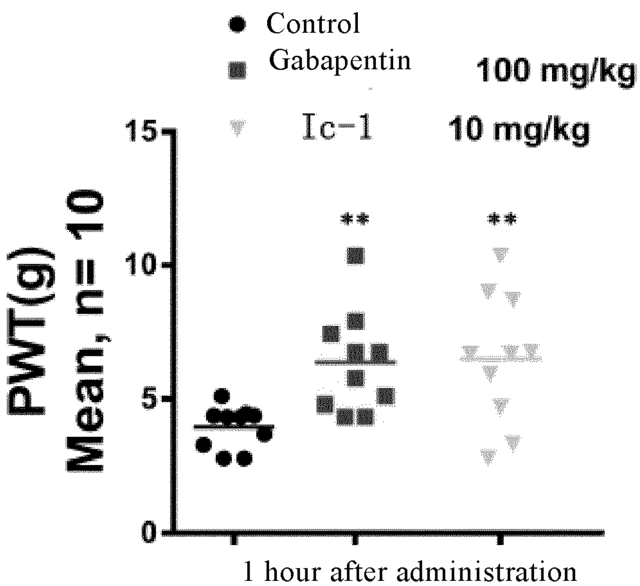
Control
Gabapentin 100 mg/kg
Ic-1 10 mg/kg
PWT(g)
Mean, n= 10
15
10
5
0
**
**
1 hour after administration

Fig. 6

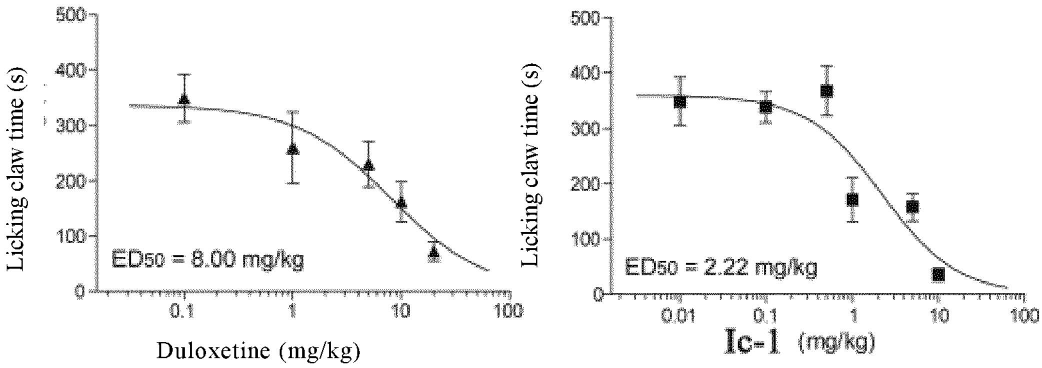
Licking claw time (s)
500
400
300
200
100
0
ED50 = 8.00 mg/kg
0.1
1
10
100
Duloxetine (mg/kg)
Licking claw time (s)
500
400
300
200
100
0
ED50 = 2.22 mg/kg
0.01
0.1
1
10
100
Ic-1 (mg/kg)

Fig. 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 107840845 A
- WO 2018115064 A1
- WO 02094262 A1
- CN 105497020 A
- WO 2010075353 A


### Non-patent literature cited in the description


- **BOOT, J.R. et al.** *Bioorganic & Medicinal Chemistry Letters*, vol. 14 (21), 5395-5399
- **CASHMAN JOHN R. et al.** *Bioorganic & Medicinal Chemistry*, vol. 17 (19), 6890-6897